(19) Europäisches Patentamt
European Patent Office
Office européen des brevets

(11) Publication number: **0 041 624**
**B1**

# (12) EUROPEAN PATENT SPECIFICATION

(45) Date of publication of patent specification: **01.02.84**

(51) Int. Cl.³: **A 01 N 43/50,**
C 07 D 401/04

(21) Application number: **81103639.1**

(22) Date of filing: **12.05.81**

(54) **Method to beneficially influence & alter the development & life cycle of agronomic and horticultural crops with certain imidazolinyl nicotinic acids and derivatives thereof.**

(30) Priority: **02.06.80 US 155866**
**17.04.81 US 255012**

(43) Date of publication of application:
**16.12.81 Bulletin 81/50**

(45) Publication of the grant of the patent:
**01.02.84 Bulletin 84/5**

(84) Designated Contracting States:
**AT BE CH DE FR GB IT LI LU NL SE**

(56) References cited:
**US - A - 4 188 487**

(73) Proprietor: **AMERICAN CYANAMID COMPANY**
**1937 West Main Street P.O. Box 60**
**Stamford Connecticut 06904 (US)**

(72) Inventor: **Orwick, Philip Lee**
**9 Rita Road**
**Morrisville Pennsylvania (US)**
Inventor: **Templeton, Andrew Robert**
**1707 Scenic Drive**
**West Trenton New Jersey (US)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 624 B1

Method to beneficially influence and alter the development and life cycle of agronomic and horticultural crops with certain imidazolinyl nicotinic acids and derivatives thereof

This invention relates to a method for increasing axillary branching, tillering, flowering and yield of agronomic and horticultural crops by applying to the foliage of said crops or to soil containing seeds or other propagating organs thereof, an axillary branching, tillering, flowering or yield increasing amount of a compound having the structure:

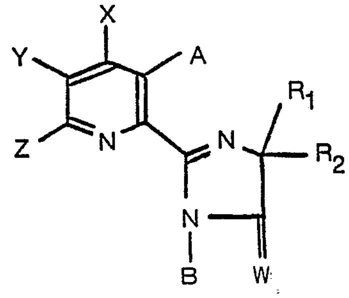

(I)

wherein

$R_1$ is $C_1$—$C_4$alkyl;

$R_2$ is $C_1$—$C_4$ alkyl or $C_3$—$C_6$ cycloalkyl; and when $R_1$ and $R_2$ are taken together they may represent $C_3$—$C_6$ cycloalkyl optionally substituted with methyl;

A is $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

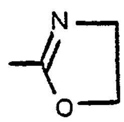

OR

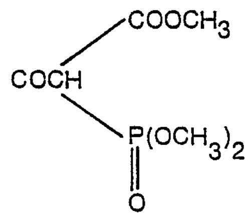

(II)

;

$R_3$ is hydrogen,

lower alkylidene amino

$C_1$—$C_{12}$ alkyl optionally substituted with one of the following groups: $C_1$—$C_3$ alkoxy, halogen, hydroxyl, $C_3$—$C_6$ cycloalkyl, benzyloxy, furyl, phenyl, halophenyl, loweralkylphenyl, loweralkoxyphenyl, nitrophenyl, carboxyl, loweralkoxycarbonyl, cyano or triloweralkylammonium;

$C_3$—$C_{12}$ alkenyl optionally substituted with one of the following groups: $C_1$—$C_3$ alkoxy, phenyl, halogen or loweralkoxycarbonyl or with two $C_1$—$C_3$ alkoxy groups or two halogen groups;

$C_3$—$C_6$ cycloalkyl optionally substituted with one or two $C_1$—$C_3$ alkyl groups;

$C_3$—$C_{10}$ alkynyl optionally substituted with one or two $C_1$—$C_3$ alkyl groups; or,

A cation selected from the group consisting of alkali metals, alkaline earth metals, manganese, copper, iron, zinc, cobalt, lead, silver, nickel, ammonium and organic ammonium;

$R_6$ is hydrogen, hydroxyl, $C_3$-alkenyl, $C_3$-alkynyl or $C_1$—$C_4$ alkyl optionally substituted with one hydroxyl or one chlorine group;

B is H, $COR_4$ or $SO_2R_5$, provided that when B is $COR_4$ or $SO_2R_5$; A is $COOR_3$ in which $R_3$ is other than H, or a salt-forming cation, $CH_3$ or CN; W is O; and Y and Z are not alkylamino, hydroxyl, or hydroxyloweralkyl;

$R_4$ is $C_1$—$C_{11}$ alkyl, chloromethyl or phenyl optionally substituted with one chloro, one nitro or one methoxy group;

$R_5$ is $C_1$—$C_4$ alkyl or phenyl optionally substituted with one methyl group;

W is O or S;

$R_8$ is $C_1$—$C_4$-alkyl or phenyl;

X is hydrogen, halogen, hydroxyl or methyl, with the proviso that when Y and Z are taken together to form a ring and YZ is represented by the structure: $-(CH_2)_n-$, where n is 3 or 4, X is hydrogen;

Y and Z each represent members selected from the group consisting of hydrogen, halogen, $C_1$—$C_6$ alkyl, hydroxy-loweralkyl, $C_1$—$C_6$ alkoxy, $C_1$—$C_4$ alkylthio, phenoxy, $C_1$—$C_4$-haloalkyl, nitro, cyano, $C_1$—$C_4$ alkylamino, diloweralkylamino or $C_1$—$C_4$ alkylsulfonyl group, or phenyl optionally substituted with one $C_1$—$C_4$ alkyl, $C_1$—$C_4$ alkoxy or halogen; and, when taken together, Y and Z may form a ring in which YZ are represented by the structure: $-(CH_2)_n-$, where n is an integer selected from 3 and 4, provided that X is hydrogen;

or

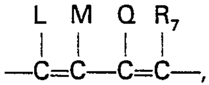

where L, M, Q and $R_7$ each represent members selected from the group consisting of hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $C_1$—$C_4$ alkylamino, diloweralkylamino, chlorophenyl, methylphenyl, or phenoxy substituted with one Cl, $CF_3$, $NO_2$ or $CH_3$ group, with the proviso that only one of L, M, Q or $R_7$, may represent a substituent other than hydrogen, halogen, $C_1$—$C_4$ alkyl or $C_1$—$C_4$-alkoxy. and when W is O and A is CN, $CH_3$ or $COOR_3$, provided that $R_3$ cannot be unsaturated alkyl and Y and Z cannot be alkylamino, dialkylamino or alkylthio, and the N-oxides thereof, and when $R_1$ and $R_2$ are not the same, the optical isomers thereof, and, except when $R_3$ is a salt-forming cation, the acid addition salts thereof.

This invention also relates to a method for effectively inducing and/or accelerating the maturation of cereal crops by applying the foliage of cereal plants and/or to soil containing seeds of said plants, a maturation accelerating amount of a 2(2-imidazolin-2-yl)pyridine or a 2-(2-imidazolin-2-yl)quinoline compound of formula (I). Imidazolinyl benzoic acid derivatives have been disclosed in US—A—4 170 462. These compounds can be used as plant growth regulants, particularly dwarfing agents, whereas the compounds of the invention increase axillary branching, tillering, flowering and yield.

Application of the formula (I) 2-(2-imidazolin-2-yl)pyridines or quinolines to the foliage of a variety legumes or herbaceous ornamentals, or to soil in which said plant species are grown, at rates between about 0.0001 kg/ha and 0.01 kg/ha, also has the advantage that such treatment frequently induces a mild dwarfing effect on the treated plants. This dwarfing effect is particularly advantageous in the treatment of ornamental plants wherein the aesthetic value of the plants can be measurably improved by reducing their height and increasing their canopy.

A preferred group of 2-(2-imidazolin-2-yl)pyridine compounds for use in the methods of the present invention has the formula shown as formula (I) above, wherein $R_1$ is methyl; $R_2$ is methyl, ethyl, isopropyl or cyclopropyl; W is oxygen; B is hydrogen, CO-alkyl $C_1$—$C_6$ or CO-phenyl optionally substituted with chloro, nitro or methoxy; A is $COOR_3$, $CH_2OH$ or CHO where $R_3$ is as described in formula (I) above, X is hydrogen, Y and Z are each selected from the group consisting of hydrogen, $C_1$—$C_6$ alkyl, $C_1$—$C_6$ alkoxy, halo, phenyl, nitro, cyano, trifluoromethyl or methylsulfonyl; and when Y and Z are taken together, YZ is $—(CH_2)_4$.

In formula I above, alkali metals include: sodium, potassium and lithium, but sodium is generally preferred. Further, the term "organic ammonium" is defined as a group consisting of a positively charged nitrogen atom joined to from one to four aliphatic groups, each containing from one to 20 carbon atoms. Among the organic ammonium groups which are illustrative for the preparation of aliphatic ammonium salts of the formula (I) imidazolinyl nicotinic acids and esters herein are: monoalkylammonium, dialkylammonium, trialkylammonium, tetraalkylammonium, monoalkenylammonium, dialkenylammonium, trialkenylammonium, monoalkynylammonium, dialkynylammonium, trialkynylammonium monoalkanolammonium, dialkanolammonium, trialkanolammonium, $C_5$—$C_6$-cycloalkylammonium, piperidinium, morpholinium, pyrrolidinium, benzylammonium and equivalents thereof. Exemplary of halogen hereinabove are chlorine, fluorine, bromine, and iodine, but chlorine and bromine are preferred.

In accordance with the process of the present invention, formula (I), 2-(2-imidazolin-2-yl)pyridine esters, wherein A is $COOR_3$ and $R_3$ represents a substituent other than hydrogen or a salt-forming cation, and $R_1$, $R_2$, X, Y and Z are as described above, can be prepared by reacting an imidazopyrrolopyridinedione, represented by formula (III), hereinbelow, with an appropriate alcohol and corresponding alkali metal alkoxide at a temperature ranging between about 20°C and about 50°C.

In these reactions, the alcohol can function both as reactant and solvent. As such, a secondary solvent is not required. However, when an expensive alcohol is employed in the reaction, a less expensive secondary solvent, such as dioxane, tetrahydrofuran or other non-protic solvent, may be added to the reaction mixture. The amount of non-protic solvent added to the reaction mixture may be widely varied.

The overall reaction can be graphically illustrated as follows:

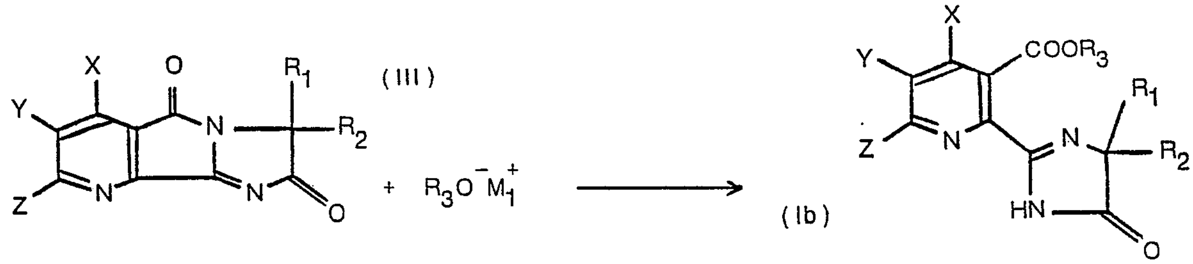
(III) (Ib)

where $M_1$ is an alkali metal, and X, Y, Z, $R_1$, $R_2$ and $R_3$ are as above defined.

Advantageously, the formula (Ib) 2-(2-imidazolin-2-yl)pyridine esters can also be prepared from a formula (IV) dioxopyrrolopyridine acetamide, wherein $R_1$, $R_2$, X, Y and Z are as described above, by cyclization thereof with a strong base, such as 1,5-diazabicyclo [5.4.0] undec-5-ene (DBU), in the

presence of an inert organic solvent such as xylene or toluene to give the crude imidazopyrrolopyridine of formula (III). The reaction mixture is heated to a temperature between 100°C and 150°C, and water is removed from the reaction mixture during the reaction using any convenient means, e.g., a Dean-Stark water separator. At least one equivalent of alcohol, represented by the formula (V) $R_3OH$, wherein $R_3$ represents a member other than hydrogen or a salt-forming cation, and $R_1$, $R_2$, X, Y and Z are as hereinabove described, is then added to the reaction mixture and the thus prepared mixture heated to reflux at a temperature between 100°C and 150°C to yield the formula (Ib) 2-(2-imidazolin-2-yl)-pyridine ester. The overall reaction can be graphically illustrated as follows:

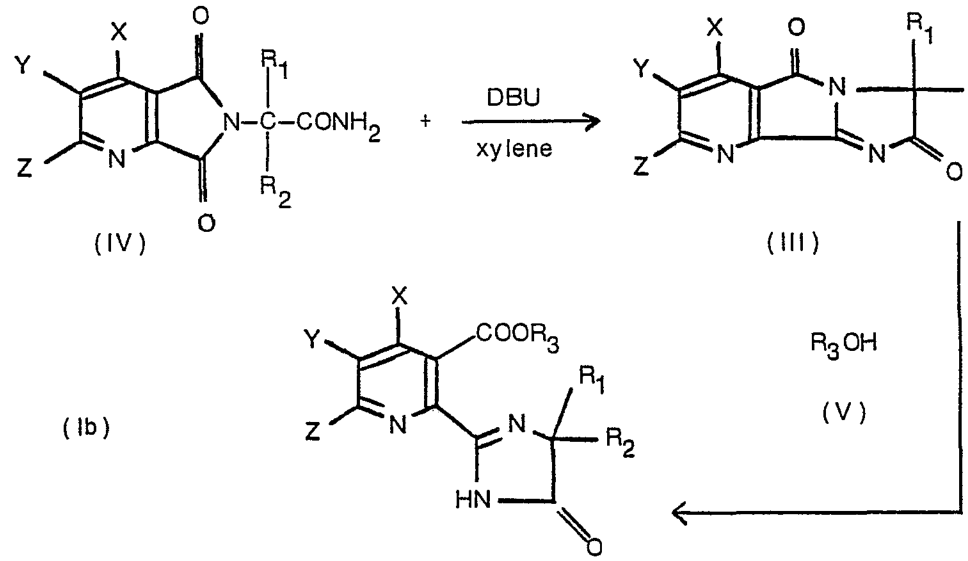


wherein X, Y Z, $R_1$, $R_2$ and $R_3$ are as described above.

In still another embodiment relating to the preparation of the formula (Ib) 2-(2-imidazolin-2-yl)pyridine esters, the cyclization of a carbamoyl nicotinic acid ester, represented by formula (VI), with phosphorus pentachloride at an elevated temperature, generally between about 60°C and 100°C occurs. The reaction is preferably conducted in the presence of an inert organic solvent, such as toluene or benzene. Good yields of the hydrochloride salt of the desired formula (Ib) ester are attained. The hydrochloride salt is then readily converted to the formula (Ib) ester by dissolution of the acid addition salt in water and neutralization of the thus-prepared solution with base, such as sodium or potassium carbonate. The overall reactions can be illustrated as follows:

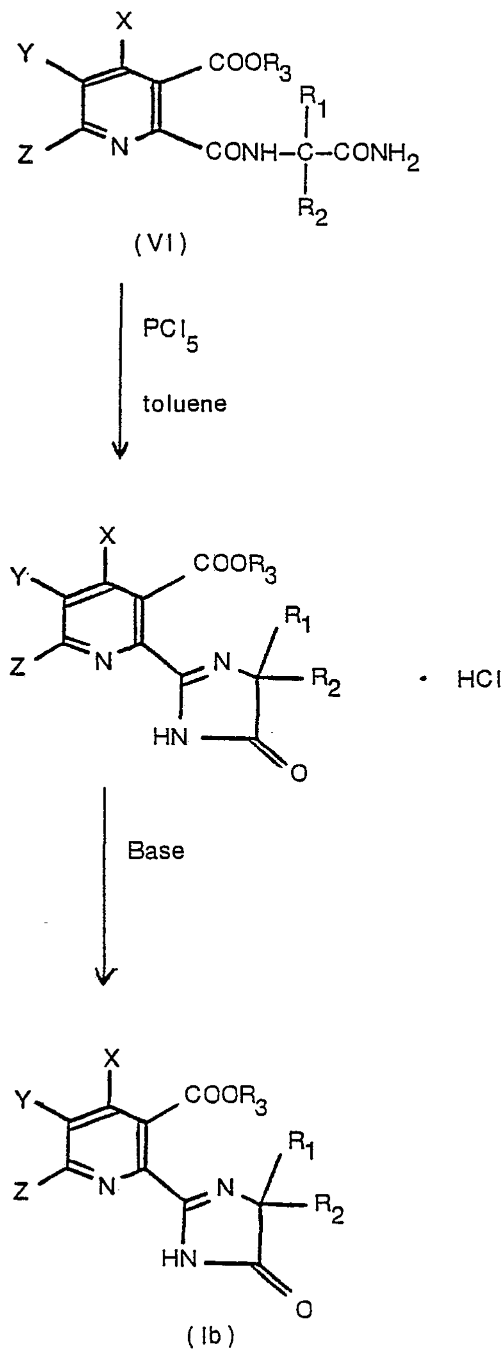


(VI)

(Ib)

wherein A is $COOR_3$ and $R_3$ is a substituent other than hydrogen or a salt-forming cation, and $R_1$, $R_2$, X, Y and Z are as hereinabove described.

In still another embodiment for the preparation of the formula (Ib) 2-(2-imidazolin-2-yl)pyridines esters of the present invention, the cyclization of a carbamoyl nicotinic acid ester represented by formula (VI) using a mixture of phosphorus pentachloride and phosphorus oxychloride is accomplished. The reaction mixture is stirred at room temperature from about four to eight hours and then the $POCl_3$ removed *in vacuo*. The remaining residue is dispersed in an organic solvent such as toluene. The solvent is removed and the residue dispersed in water and heated to between 80°C and 100°C. After cooling, the pH of the aqueous mixture is adjusted to 5—6 with sodium bicarbonate, and the product extracted into methylene chloride to give the desired formula (Ib) 2-(2-imidazolin-2-yl)pyridine ester. The reaction can be graphically illustrated as follows:

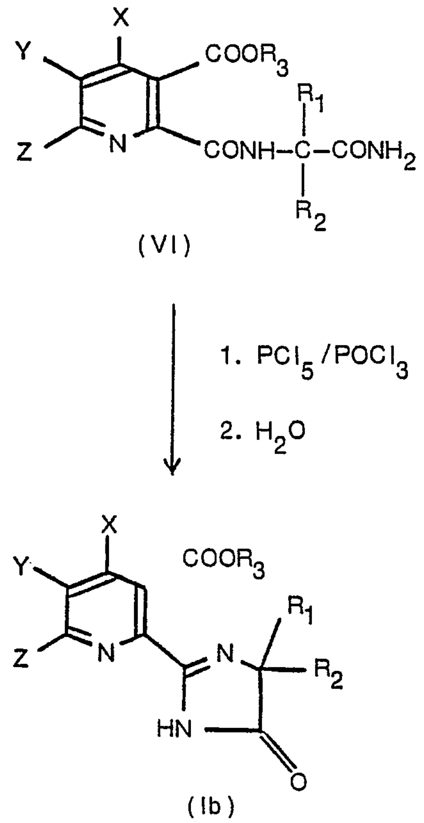


(VI)

(Ib)

where A is $COOR_3$ and $R_3$ is a substituent other than hydrogen or a salt-forming cation, and $R_1$, $R_2$, X, Y and Z are as described above.

The formula (Ib) 2-(2-imidazolin-2-yl)pyridine ester in which A is $COOR_3$ and $R_3$ is alkyl $C_1$—$C_{12}$, alkenyl $C_3$—$C_{12}$, alkynyl $C_3$—$C_{10}$, cycloalkyl $C_3$—$C_6$ or the substituted derivatives of these groups and X, Y, Z, $R_1$ and $R_2$ are as described above, may be converted to the corresponding amide where A is $CONH_2$ by reaction with ammonia under superatmospheric pressure at a temperature between about 25°C and 125°C. This reaction can be conducted in a protic solvent such as a lower alkanol or an aprotic solvent such as tetrahydrofuran, dioxane or the like. Likewise, using similar conditions but substituting hydroxylamine for ammonia in the above reaction yields the hydroxamic acid. These reactions may be graphically illustrated as follows:

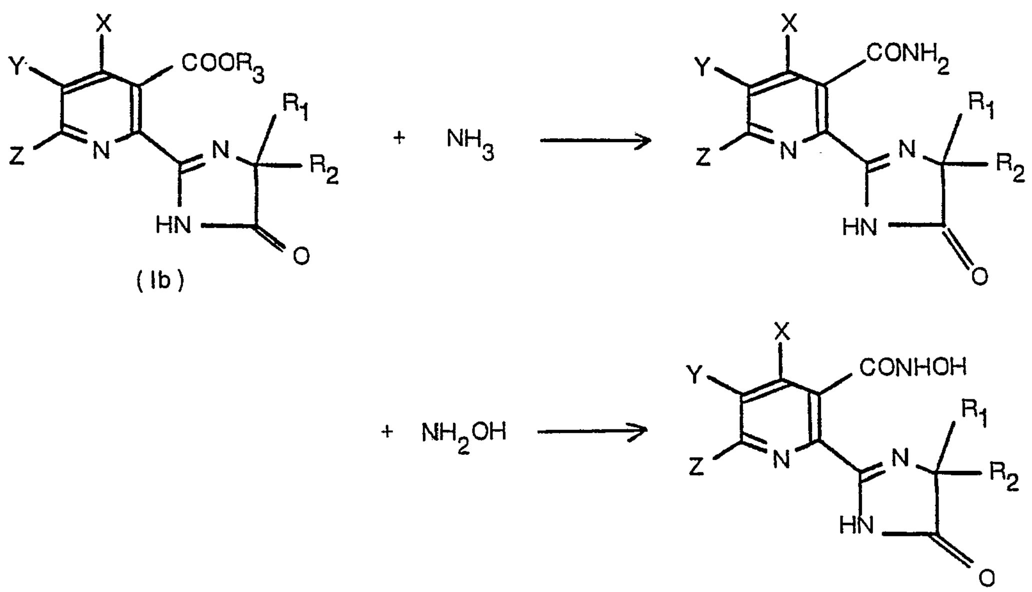


(Ib)

Treatment of the thus prepared primary amide described above with titanium tetrachloride and triethylamine, preferably in the presence of an inert aprotic solvent, such as tetrahydrofuran yields the

corresponding nitrile. The reaction is generally conducted under a blanket of inert gas, such as nitrogen, at a temperature between about 0°C and 10°C. The reaction may be illustrated as follows:

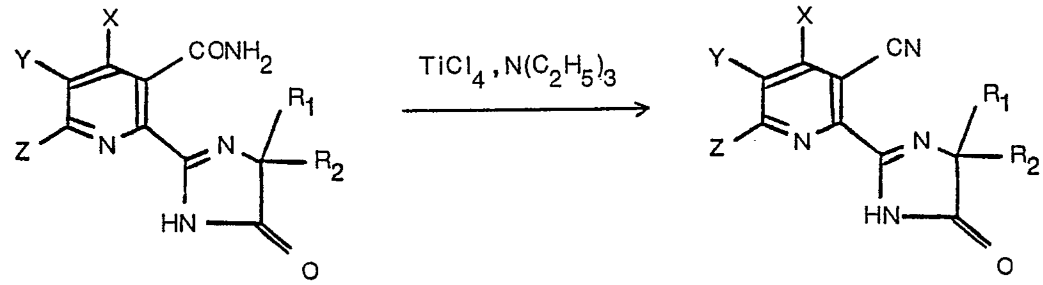


where X, Y, Z, $R_1$ and $R_2$ are as described above.

Preparation of the formula (VIII) N-substituted imidazolinone derivatives, wherein B is $COR_4$ or $SO_2R_5$; and A is $CH_3$, CN or $COOR_3$; and W is O; and $R_1$, $R_2$, $R_3$, X, Y and Z are as described above, excepting that Y and Z cannot be alkylamino, hydroxy or hydroxyloweralkyl; can be achieved by reaction of the appropriately substituted formula (I) 2-(2-imidazolin-2-yl)pyridine with an excess of acyl halide, acyl anhydride, or sulfonyl halide, alone or in a solvent such as pyridine or toluene at an elevated temperature between about 50°C and 125°C. The reaction can be graphically illustrated as follows:

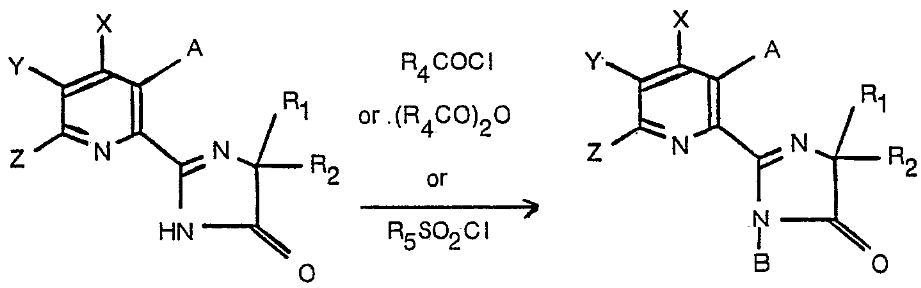


(VIII)

where A is $CH_3$, CN or $COOR_3$; $R_1$, $R_2$, $R_3$, $R_4$, $R_5$, X, Y and Z are as described above excepting that Y and/or Z cannot be alkylamino, hydroxyl, hydroxyloweralkyl.

Reaction of either the formula (I) 2-(2-imidazolin-2-yl)pyridine or the formula (VIII) N-substituted imidazolinone derivatives, described and illustrated immediately above, wherein A is $CH_3$, CN or $COOR_3$ provided that $R_3$ is as described above, excepting that it cannot be an unsaturated alkyl group, B is $R_4CO$ or $R_5SO_2$ and Y or Z cannot be alkylamino, alkylthio or dialkylamino; with an excess of m-chloroperbenzoic acid in the presence of an inert solvent such as methylene chloride, at refluxing temperature, yields the N-oxide corresponding to the pyridine derivative utilized as starting material. The reaction may be illustrated as follows:

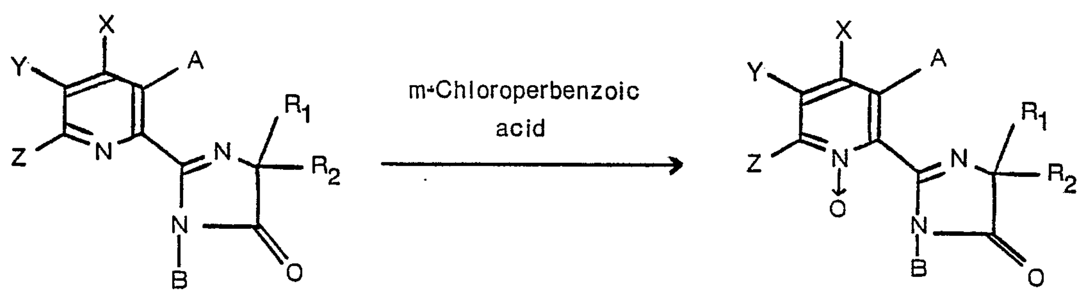


wherein A is $CH_3$, CN or $COOR_3$ is as described above excepting that $R_3$ cannot be an unsaturated alkyl group; B is $COR_4$ or $SO_2R_5$, $R_1$, $R_2$, $R_4$, $R_5$, X, Y and Z are as described above excepting that Y and Z cannot be alkylamino, alkylthio or dialkylamino.

Hydrolysis of the thus prepared N-oxide using a strong base such as sodium hydroxide in a lower alcohol yields the corresponding N-oxide in which B is H.

Advantageously, formula (I) esters in which B is hydrogen; W is oxygen and A is $COOR_3$ wherein $R_3$ is a saturated $C_1$—$C_{12}$ alkyl, $C_3$—$C_6$ cycloalkyl or benzyl substituent, and $R_1$, $R_2$, X, Y and Z are as defined above; can be prepared by reaction of the corresponding acid, i.e., where A is COOH, with an appropriate alcohol in the presence of a catalytic amount of a strong mineral acid such as hydrochloric

acid, sulfuric acid or the like; at a temperature between about 50°C and 100°C. The reaction may be illustrated as follows:

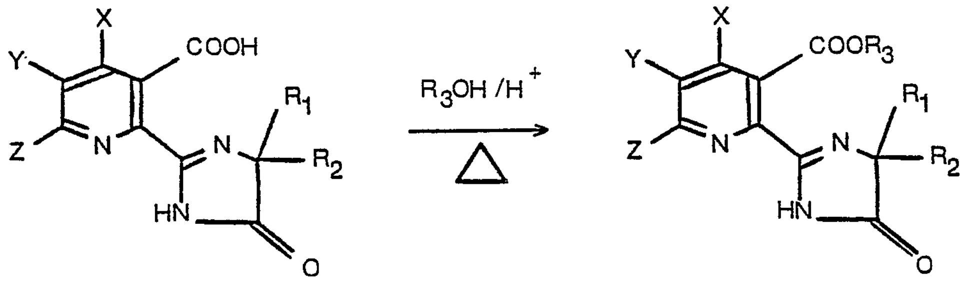


wherein $R_3$ is $C_1$—$C_{12}$ alkyl, $C_3$—$C_6$ cycloalkyl or benzyl; and $R_1$, $R_2$, X, Y and Z are as defined above.

The formula (I) acid as shown immediately above where A is COOH; B is hydrogen; W is oxygen and $R_1$, $R_2$, X, Y and Z are as defined above, is also readily converted to the corresponding methyl ester by reaction with diazomethane at a temperature between about 0°C and 25°C. The thus prepared methyl ester may then be reacted with an alkali metal alkoxide such as a sodium or potassium alkoxide, for convenience shown as $R_3ONa$, and an appropriate alcohol represented by the structure $R_3OH$, wherein $R_3$ is $C_1$—$C_{12}$ alkyl optionally substituted with one $C_1$—$C_3$ alkoxy, $C_3$—$C_6$ cycloalkyl, benzyloxy, furyl, phenyl, halophenyl, loweralkylphenyl, loweralkoxyphenyl, nitrophenyl or cyano; $C_3$—$C_{12}$ alkenyl optionally substituted with one or two $C_1$—$C_3$ alkoxy, phenyl or halogen groups; $C_3$—$C_8$ cycloalkyl optionally substituted with one or two $C_1$—$C_3$ alkyl groups or with $C_3$—$C_{10}$ alkynyl optionally substituted with one or two $C_1$—$C_3$ alkyl groups. The above reactions may be illustrated as follows:

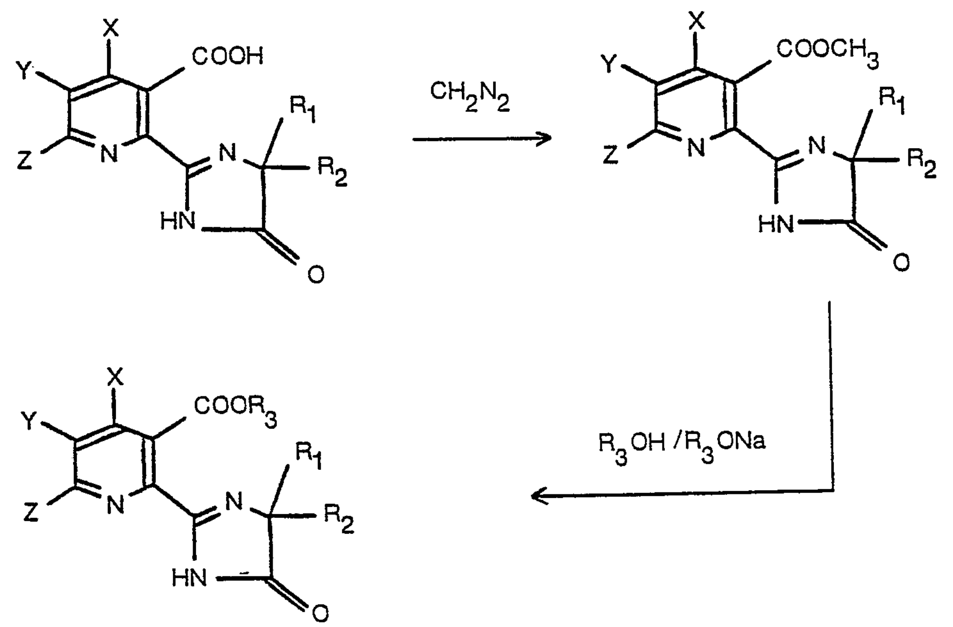


wherein $R_1$, $R_2$, $R_3$, X, Y and Z are as defined above.

Conversion of the above-identified formula (I) esters to their corresponding acid addition salts is readily achieved by treatment of said esters with strong acids, particularly strong mineral acids such as hydrochloric acid, sulfuric acid or hydrobromic acid.

Where the hydrohalide acid addition salts are desired, the formula (I) ester, wherein A is $COOR_3$ and $R_3$ is other than hydrogen or a salt-forming cation, and $R_1$, $R_2$, X, Y and Z are as described above, is dissolved in an organic solvent such as methylene chloride, chloroform, ether or the like. Addition of at least one equivalent of acid to the thus-prepared solution then yields the desired acid addition salt. The reaction may be illustrated as follows:

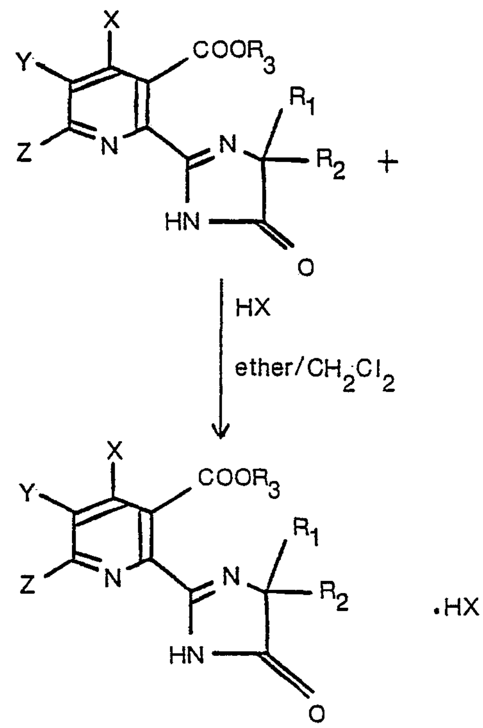


When the sulfuric acid salt of the ester is desired, the formula (I) ester is generally dissolved in a lower aliphatic alcohol such as methanol, ethanol, isopropanol or the like or mixtures of the above with water. Treatment of the mixture with at least one equivalent of sulfuric acid yields the sulfuric acid addition salt of the formula (I) ester.

In a further embodiment of the invention, the formula (I) compounds, wherein A is $COOR_3$ and $R_3$ is hydrogen and $R_1$, $R_2$, X, Y and Z, are as defined above, except that X, Y and Z cannot be $NO_2$ or halogen, can be prepared by hydrogenolysis of the benzyl ester of the imidazolinyl pyridine shown in formula (XV), wherein $R_1$, $R_2$, X, Y and Z, are as above-defined employing a palladium or platinum catalyst. In this reaction, the formula (XV) benzyl ester is dissolved or dispersed in an organic solvent, such as lower alcohol, an ether such as dioxane, tetrahydrofuran or the like, toluene or xylene. The catalyst, preferably palladium on a carbon carrier, is added to the mixture and the mixture heated to between 20°C and 50°C. The heated mixture is then treated with hydrogen gas to yield the desired acid. The reaction may be graphically illustrated as follows:

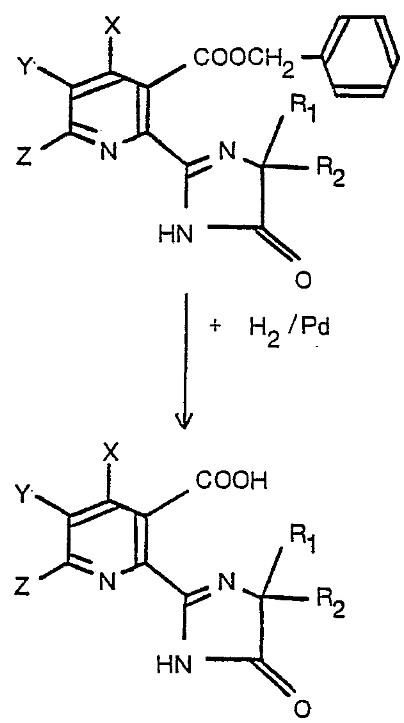


(XV)

(I)

Alternatively, the formula (I) acids where A is COOH may be prepared by treatment of an aqueous solution of the formula (I) ester with a strong base. In practice the formula (I) ester is generally treated with one equivalent of base in aqueous solution, and the mixture heated to between 20°C and 50°C. The mixture is then cooled and adjusted to pH 6.5 to 7.5 and preferably pH 7, with a strong mineral acid. Such treatment yields the desired acid. The reaction can be illustrated as follows:

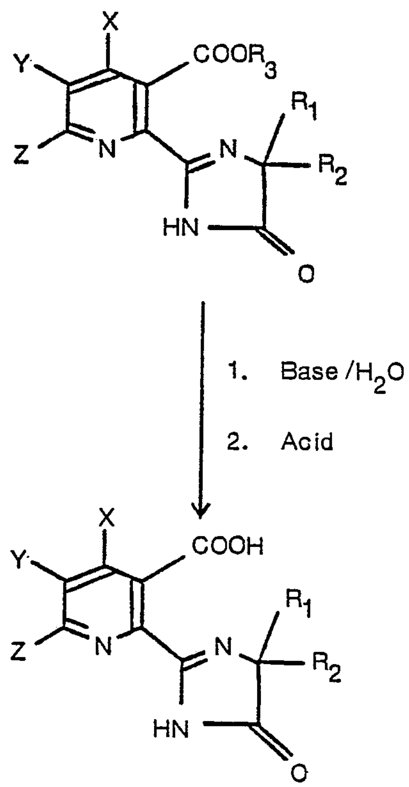


where $R_3$ is other than hydrogen or a salt-forming cation, and $R_1$, $R_2$, X, Y and Z are as described with reference to formula (I).

The formula (I) acids wherein A is COOH; B is hydrogen; W is oxygen and X, Y, Z, $R_1$ and $R_2$ are as described, can be prepared by reaction of the appropriately substituted formula (XVIII) imidazolinone with alkyl lithium, preferably in the presence of an inert solvent such as tetrahydrofuran under a blanket of nitrogen at a temperature between about −70°C and −80°C. The thus-formed mixture is then treated with hexamethylphosphoramide and carbon dioxide preferably in an inert solvent such as tetrahydrofuran, to yield the desired product. Where it is desirable to obtain the formula (I) pyridine derivatives in which A is $CH_3$ and X, Y, Z, $R_1$ and $R_2$ are as stated above, the formula (XVIII) imidazolinone is treated in the same manner as described for the preparation of the acid, excepting that methyl iodide is substituted for the carbon dioxide. If dimethylformamide is substituted for the methyl iodide, the corresponding formyl derivatives are obtained. These reactions may be illustrated as follows:

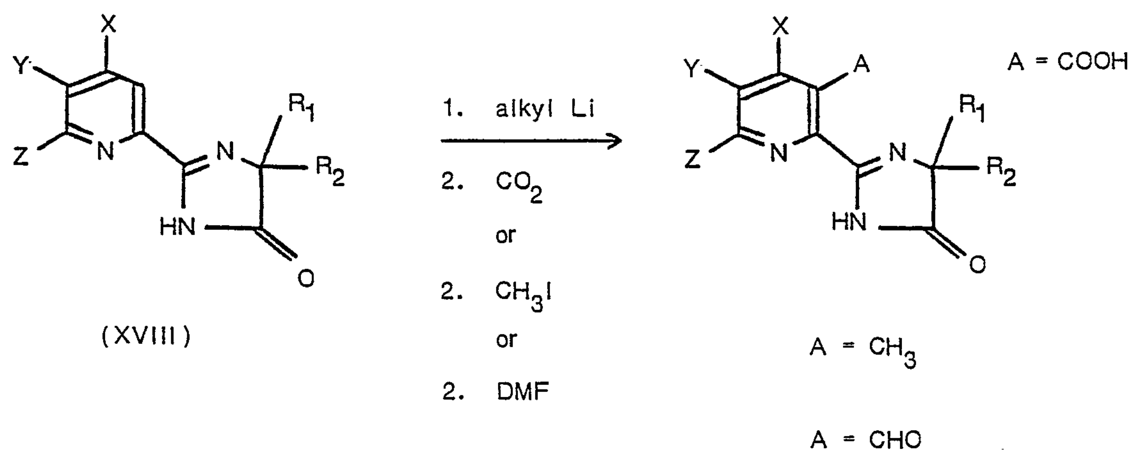


(XVIII)

Advantageously, the formula (I) acids may be converted to the formula (VII) 5-H-

imidazo[1′,2′:1,2]-pyrrolo[3,4-*b*]pyridine-3(2*H*),5-diones by reaction with dicyclohexylcarbodiimide (DCC). The reaction is preferably conducted using approximately an equimolar amount of the carbodiimide in the presence of a chlorinated hydrocarbon solvent at a temperature between about 20°C to 32°C. The reaction may be graphically illustrated as follows:

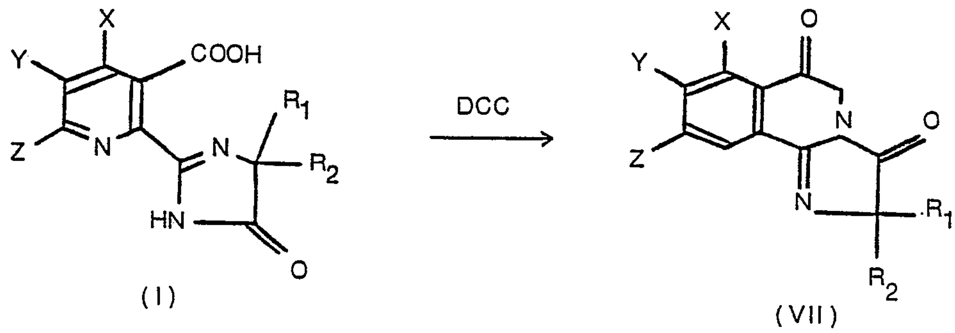


(I) (VII)

The formula (VII) 5H-imidazo[1′,2′:1,2]pyrrolo-[3,4-*b*]pyridine-3(2*H*),5-diones are isomers of the formula (III) imidazopyrrolopyridinediones referred to above, and are especially useful in the preparation of a variety of the formula (I) 2-(2-imidazolin-2-yl)-pyridine derivatives of the present invention, as will become apparent from the following discussion.

In practice, it is found that the formula (VII) 3(2*H*),5-diones can be reacted with at least one equivalent of an appropriate formula (V) $R_3OH$ alcohol in the presence of triethylamine as catalyst to yield the formula (I) pyridine ester corresponding to the alcohol used. The reaction is preferably conducted at a temperature between about 20°C and 50°C in the presence of an inert aprotic solvent, such as tetrahydrofuran, dioxane or the like. The reaction may be illustrated as follows:

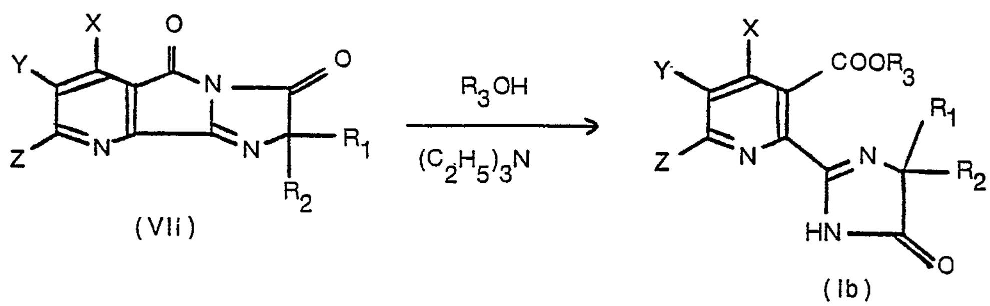


(VII) (Ib)

wherein $R_3$ represents a substituent as described above, excepting that hydrogen and salt-forming cations are excluded, and $R_1$, $R_2$, X, Y and Z, are as described above.

The formula (VII) 3(2H),5-diones are also readily converted to the formula (Ib)-2-(2-imidazolin-2-yl)-pyridine derivates wherein $R_1$, $R_2$, X, Y and Z, are as described above; W is oxygen; B is hydrogen, and A is acetyl, benzoyl, trimethylphosphonoacetate or hydroxymethyl; by reaction thereof with methyl magnesium bromide, phenyl lithium, sodium trimethyl phosphonoacetate or sodium borohydride; respectively. The methyl magnesium bromide, phenyl lithium and sodium trimethylphosphonoacetate reactions are preferably carried out at a temperature between about −50°C and −80°C, in the presence of an inert solvent such as tetrahydrofuran or dioxane und a blanket of inert gas, such as nitrogen. Reaction of the above-said formula (VII) diones with sodium borohydride is relatively mild. The reaction does not require a blanket of inert gas and can be conducted at temperatures between about −10°C and +15°C.

Reaction of the formula (VII) diones with at least one equivalent of acetone oxime yields the acetone oxime ester of the formula (I) 2-(2-imidazolin-2-yl)pyridine where A is $COON{=}C(CH_3)_2$, B is hydrogen and $R_1$, $R_2$, X, Y and Z, are as described for formula (I) pyridine derivatives. The above reaction

is generally conducted in the presence of an inert organic solvent such as toluene, benzene, xylene or the like at a temperature between about 40°C and 80°C.

The above reactions are graphically illustrated below:

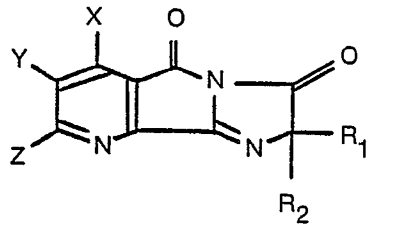

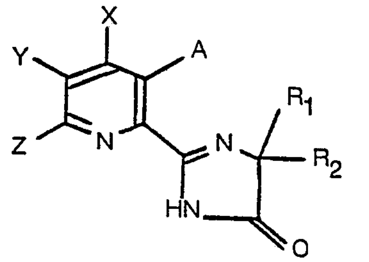

Reactants

1. $CH_3MgBr$
2. Phenyl lithium
3. Sodium trimethyl phosphonoacetate
4. $NaBH_4$
5. $HON{=}C(CH_3)$

A in Formula I

1. $COCH_3$
2. $COC_6H_5$
3. $COCH{-}COOCH_3$
   |
   $P(OCH_3)_2$
   ||
   O
4. $CH_2OH$
5. $COON{=}C(CH_3)_2$

wherein $R_1$, $R_2$, X, Y and Z are as described above.

Formula (I) compounds, wherein A is $COOR_3$ and $R_3$ represents a salt-forming cation such as an alkali metal, alkaline earth metal, ammonium or aliphatic ammonium and $R_1$, $R_2$, X, Y and Z are as described above, can be prepared by dissolving the formula (I) 2-(2-imidazolin-2-yl)pyridine acid in an appropriate solvent and, thereafter, treating the solution of the acid with one equivalent of salt-forming cation. For compounds in which the salt-forming cation is an inorganic salt such as sodium, potassium, calcium, barium or the like, the formula (I) acid may be dissolved or dispersed in water or a lower alcohol or mixtures thereof. One equivalent of the salt-forming cation generally in the form of the hydroxide, carbonate, bicarbonate or the like, but preferably as the hydroxide, is admixed with the solution of the formula (I) acid. After several minutes, the formula (I) compound, wherein $R_3$ is the inorganic salt-forming cation, generally precipitates and can be recovered from the mixture by either filtration or through azeotropic distillation with an organic solvent such as dioxane.

To prepare the formula (I) compound in which A is $COOR_3$ and $R_3$ is ammonium or organic ammonium, the formula (I) acid is dissolved or dispersed in an organic solvent such as dioxane, tetrahydrofuran or the like, and the mixture treated with one equivalent of ammonia or the amine or the tetraalkylammonium hydroxide. Among the amines which may be used in the above-said reaction are: methylamine, ethylamine, *n*-propylamine, isopropylamine, *n*-butylamine, isobutylamine, *sec*-butylamine, *n*-amylamine, *iso*-amylamine, hexylamine, heptylamine, octylamine, nonylamine, decylamine, undecylamine, dodecylamine, tridecylamine, tetradecylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-*n*-propylamine, diisopropylamine, di-*n*-amylamine, diisoamylamine, dihexylamine, diheptylamine, dioctylamine, trimethylamine, triethylamine, tri-*n*-propylamine, triisopropylamine, tri-*n*-butylamine, triisobutylamine, tri-*sec*-butylamine, tri-*n*-amylamine, ethanolamine, *n*-propanolamine, isopropanolamine, diethanolamine, N,N-diethylethanolamine, N-ethylpropanolamine, N-butylethanolamine, allylamine, *n*-butyenyl-2-amine, *n*-pentenyl-2-amine, 2,3-dimethylbutenyl-2-amine, dibutenyl-2-amine, *n*-hexenyl-2-amine, propylenediamine, tallowamine, cyclopentylamine, cyclohexylamine, dicyclohexylamine, piperidine, morpholine, and pyrrolidine. Among tetralkylammonium hydroxides contemplated methyl, tetraethyl, trimethylbenzylammonium hydroxides. In practice, after several minutes, the ammonium or organic ammonium salt precipitates and can be separated from the solution by any convenient means, as by filtration or centrifugation. Additionally, the reaction mixture may be concentrated, and the remaining solvent removed with hexane, and the residue then dried to recover the ammonium or organic ammonium formula (I) salt. The above reactions may be graphically illustrated as follows:

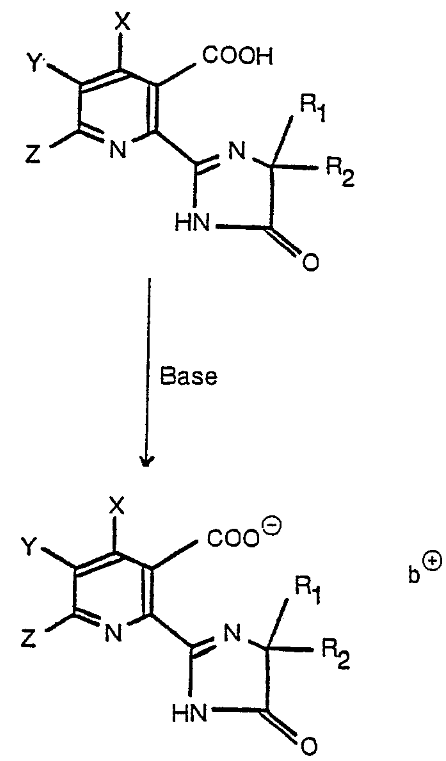


wherein $R_1$, $R_2$, X, Y and Z are as described above, and b is the salt forming cation.

When $R_1$ and $R_2$ represent different substituents, the carbon to which $R_1$ and $R_2$ are attached is an asymmetric center, and the products (as well as their intermediates) exist in *d*- and *l*- forms as well as *dl*-forms.

It should also be understood that the 2-(2-imidazolin-2-yl)pyridines and quinolines represented by formula (I) in which B = H may be tautomeric, while for convenience, they are depicted by a single structure identified as formula (I), they may exist in either of the isomeric forms illustrated as follows:

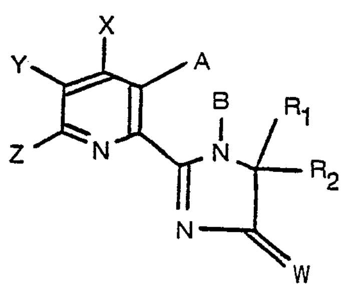
(I) OR
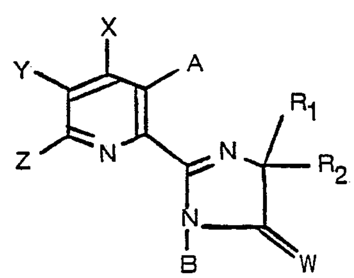
(I)

wherein A, W, X, Y, Z, $R_1$ and $R_2$ are as hereinabove defined and B is H. As such, both isomeric forms of the 2-(imidazolin-2-yl)pyridines and 2-(2-imidazolin-2-yl)quinolines are meant to be included under the formula (I) definitions.

One general method for the preparation of the formula (I) compounds involves the reaction of a quinolinic anhydride of formula (XVI) hereinbelow, with an appropriately substituted $\alpha$-aminocarbonitrile of formula (XVII), hereinbelow, to yield a mixture of the monoamides of quinolinic acid of formula (IX) and formula (X).

This reaction is carried out at a temperature between about 20°C and 70°C and preferably between about 35°C and 40°C in an inert solvent, such as tetrahydrofuran, methylene chloride, ether, chloroform, toluene or the like. The thus-formed acids are then cyclized to the corresponding pyrrolopyridine acetonitrile, depicted by formula (XI), by heating the reaction mixture with an excess of acetic anhydride in the presence of a catalytic amount of sodium acetate or potassium acetate.

In general, the above reaction is carried out by treating the reaction mixture with acetic anhydride, acetyl chloride, thionyl chloride or the like and heating said mixture to a temperature between about 20°C and 100°C. Hydration of the thus-formed pyrrolopyridine acetonitrile formula (XI) is carried out by treating said acetonitrile with a strong acid such as sulfuric acid. This reaction yields the formula (XII) pyrrolopyridine acetamide. Although the addition of a non-miscible solvent such as methylene chloride, chloroform or the like is not essential to the conduct of the above described reaction, addition of such a

solvent to the reaction mixture is generally preferred. Said reaction is usually carried out at a temperature between about 10°C to 70°C.

The cyclization of the formula (XII), hereinbelow; pyrrolopyridine acetamide yields the tricyclic formula (III) imidazopyrrolopyridinediones which are intermediates for the imidazolinyl nicotinic acids and esters of the present invention referred to above and represented by formula (Ib).

The product of this reaction is predominantly the desired imidazopyrrolopyridinedione (85%) together with the isomer of formula (IIIa). Mixtures of this ratio of the two isomers generally give substantially isomerically pure nicotinate product.

The cyclization reaction is preferably conducted at a temperature of from 80°C to 150°C in the presence of a base such as sodium or potassium hydride or an acid such as an aromatic sulfonic acid and a solvent which will form an azeotropic mixture with water, permitting virtually immediate removal thereof from the reaction mixture as it is formed. Among the solvents which may be employed are toluene, benzene, xylenes and cyclohexane. Bases which may be used include alkali metal hydroxides, alkali metal hydrides, alkali metal oxides, tertiary amines such as diisopropyl ethylamine, 1,5-diazabicyclo[3.4]nonene-5,1,5-diazobicyclo[5.4.0]undecene-5,1,4-diazabicyclo[2.2.2]octane, tetramethylguanidine, potassium fluoride and quaternary ammonium hydroxide, such as trimethylbenzyl ammonium hydroxide and strongly basic ion exchange resins.

Finally, acidic reagents which can be employed herein include aromatic sulfonic acids, such as *p*-toluenesulfonic acid, *β*-naphthalenesulfonic acid, naphthalenedisulfonic acid, and the like.

The mixture of compounds of formula (III) and formula (IIIa) is then converted to formula (Ib), as discussed above, with an alkali metal alkoxide and alcohol.

The above reactions are graphically illustrated on Flow Diagram I below, when X, Y, Z, $R_1$, $R_2$ and $R_3$ are as defined above.

FLOW DIAGRAM I

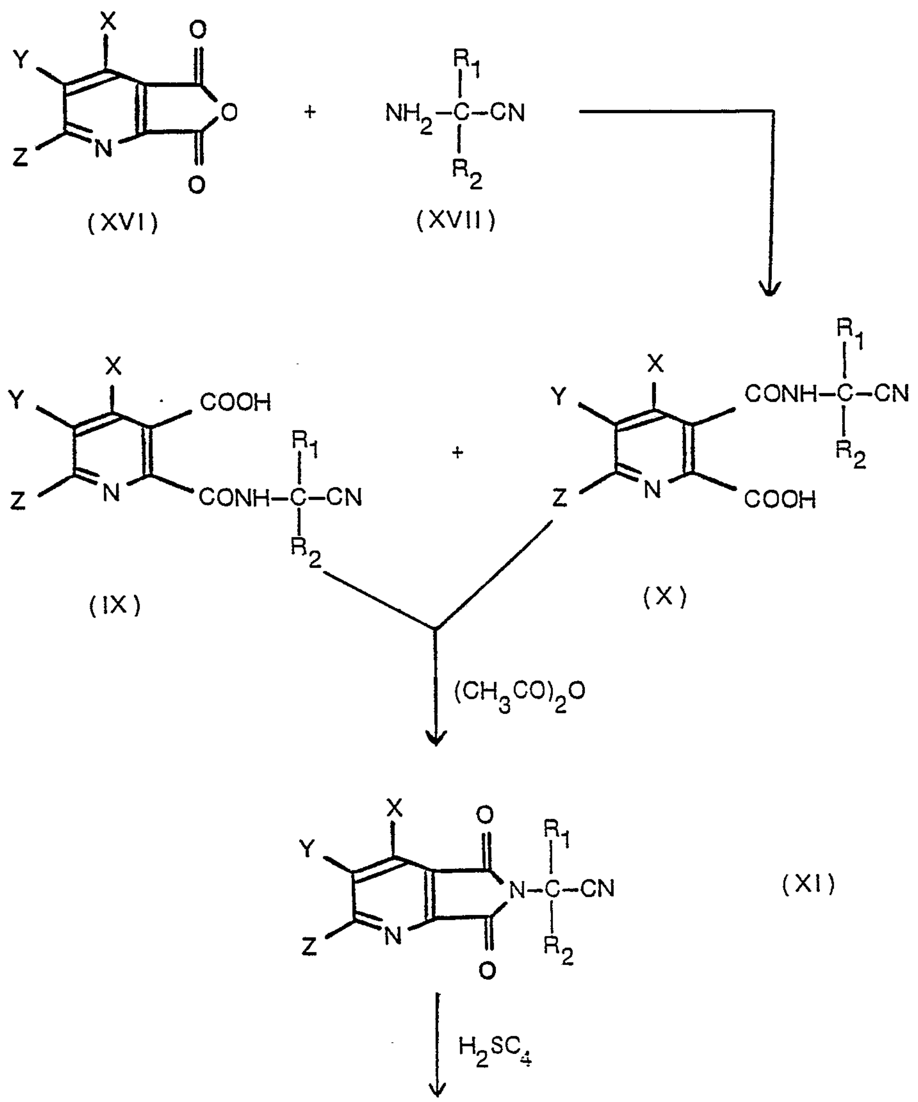


(XVI) (XVII)

(IX) (X)

(XI)

FLOW DIAGRAM I (Continued)

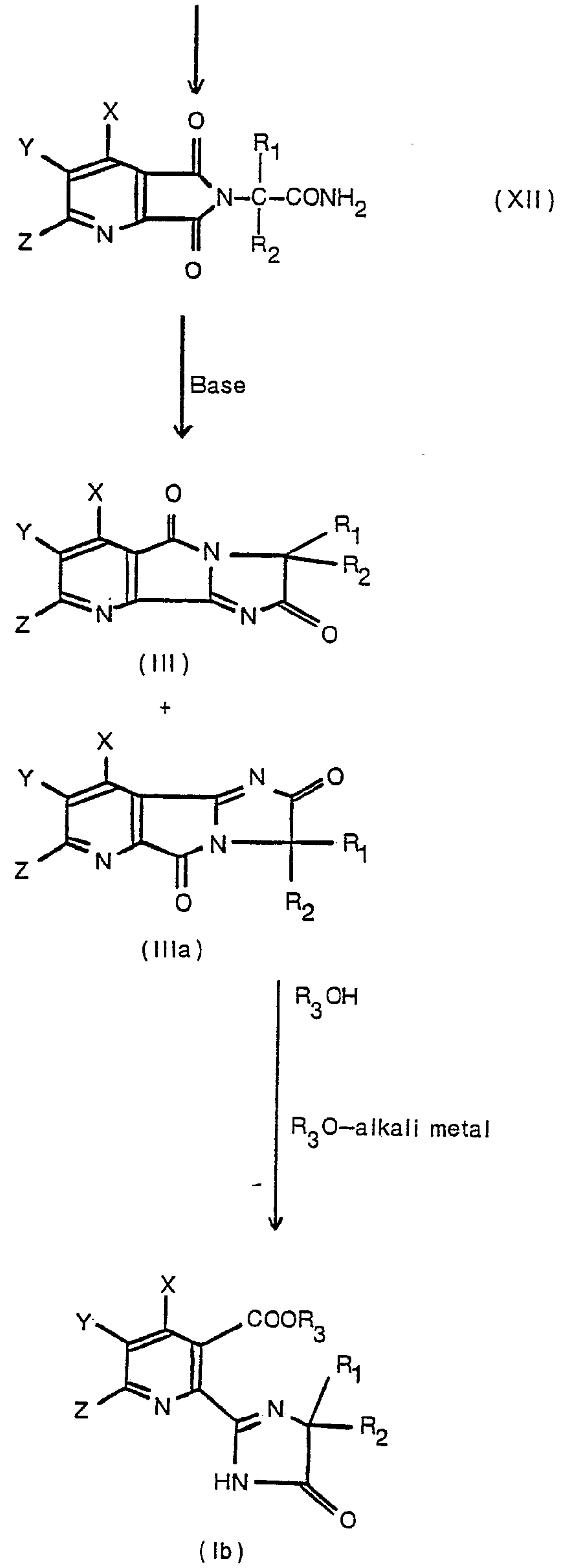


(XII)

(III)

+

(IIIa)

(Ib)

Another general method for the preparation of the formula (I) pyridine derivatives involves the reaction of a quinolinic anhydride of formula (XVI), with an appropriately substituted $\alpha$-amino-carboxylic acid such as $\alpha$-methylvaline represented by formula (XIX), preferably in a ketonic solvent such as acetone under a blanket of nitrogen to yield an isomeric mixture of the formula (XX) and

formula (XXI) acids. The mixture is then treated with acetic anhydride and a catalytic amount of sodium acetate at an elevated temperature to give the dihydrodioxopyrrolopyridine acid formula (XXII). Reaction of the thus-formed acid with a thionyl halide, such as thionyl chloride or thionyl bromide, in the presence of an organic solvent such as toluene, xylene, benzene or the like, at an elevated temperature, i.e., 80°C to 150°C, gives the formula (XXIII) acid halide corresponding to the formula (XXII) acid. Treatment of this acid halide with excess ammonia then yields the formula (IV) dihydrodioxopyrrolopyridine acetamide. The reaction is preferably carried out in the presence of an aprotic solvent.

Reaction of the formula (IV) acetamide with 1,8-diazabicyclo[5.4.0]undec-7-ene in an inert organic solvent such as toluene or xylene at an elevated temperature between about 80°C and 125°C gives the formula (III) imidazopyrrolopyridinedione which may be heated with morpholine or an appropriate $NH_2R_6$ amine to yield the 2-(2-imidazolin-2-yl)nicotinamides. These reactions are illustrated as Flow Diagram II.

FLOW DIAGRAM II

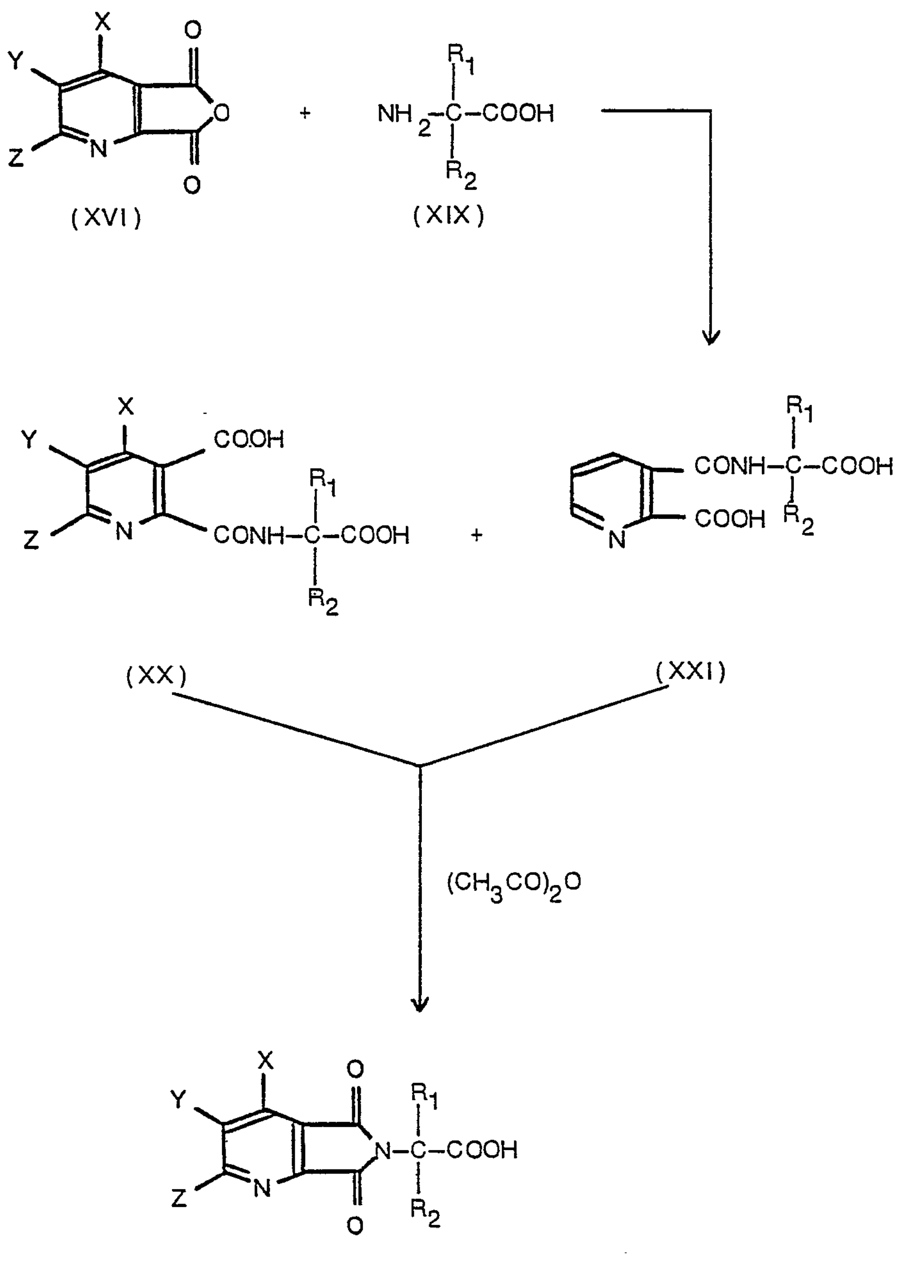


(XVI) (XIX)

(XX) (XXI)

(XXII)

FLOW DIAGRAM II (Continued)

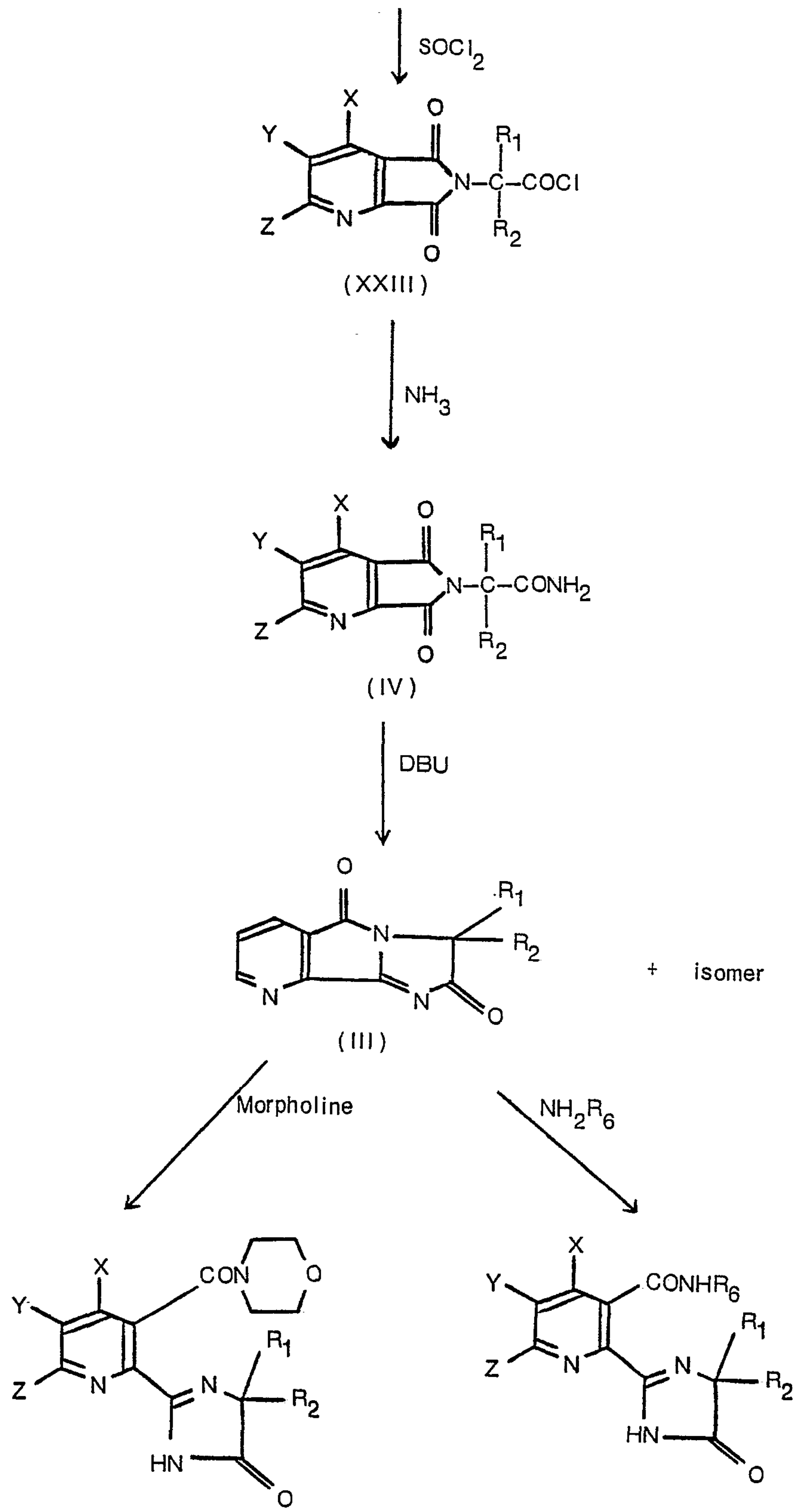


(XXIII)

(IV)

(III)

In another general procedure, the 2-(2-imidazolin-2-yl)pyridine acids and esters of formula (I), can be prepared by reacting the 2-carboalkoxynicotinoyl chloride of formula (XIV), hereinbelow, preferably as the methyl ester and preferably in the form of the hydrochloride salt, with the appropriate amino-carboxamide depicted by formula (XIII). The reaction yields the carbamoyl picolinate, formula (XV), and

is preferably carried out in an inert blanket of gas such as nitrogen. The reaction mixture is generally maintained at a temperature below 30°C during the reaction period.

The thus-formed carbamoyl picolinate, formula (XV), hereinbelow, can then be dispersed in an inert non-protic solvent such as xylene or toluene and heated to about 50°C to 130°C with 1,5-diazabicyclo-[5.4.0]undec-5-ene. The reaction yields a mixture of the formula (III) and formula (IIIa), imidazopyrrolopyridinedione isomers which can be used without separation in the following reaction, wherein the reaction mixture is treated with an alkali metal alkoxide, in the presence of an alcohol to yield a mixture of the imidazolinyl nicotinate and the imidazolinyl picolinate. The desired formula (Ib) nicotinate can be readily separated from the picolinate by neutralizing the reaction mixture, preferably with glacial acetic acid, concentrating the neutralized solution and chromatographing the resulting residue on silica gel in ether.

Conversion of the imidazolinyl nicotinate esters to the corresponding acids or acid addition salts can be readily achieved by the methods previously described. Similarly, the imidazolinyl nicotinic acids can be converted to the corresponding alkali metal, ammonium, or organic ammonium salts by the methods previously described.

Preparation of the formula (I) acids and esters, by the route described above, is graphically illustrated in Flow Diagram III below.

FLOW DIAGRAM III

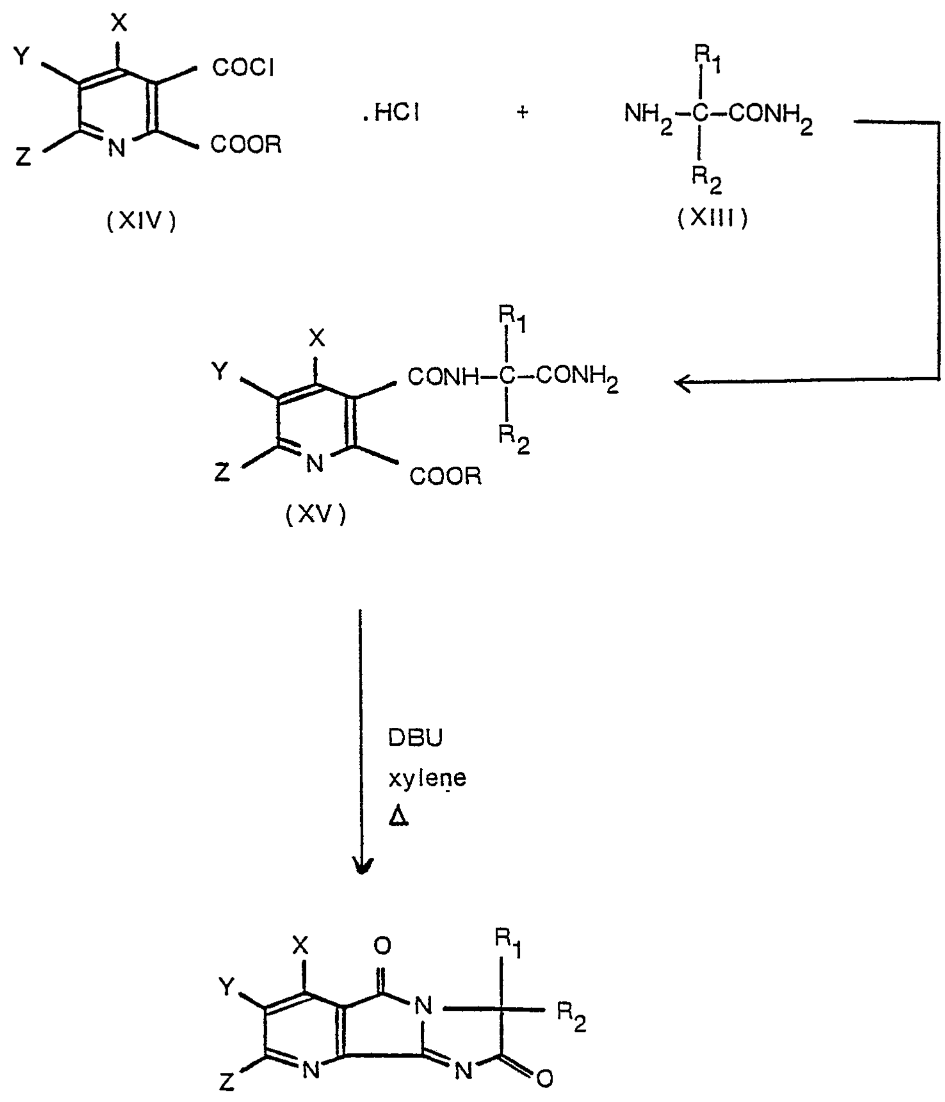


(XIV) (XIII) (XV)

+

(III)

FLOW DIAGRAM III (Continued)

+

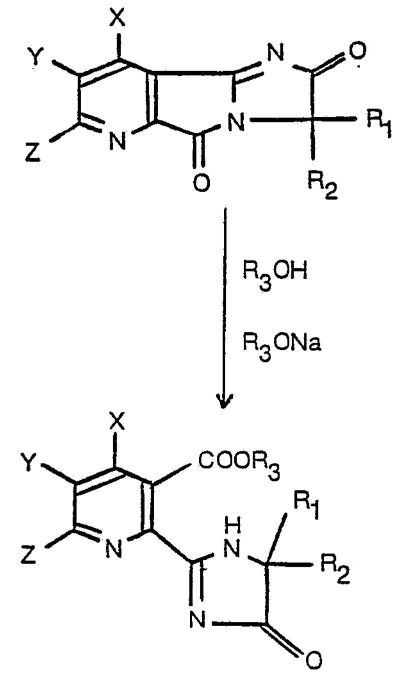


(IIIa)

(Nicotinate)

(Ib)

+

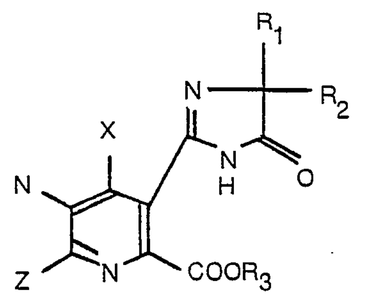

(Picolinate)

Advantageously, many of the formula (II) 2-(2-imidazolin-2-yl)quinoline derivatives of the present invention can be prepared by the procedures described above for the preparation of the formula (I) 2-(2-imidazolin-2-yl)pyridine compounds. For example, the formula (XXXVI) 2-(2-imidazolin-2-yl)quinoline-carboxylate esters, wherein $R_3$ represents a substituent other than hydrogen or a salt-forming cation, and $R_1$, $R_2$, X, L, M, Q and $R_7$ are as described above, can be prepared by reacting the formula (XXXVII) dione, with an appropriate alcohol and alkali metal alkoxide at a temperature between about 20°C and 50°C. In these reactions, as with similar reactions in which the formula (I) pyridines are prepared, the alcohol can function both as a reactant and a solvent. As such, a secondary solvent is not required, but can be employed if so desired. When a secondary solvent is used, it is preferable to employ a non-protic solvent such as tetrahydrofuran or dioxane. The reaction may be illustrated as follows:

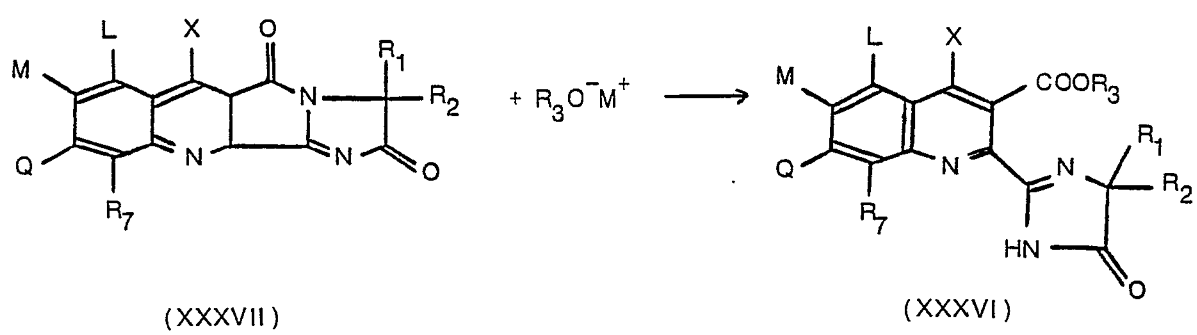


(XXXVII) (XXXVI)

where M is an alkali metal; X is hydrogen, halogen, hydroxyl or methyl, with the proviso that when one of L, M, Q, or $R_7$ is a substituent other than hydrogen, halogen, $C_1$—$C_6$ alkyl, or $C_1$—$C_4$ alkoxy, X is hydrogen; L, M, Q and $R_7$ each represent members selected from the group consisting of hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $C_1$—$C_4$ alkylamino, diloweralkylamino, chlorophenyl, methylphenyl or phenoxy substituted with one Cl, $CF_3$, $NO_2$ or $CH_3$ group, with the proviso that only one of L, M, Q, or $R_7$ may represent a substituent other than hydrogen, halogen, $C_1$—$C_4$ alkyl or $C_1$—$C_4$ alkoxy, and $R_1$, $R_2$ and $R_3$ are as described above.

The formula (XXXVI) 2-(2-imidazolin-2-yl)quinolinecarboxylate esters can also be prepared from a formula (XXXVIII) dioxopyrroloquinoline acetamide, wherein $R_1$, $R_2$, X, L, M, Q and $R_7$ are as described above, by cyclization thereof with a strong base, such as 1,5-diazabicyclo[5.4.0]undec-5-ene (DBU) in the presence of an inert organic solvent such as xylene or toluene to give the crude imidazopyrroloquinolinedione of formula (XXXVII). The reaction mixture is heated to a temperature between 100°C and 150°C, and water removed from the reaction mixture using a Dean-Stark water separator. At least one equivalent of alcohol $R_3OH$ represented by formula (V); wherein $R_3$ is a substituent as described above, but excluding hydrogen and salt-forming cations, is then added to the reaction mixture, and the thus prepared mixture heated to reflux at a temperature between 100°C and 150°C to yield the formula (XXXVI) ester. The reaction may be graphically illustrated as follows:

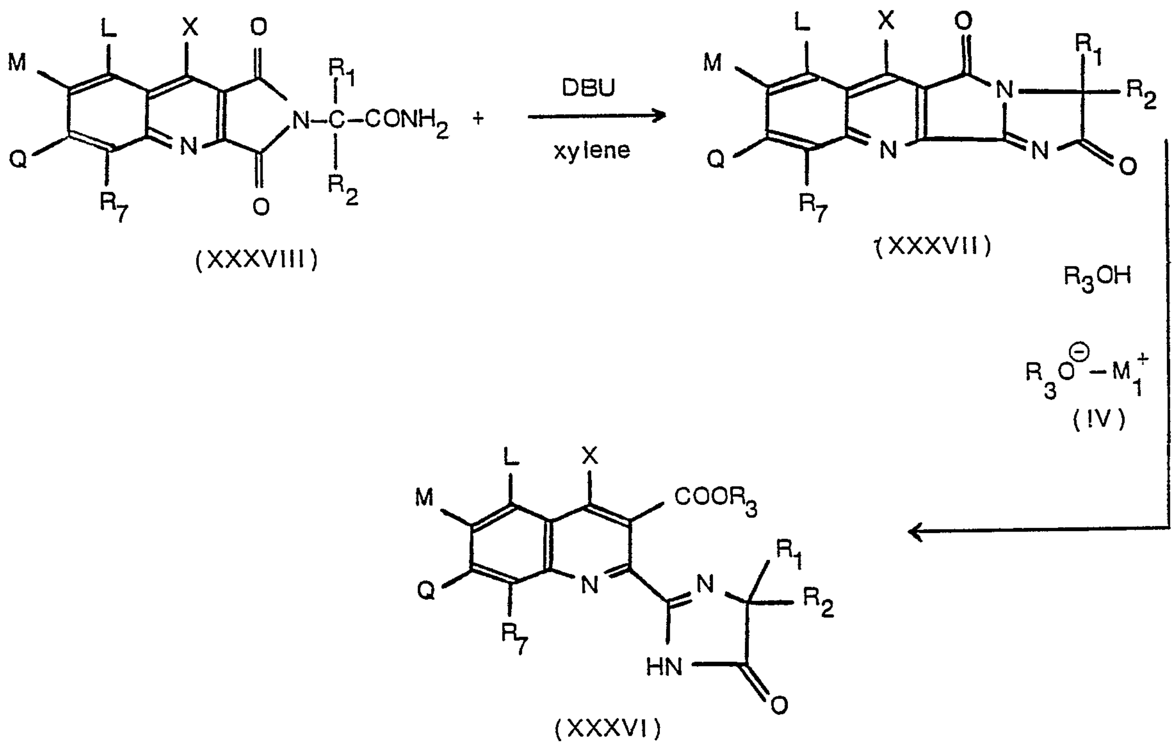

(XXXVIII) (XXXVII) (IV) (XXXVI)

wherein $R_1$, $R_2$, $R_3$, X, L, M, Q and $R_7$ are as described above.

The formula (XXXVI) 2-(2-imidazolin-2-yl)quinolinecarboxylate esters can also be prepared by cyclization of a carbamoyl quinolinecarboxylate ester, represented by formula (XXXIX), with phosphorus pentachloride at an elevated temperature between about 60°C and 100°C. The reaction is generally conducted in the presence of an inert organic solvent, such as toluene or benzene, and yields the hydrochloride salt of the formula (XXXVI) 2-(2-imidazolin-2-yl)quinolinecarboxylate ester. Treatment of the thus formed hydrohalide salt with base, such as sodium or potassium carbonate, then yields the formula (XXXVI) 2-(2-imidazolin-2-yl)quinolinecarboxylate ester. The carbamoyl quinolinecarboxylate ester, formula (XXXIX), used in the above reaction may be shown as follows:

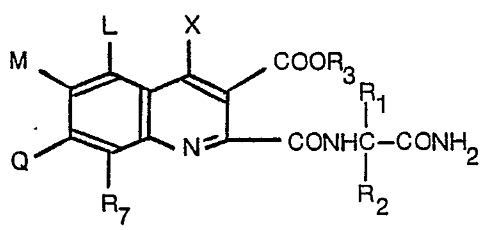

(XXXIX)

wherein $R_3$ is a substituent as described above, but excluding hydrogen or a salt-forming cation and $R_1$, $R_2$, X, L, M, Q and $R_7$ are as described above.

The 2-(2-imidazolin-2-yl)quinolinecarboxylate esters of formula (XXXVI) are also formed by cyclization of the carbamoyl quinolinecarboxylate esters represented by formula (XXXIX), and hvaing the structure:

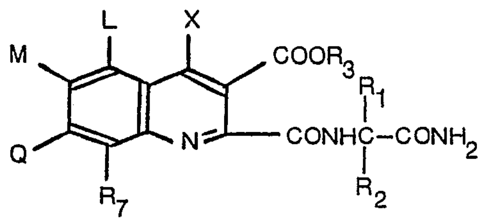

(XXXIX)

wherein $R_3$ is as described above excepting that hydrogen and salt-forming cations are excluded and $R_1$, $R_2$, X, L, M, Q and $R_7$ are as described above. Cyclization of the carbamoyl quinolinecarboxylate ester is achieved by reaction thereof with a mixture of phosphorus pentachloride and phosphorus oxychloride. The reaction mixture is stirred for several hours at a temperature between about 15°C and 35°C, and the $POCl_3$ then removed *in vacuo*. The residue from this treatment is dispersed in an organic solvent such as toluene. The solvent is then separated from the resulting mixture, and the remaining residue dispersed in water heated to between 80°C and 100°C. After cooling, the pH of the aqueous mixture is adjusted to 5—6 with sodium or potassium bicarbonate, and the product extracted into methylene chloride to give the 2-(2-imidazolin-2-yl)quinolinecarboxylate ester, formula (XXXVI).

The formula (XXXVI) quinoline ester in which $R_3$ is as described above, but excluding hydrogen or salt-forming cations, and $R_1$, $R_2$, X, L, M, Q and $R_7$ are as described above, is readily converted to its corresponding acid addition salt by reaction of said ester with at least one equivalent of a strong acid. Strong mineral acids such as hydrochloric acid, sulfuric acid, and hydrobromic acid are employed although organic acids may also be used. In practice, it is also found that the reaction proceeds most satisfactorily when conducted in the presence of an inert organic solvent such as ether, chloroform, methylene, chloride, or mixtures thereof. Sulfuric acid salts are generally prepared by this procedure, but substituting a lower aliphatic alcohol for the above-mentioned solvents.

Preparation of the formula (II) 2-(2-imidazolin-2-yl)quinoline derivatives wherein A is COOH: B is hydrogen; W is oxygen and $R_1$, $R_2$, X, L, M, Q and $R_7$ are as defined, with the proviso that X, L, M, Q and $R_7$ are not halogen or nitro, can be achieved by hydrogenolysis of the benzyl ester of the formula (XXXVI) 2-(2-imidazolin-2-yl)quinolinecarboxylate. The reaction involves dispersion of the benzyl ester in an organic solvent, such as described above for the hydrogenolysis of the formula (XV) benzyl ester in the 2-(2-imidazolin-2-yl)pyridine series, and treatment of the thus-prepared reaction mixture with hydrogen gas in the presence of a catalyst such as palladium or platinum on a carbon carrier. The hydrogenolysis is generally carried out at a temperature between about 20°C and 50°C.

The acids of the formula (II) 2-(2-imidazolin-2-yl)quinoline derivatives in which A is COOH; B is hydrogen; W is oxygen and $R_1$, $R_2$, X, L, M, Q and $R_7$ are as described above, are also prepared by reaction of a formula (XXXVI) ester, in which $R_3$ is a substituent as described above, but excluding hydrogen and salt-forming cations and $R_1$, $R_2$, X, L, M, Q and $R_7$ are as described, with at least one equivalent of strong aqueous base, such as an aqueous solution of an alkali metal hydroxide, at a temperature between 20°C and 50°C. The mixture is cooled and then adjusted to pH 6.5 to 7.5 with a strong mineral acid. Such treatment yields the desired acid.

Preparation of the formula (II) 2-(2-imidazolin-2-yl)quinoline derivatives in which A is $COOR_3$; $R_3$ is a salt-forming cation; B is hydrogen; W is oxygen and $R_1$, $R_2$, L, M, Q and $R_7$ are as described above, can be prepared by dissolving an acid, represented by formula (II) wherein A is COOH; B is hydrogen; W is oxygen and $R_1$, $R_2$, L, M, Q and $R_7$ are as described above, in an appropriate solvent and treating the thus prepared mixture with at least one equivalent of a salt-forming cation. The reaction is essentially the same as described for the preparation of the formula (I) pyridines in which A is $COOR_3$ and $R_3$ is a salt-forming cation.

It should also be understood that the imidazolinyl quinolinecarboxylic acids and esters depicted by formula (II) in which B is H may be tautomeric.

It should also be understood that when $R_1$ and $R_2$ represent different substituents on the formula (II) 2-(2-imidazolin-2-yl)quinoline derivatives and the formula (XXXVII) imidazolpyrroloquinolinediones, the carbon to which $R_1$ and $R_2$ are attached is an asymmetric center, and the products (as well as their intermediates) exist in *d*- and *l*- forms as well as *dl* forms.

Cyclization of the formula (XXXVIII) imidazopyrroloquinoline acetamide yields the tetracyclic formula (XXXVII) and formula (XXXVIIa) imidazopyrroloquinolinediones which are intermediates for the formula (II) 2-(2-imidazolin-2-yl)quinolinecarboxylate acids and esters.

The product of this reaction is predominantly the desired imidazolpyrroloquinolinedione together

with a small amount of the formula (XXXVIIa) isomer. Treatment of the isomeric mixture with an alkali metal alkoxide gives substantially isomerically pure quinolinecarboxylate product.

The cyclization reaction is preferably conducted at a temperature of from 80°C to 150°C in the presence of a base such as sodium or potassium hydride or an acid such as an aromatic sulfonic acid and a solvent which will form an azeotropic mixture with water, permitting virtually immediate removal thereof from the reaction mixture as it is formed. Among the solvents which may be employed are toluene, benzene, xylenes and cyclohexane. Bases which may be used include alkali metal hydroxides, alkali metal hydrides, alkali metal oxides, tertiary amines such as diisopropyl ethylamine, 1,5-diazabicyclo[3.4]nonene-5,1,5-diazobicyclo[5.4.0]undecene-5,1,4-diazabicyclo[2.2.2]octane, tetramethylguanidine, potassium fluoride and quaternary ammonium hydroxide, such as trimethylbenzyl ammonium hydroxide and srongly basic ion exchange resins.

Finally, acidic reagents which can be employed herein include aromatic sulfonic acids, such as $p$-toluenesulfonic acid, $\beta$-naphthalenesulfonic acid, naphthalenedisulfonic acid, and the like.

The reactions are graphically illustrated in Flow Diagram IV.

FLOW DIAGRAM IV

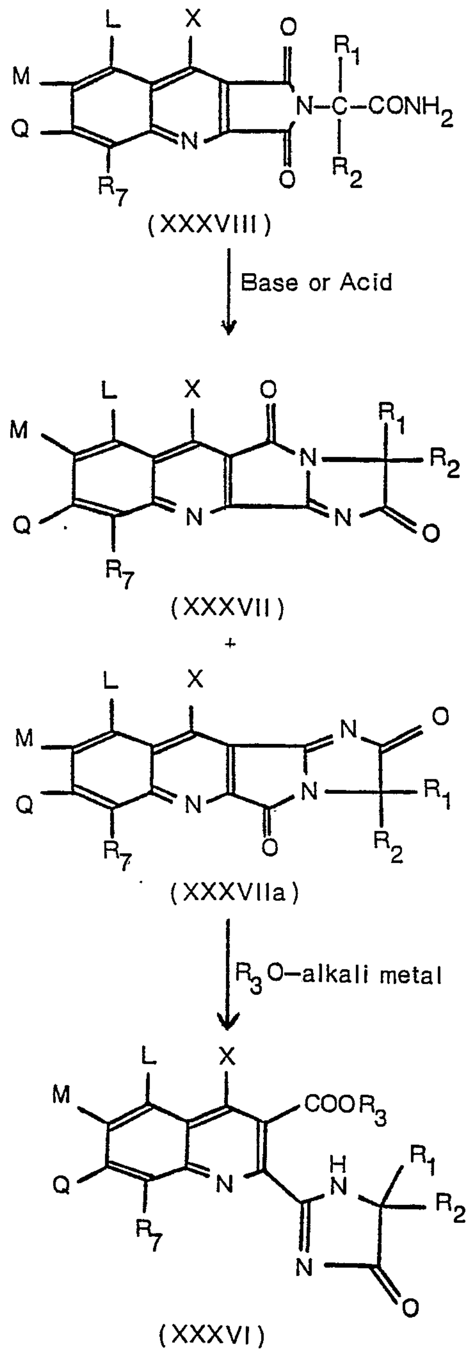


(XXXVIII)

(XXXVII)

+

(XXXVIIa)

(XXXVI)

Several routes are employed to prepare pyrroloquinoline acetamides represented by formula (XXXVIII). Preferably hydration of the pyrroloquinoline acetonitrile of formula (XXXX) is accomplished by treatment with a strong acid such as sulfuric acid. This reaction yields the formula (XXXVIII) pyrroloquinoline acetamide. Although the addition of a non-miscible solvent such as methylene chloride, chloroform or the like is not essential to the conduct of the above described reaction, addition of such a solvent to the reaction mixture may be preferred. Said reaction is usually carried out at a temperature between 10°C to 70°C.

Alternatively, the pyrroloquinoline acetamide of formula (XXXVIII) may be obtained by the Diels-Alder cycloaddition reaction between substituted anthranils of formula (XXXXI) and the dioxopyrroline acetamide of formula (XXXXII). These reactions are carried out in a wide temperature range: below 130°C significant amounts of the intermediate aldehyde of formula (XXXXIII) are obtained, but at 130°C—200°C the pyrroloquinoline acetamide of formula (XXXVIII) is formed. Alternatively, the intermediate aldehyde of formula (XXXXIII) may be isolated and cyclized in refluxing xylene in the presence of an acid catalyst, such as *p*-toluene sulfonic acid. This reaction yields the desired formula (XXXVIII) pyrroloquinoline acetamide. These reactions may be graphically illustrated as shown below on Flow Diagram V.

FLOW DIAGRAM V

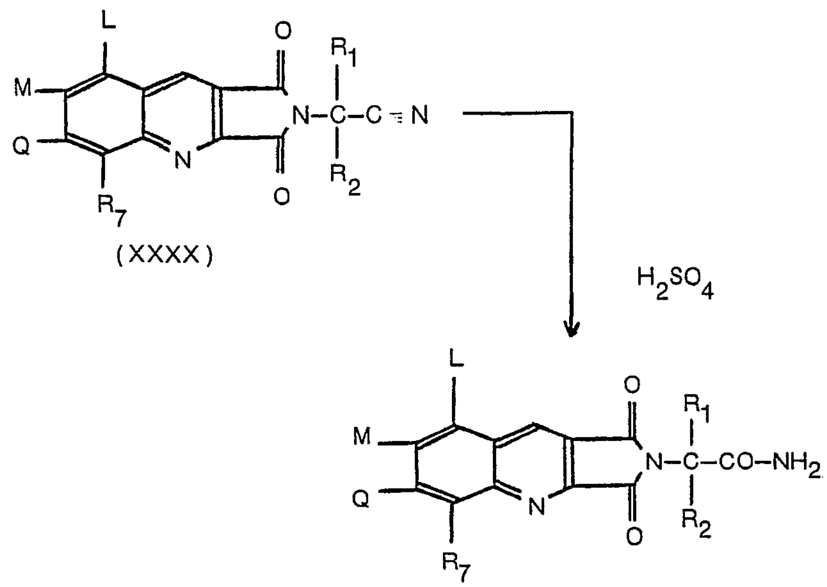


(XXXX)

(XXXVIII)

FLOW DIAGRAM V (Continued)

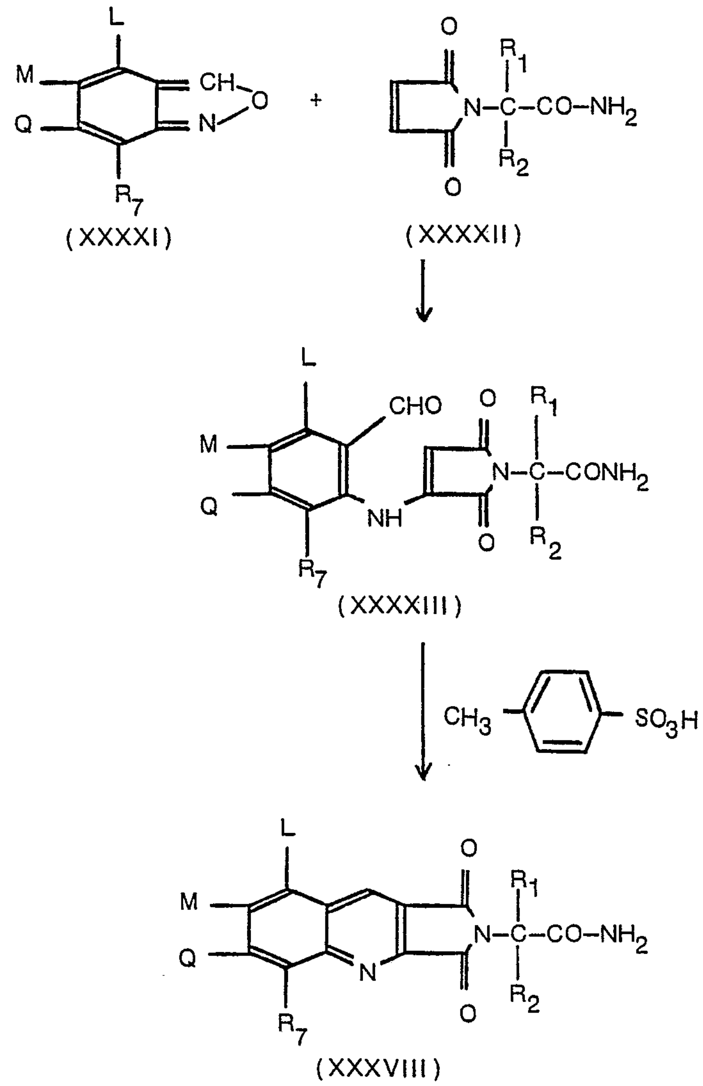

(XXXXI) (XXXXII)

(XXXXIII)

(XXXVIII)

A variety of routes may also be employed to obtain the formula (XXXX) pyrroloquinoline acetonitriles depending on the nature of the L, M, Q and $R_7$ substituents.

The formula (XXXX) pyrroloquinoline acetonitriles may be prepared by reacting the appropriately substituted anhydride (XXXXIV) with an appropriately substituted $\alpha$-aminocarbonitrile of formula (XVII), to yield a mixture of the monoamides of the formula (XXXXVa) and formula (XXXXVb) acids.

This reaction is carried out at a temperature between 20°C and 70°C and preferably between about 35°C and 40°C in an inert solvent, such as tetrahydrofuran, methylene chloride, ether, chloroform, toluene or the like. The thus-formed acids are then cyclized to the corresponding pyrroloquinoline acetonitrile, depicted by formula (XXXX). This is accomplished by heating the reaction mixture to a temperature between about 75°C and 150°C with an excess of acetic anhydride in the presence of a catalytic amount of sodium acetate or potassium acetate.

In general, the above reaction is carried out by treating the reaction mixture with acetic anhydride, acetyl chloride, thionyl chloride or the like, and heating said mixture to a temperature between about 20°C and 100°C. The above reactions are illustrated graphically on Flow Diagram VI below, wherein $R_1$, $R_2$, X, L, M, Q and $R_7$, are as defined above.

FLOW DIAGRAM VI

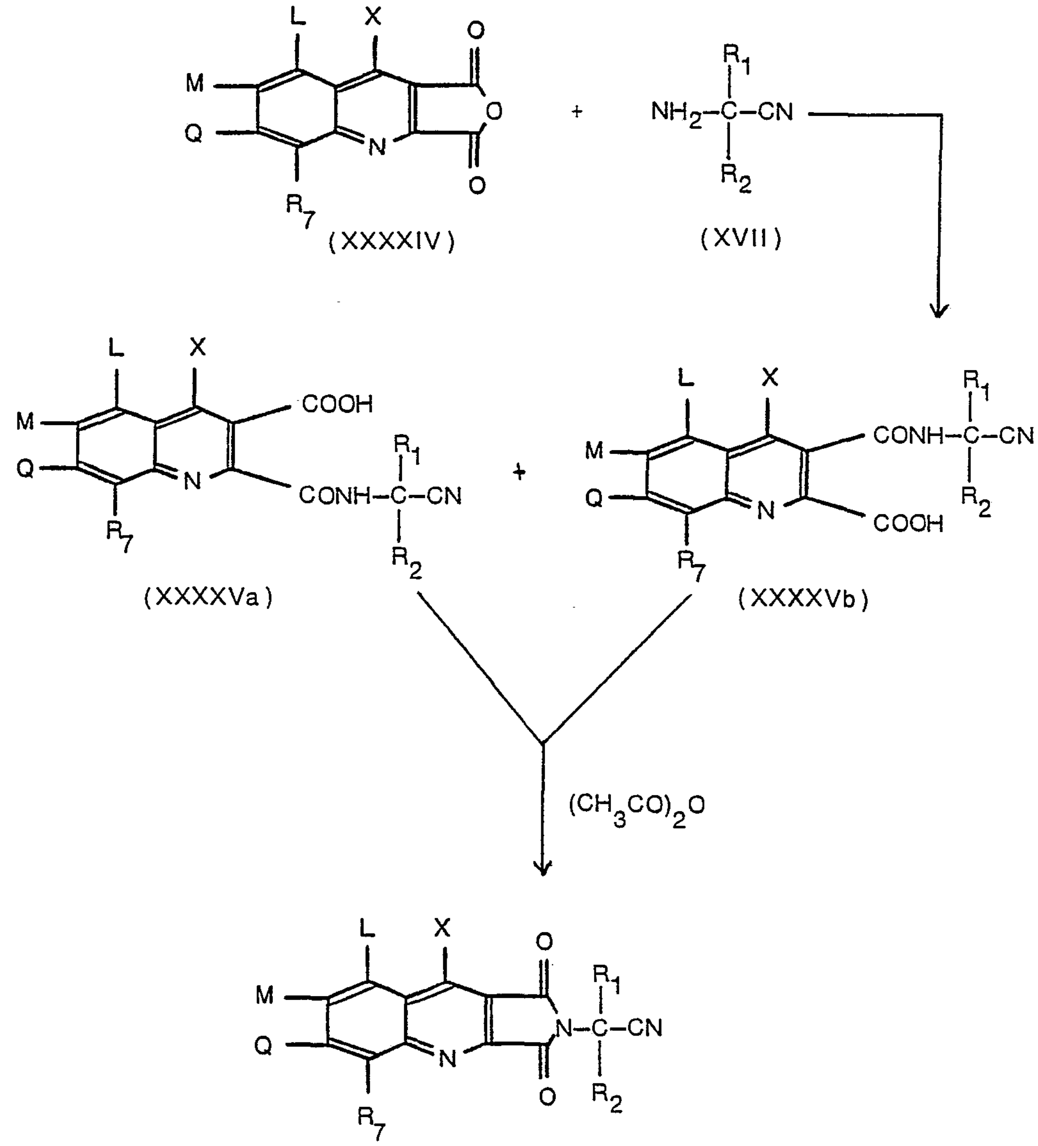


(XXXXIV) (XVII)

(XXXXVa) (XXXXVb)

(XXXX)

A preferred route to pyrroloquinoline acetonitrile of formula (XXXX) is the Diels-Alder thermal cycloaddition reaction of an appropriately substituted maleimide of formula (XXXXVI) with an appropriately substituted anthranil. In this route X must be hydrogen.

The outcome of this reaction is dependent upon the reaction temperature. Intermediate of formula (XXXXVII) is obtained at −55°C. As the reaction mixture is heated further, to temperatures between 55°C and 130°C, the intermediate of formula (XXXXIII) aldehyde is obtained. If the reaction is conducted in the presence of an aprotic solvent such as *o*-dichlorobenzene, and the reaction mixture heated to between 140°C and 200°C, the reaction yields the pyrroloquinoline acetonitrile of formula (XXXX). The reaction is illustrated on Flow Diagram VII below.

FLOW DIAGRAM VII

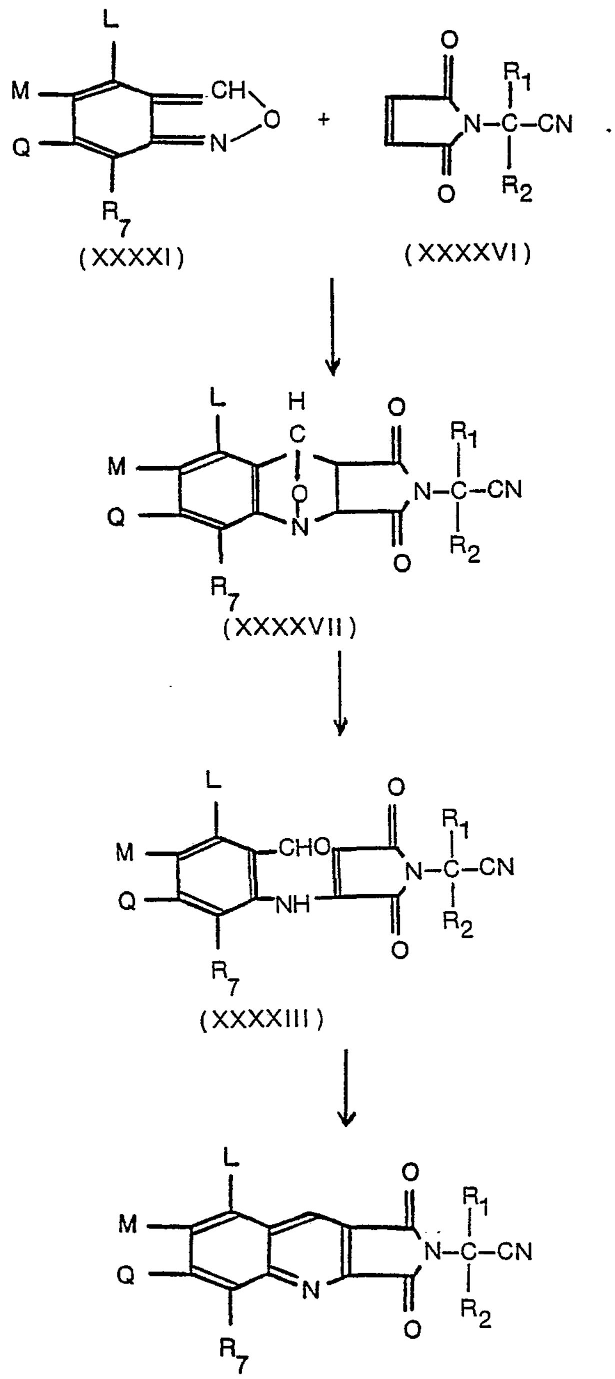

(XXXXI) (XXXXVI)

(XXXXVII)

(XXXXIII)

(XXXX)

The above route is particularly effective when L, M, Q and $R_7$ groups are electronegative, e.g., halogens, nitro, $CF_3$, $SO_2CH_3$, CN.

A variation of this procedure involves the reaction of an *o*-aminoacetal of formula (XXXXVIII) with the appropriately substituted formula (XXXXX) maleimide or dioxopyrroline acetamide, in the presence of an aprotic solvent such as xylene or toluene at a temperature between about 50°C and 130°C. These reactions are graphically illustrated below.

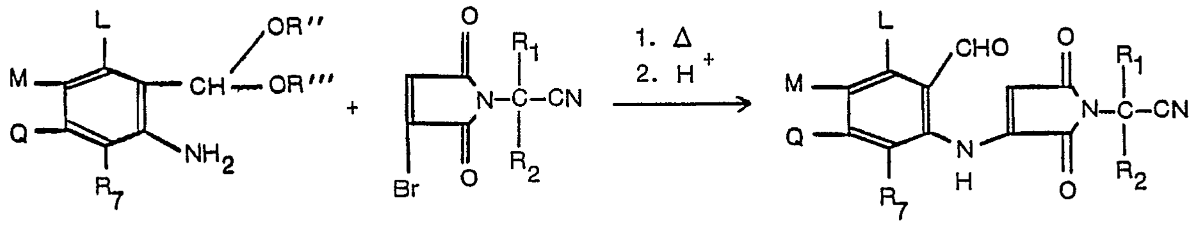


(XXXXVIII) (XXXXX) (XXXXXI)

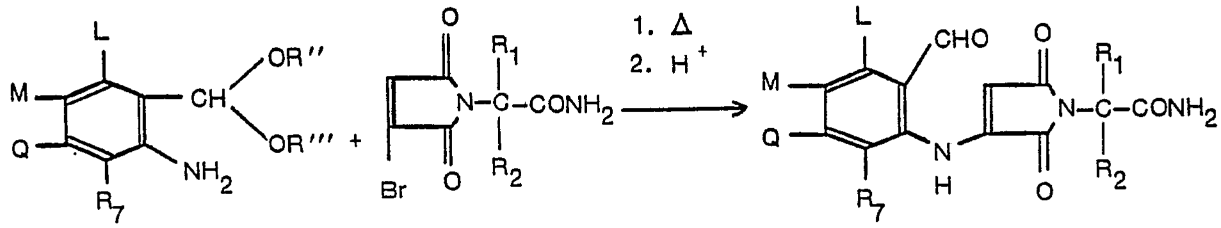


(XXXXVIII) (XXXXIII)

Yet a further variation, useful for synthesis from anilines bearing electron donor substitution in the L, M, Q and $R_7$, positions or one halogen or $CF_3$ function, is the interaction of *o*-alkyl or arylthiomethylanilines formula (XXXXIX) with a bromomaleimide of formula (XXXXX) to give the maleimide of formula (XXXXXI) which is oxidized to the maleimide of formula (XXXXXII). The maleimide of formula (XXXXXII) is then cyclized in an acid catalyzed reaction to yield the pyrroloquinoline acetonitrile of formula (XXXX). The reactions are graphically illustrated below. $R_1$, $R_2$, L, M, Q and $R_7$ are as defined above.

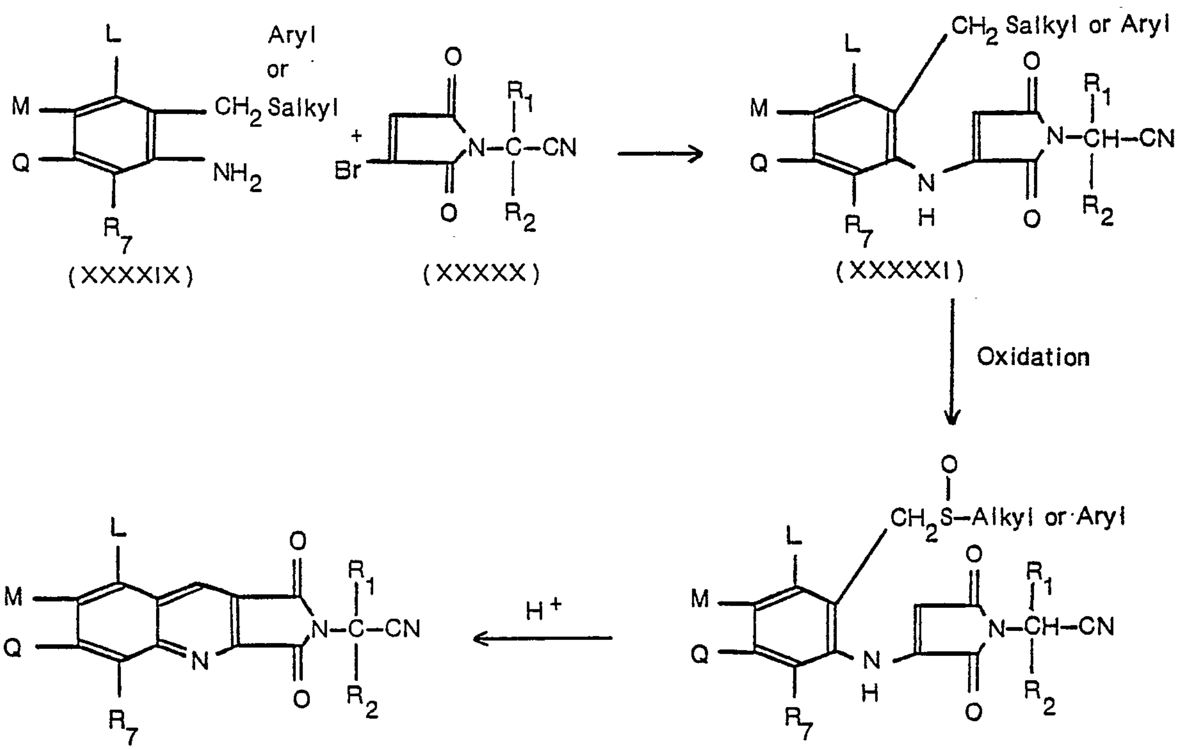


(XXXXIX) (XXXXX) (XXXXXI)

(XXXX) (XXXXXII)

Compounds of formula (XXXX) bearing electron donor substitution on the L, M, Q and $R_7$ positions, such as alkyl, alkoxy, alkylthio, dialkylamino, hydroxy and a single halogen may be prepared by reaction of an appropriately substituted *o*-aminobenzyl alcohol of formula (XXXXXIII) or anthranilic acid of formula (XXXXXIV) with a bromo (or chloro) maleimide of formula (XXXXX).

This reaction is conducted in the presence of a protic solvent, such as isopropyl or *t*-butylalcohol at 0°C to 30°C to yield respectively, the hydroxymethylanilinomaleimide of formula (XXXXXV) or dioxopyrrolinyl anthranilic acid of formula (XXXXXVI). A variety of base acceptors can be employed in

the above reactions, e.g., alkaline earth metal hydroxides, such as $Ba(OH)_2$, BaO or sodium acetate. However, the reactions in many instances proceed satisfactorily without the aid of the base acceptor.

Oxidation of the formula (XXXXXV) alcohol to the formula (XXXXIII) aldehyde may be accomplished using a wide variety of oxidizing agents exemplified by pyridiniumchlorochromate in methylene chloride or activated manganese dioxide in *t*-butanol. Cyclization of the aldehyde of formula (XXXXIII) to the pyrroloquinoline acetonitrile of formula (XXXX) is achieved by one of the methods described above, such as heating said aldehyde to between 140°C and 200°C in the presence of an aprotic solvent.

Cyclization of the *o*-anilinocarboxylic acid of formula (XXXXXVI) to the acetoxyquinoline of formula (XXXXXVII) is accomplished with acetic anhydride, triethylamine and 4-dimethylaminopyridine at ambient temperatures. Mild reductive elimination gives the pyrroloquinoline acetonitrile of formula (XXXX). Hydrolysis in warm aqueous acetic acid gives formula (XXXX) where X = OH, further reaction with phosphorus oxychloride and pyridine, affords compounds where X is chlorine. These reactions are illustrated in Flow Diagram VIII below.

FLOW DIAGRAM VIII

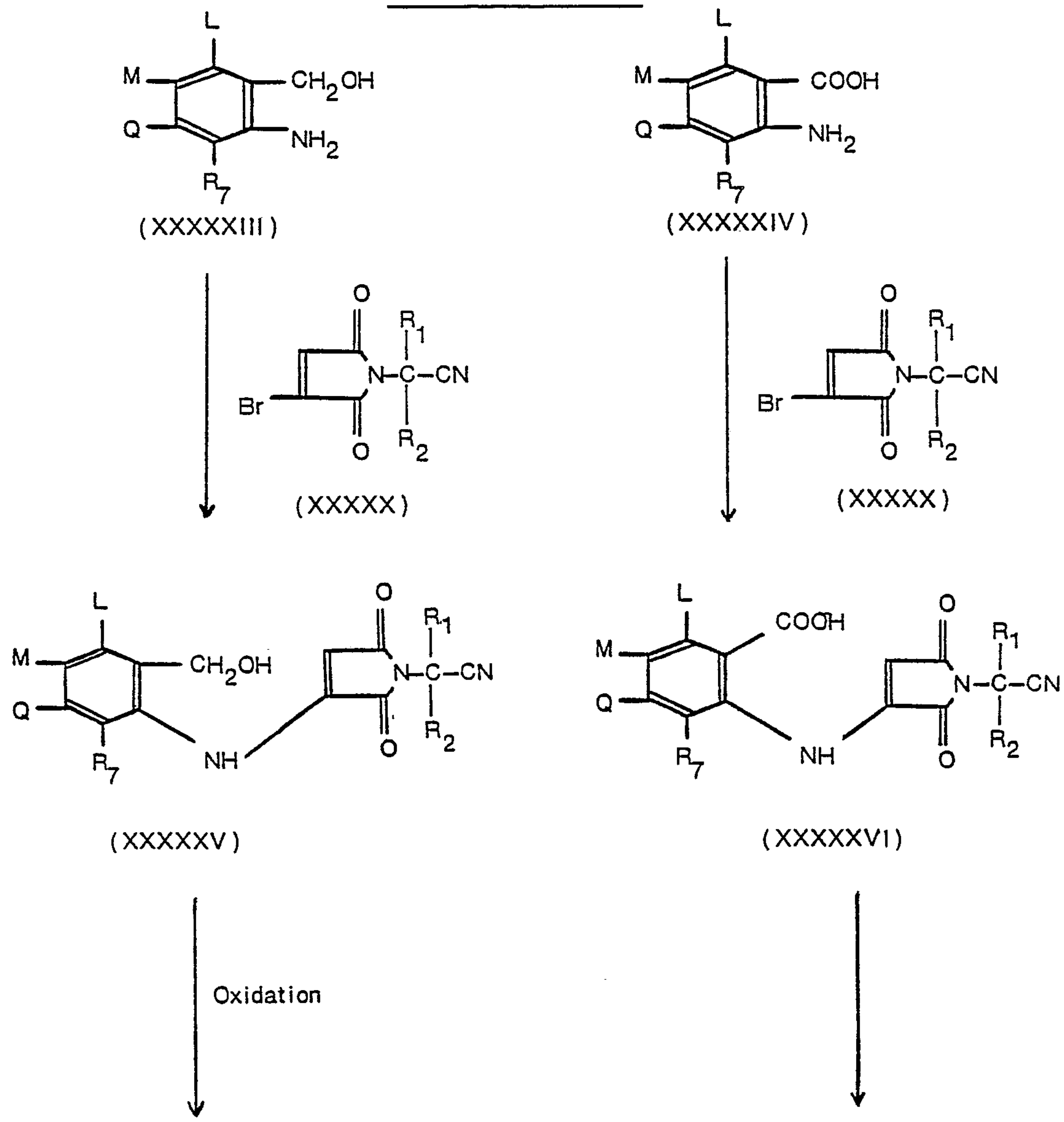


(XXXXXIII) (XXXXXIV)

(XXXXX) (XXXXX)

(XXXXXV) (XXXXXVI)

FLOW DIAGRAM VIII (Continued)

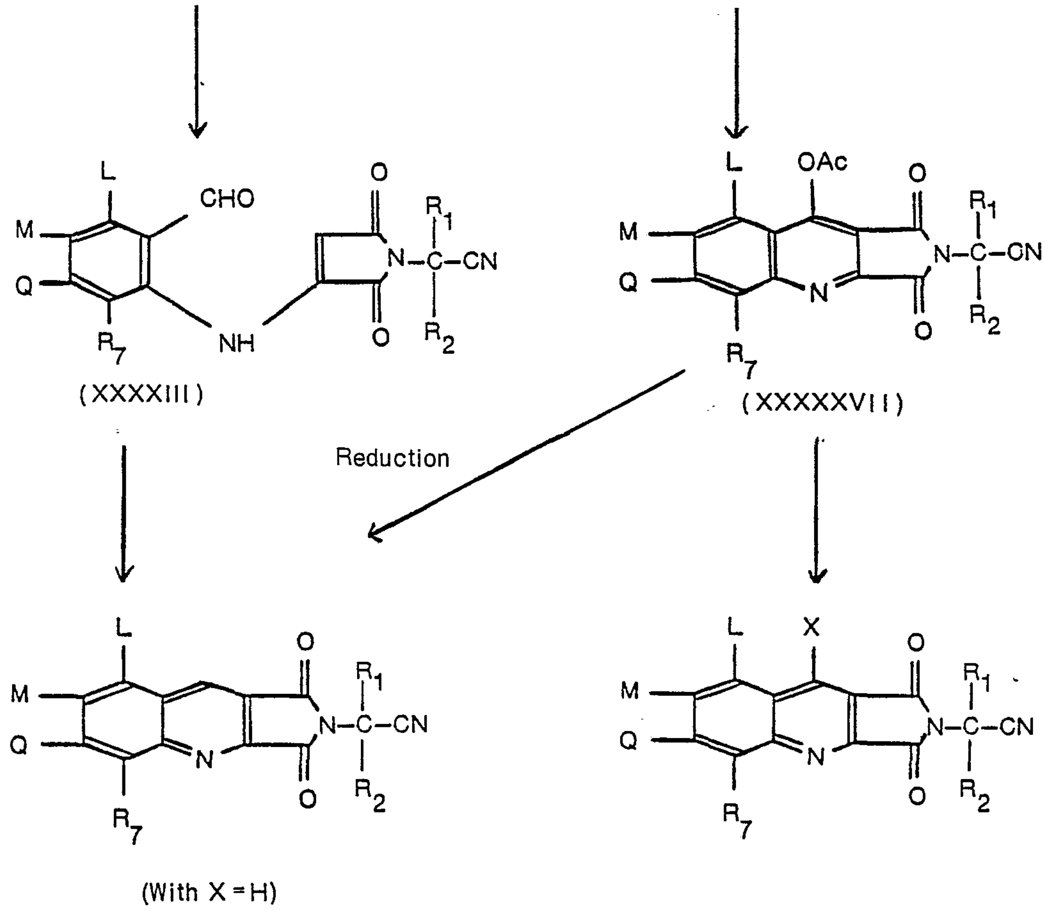


(XXXXIII) (XXXXXVII)

(With X=H)

(XXXX) (XXXX)

Another route to the 2-(2-imidazolin-2-yl)quinoline compounds of formula (II), particularly useful in synthesizing analogs in which the A group is varied, employs the 2-(2-imidazolin-2-yl)quinoline of formula (XXXXXIX). This intermediate is prepared from quinolinecarboxylic acid of formula (XXXXXVIII) which is converted to the acid chloride or anhydride and then reacted with either an appropriately substituted $\alpha$-aminocarbontrile of formula (XVII) to give the formula (XXXXXX) nitrile, or with the aminoamide of formula (XXXXXXI) to give the carboxamidoamide of formula (XXXXXXII). Cyclization of said carboxyamidoamide is accomplished by the previously discussed procedures, although cyclization with sodium hydride in the presence of xylene is preferred. Introduction of a variety of groups, A, is made possible by treatment of the 2-(2-imidazolin-2-yl)quinoline of formula (XXXXXIX) with metallating reagents. Most organo metallic agents have some effect when two moles are used with the formation of a dianion, but the yield and reaction composition is dependent upon the nature of the organometallic reagent, reaction solvent, reaction temperature and the electrophile used to quench the reaction. In practice organometallic reagents such as alkyl lithiums are preferred and anion formation is obtained with methyl, *n*-butyl, *sec*-butyl and *tert*-butyl lithium. Phenyl lithium and lithium diisopropylamide may also be employed. Solvents must be aprotic and diethylether is preferred. Reaction temperatures employed to form the dianion range from $-78°C$ to $0°C$ with $-30°C$ to $-10°C$ preferred. Quenching with an electrophile is usually accomplished at $-78°C$ to $+20°C$. If necessary, this is followed by acid treatment. All reactions are run under an inert atmosphere. Examples of reactive electrophiles include: $CO_2$, $ClCO_2CH_3$, $(CH_3)_2NCHO$, $CH_3HCO$, $C_6H_5CHO$, $CH_3I$. The corresponding values for A in formula (IIa) are COOH, $COOCH_3$, CHO, $CH(OH)CH_3$, $CH(OH)C_6H_5$, $CH_3$. Futhermore, following the quenching of the electrophile, the products may be modified. Thus the aldehyde (A=CHO) prepared from a DMF quench, reacts with hydroxylamine to afford an oxime. The above route is also useful for the synthesis of A=COOH compounds by treatment of the dianion with carbon dioxide.

Not all X, L, M, Q and $R_7$, substituents are compatible with this organometallic process. Thus, if the above mentioned substituents are Br, I or sometimes fluorine, competitive loss of these groups occurs as well as replacement of the 3-proton of quinoline. Chlorine, however, is compatible with this

process. When L, M, Q or $R_7$, is methoxy, competitive anion formation can occur ortho to the methoxy group.

The above-described reactions are graphically illustrated on Flow Diagram IX below.

FLOW DIAGRAM IX

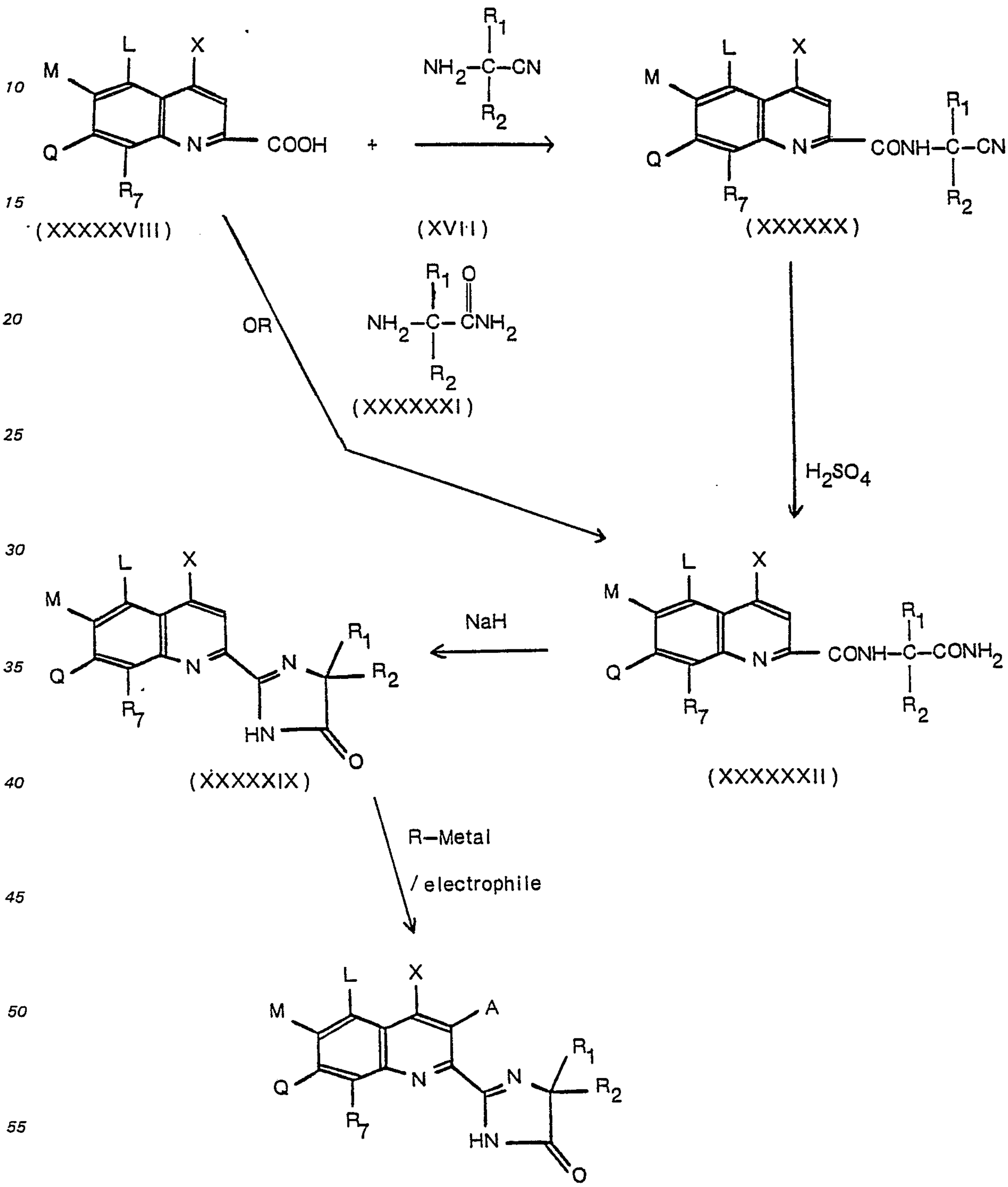


(XXXXXVIII) (XVII) (XXXXXX) (XXXXXXI) (XXXXXXII) (XXXXXIX)

(IIa)

Several functional groups, A, are prepared by an alternative approach. Thus reaction of the imidazopyrroloquinolinedione of formula (XXXVII) with $R_3OM^+$ affords the 2-(2-imidazolin-2-yl)-quinoline esters of formula (XXXVI). In the case of formula (II) compounds in which A is the $CONHCH_2CH_2OH$ group, this may be cyclized to the oxazoline group via reaction of the above-identified compound with triethylphosphite in refluxing xylene. Formula (II) derivatives, in which A is —$CONH_2$,

may be converted to the corresponding cyano derivative, i.e., A=CN, by dehydration of the —$CONH_2$ function.

The majority of compounds containing L, M, Q and $R_7$, substituents may be prepared by the procedures previously outlined; however, amino, alkylamino, and dialkylamino compounds are conveniently formed by reductive alkylation of the appropriate nitro substituent in the L, M, Q or $R_7$ position. Alkylsulfonyl compounds are most readily formed by mild oxidation of the alkylthio group in the L, M, Q or $R_7$ position at 0°C—20°C using *m*-chloroperbenzoic acid. Under these conditions, N-oxidation of the quinoline nitrogen is minimized.

Oxidation of the quinoline to the N-oxide can be accomplished by prior N-protection of the imidazolone ring with, e.g., a $COCH_3$ group; N-oxidation can now be accomplished by peracetic acid or trifluoroperacetic acid, at elevated temperatures, if necessary.

The formula (I) 2-(2-imidazolin-2-yl)pyridine and quinoline compounds as hereinabove defined are surprisingly effective for inducing and/or accelerating the maturation of cereal crops such as wheat, barley, rye, oats, rice and corn, without adversely affecting the quality of seeds and the yields thereof, when applied to said plants and/or to the soil containing the seeds of said plants in amounts of from 0.0001 kg/ha to 0.01 kg/ha and preferably 0.0002 kg/ha to 0.005 kg/ha.

The method of using formula (I) compounds to accelerate and/or induce maturing, and thus allow the earlier than usual harvesting of cereal crops is, or could be, quite important to the users thereof. Thus, for instance, in any given location the so treated cereal crops may be harvested well ahead of their usual time of harvest, and the land so freed is immediately available for the planting of a second crop which otherwise would not have sufficient time to grow and reach a stage or, size suitable to harvest.

Such a method, would also make it possible to allow a cereal crop to be harvested on time even though its planting may have been delayed due to adverse weather conditions.

Furthermore, the use of formula (I) compounds to accelerate the maturing of cereal crops may make possible the planting and harvesting of such crops in certain regions of the world where the growing season is normally of shorter duration than the time needed to allow said crops to mature.

Interestingly, formula (I) 2-(2-imidazolin-2-yl)-pyridines and quinolines, when applied to legumes such as soybeans, beans, peas and lentils and/or to soil containing the seeds of said plants, in amounts of from about 0.0001 kg/ha to about 0.01 kg/ha and preferably 0.002 kg/ha to 0.005 kg/ha, will improve the axillary branching and tillering of said plants. The thus-treated legumes also show increased flowering, increased pod set, and increased yields.

The formula (I) compounds, as defined above, are also quite effective for inducing branching and increasing flowering of herbaceous ornamentals such as colei, dahlias, tulips, daffodils, crocuses, crysanthemums and roses when applied to said plants and/or to the soil containing the seeds or other propagating organs of said plants in amounts of from about 0.0001 kg/ha to 0.01 kg/ha preferably 0.0002 kg/ha to 0.005 kg/ha.

Advantageously, the compounds of this invention can be formulated as solid or liquid compositions which may be dispersed in a liquid or solid diluent for application to vegetative matter.

Since the formula (I) derivatives, wherein $R_3$ is a salt forming cation, are water soluble, these compounds can simply be dispersed in water and applied as a dilute aqueous spray to the foliage of agronomic and horticultural plants or to soil containing the seeds or other propagating organs thereof. These salts also lend themselves to formulation as flowable concentrates.

The 2-($\alpha$-imidazolin-2-yl)pyridines and quinolines of formula (I) can be formulated as wettable powders, flowable concentrates, emulsifiable concentrates and granular formulations.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

A typical emulsifiable concentrate can be prepared by dissolving about 25% by weight of the active ingredient in about 65% by weight of N-methylpyrrolidone, isophorone, butyl cellosolve, methylacetate or the like and dispersing therein about 10% by weight of a nonionic surfactant such as alkylphenoxypolyethoxy alcohol. This concentrate is dispersed in water for application as a liquid spray.

When the above compounds are to be used where soil treatments are involved, the compounds may be prepared and applied as granular products. Preparation of the granular product can be achieved by dissolving the active compound in a solvent such as methylene chloride, N-methylpyrrolidone or the like and spraying the thus prepared solution on a granular carrier such as corncob grits, sand, attapulgite, kaolin or the like.

Wettable powders can be prepared by grinding together about 20% to 45% by weight of a finely divided carrier such as kaolin, bentonite, diatomaceous earth, attapulgite, or the like, 45% to 80% by weight of the active compound, 2% to 5% by weight of a dispersing agent such as sodium lignosulfonate, and 2% to 5% by weight of a nonionic surfactant, such as octylphenoxy polyethoxy ethanol, nonylphenoxy polyethoxy ethanol or the like.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

A typical emulsifiable concentrate can be prepared by dissolving about 5% to 25% by weight of the active ingredient in about 65% to 90% by weight of N-methylpyrrolidone, isophorone, butyl cellosolve,methylacetate or the like and dispersing therein about 5% to 10% by weight of a nonionic surfactant such as an alkylphenoxy polyethoxy alcohol. This concentrate is dispersed in water for application as a liquid spray.

When the compounds of the invention are to be used where soil treatments are involved, the compounds may be prepared and applied as granular products. Preparation of the granular product can be achieved by dissolving the active compound in a solvent such as methylene chloride, N-methylpyrrolidone or the like and spraying the thus prepared solution on a granular carrier such as corncob grits, sand, attapulgite, kaolin or the like.

The granular product thus prepared generally comprises about 3% to 20% by weight of the active ingredient and about 97% to 80% by weight of the granular carrier.

A composition for the treatment of a one hectare plot, to increase the crop yield of cereal grains or legumes grown thereon, comprises from 100 to 500 liters of water; from 0.01 to 0.001 kg of a compound having the structure:

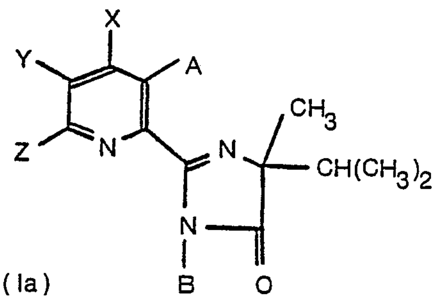

(Ia) ;

wherein B is hydrogen, CO-alkyl $C_1$—$C_6$ or CO-phenyl; A is $COOR_3$ where $R_3$ is as described in formula (I) above; X is hydrogen and Y and Z each represent hydrogen, $C_1$—$C_6$ alkyl, $C_1$—$C_4$-alkoxy, halo, $C_1$—$C_4$-haloalkyl, or phenyl and, when taken together, YZ represent $—(CH_2)_4—$, and, from 0.01% to 3% by weight of a dispersing agent and/or non-ionic surfactant.

In order to facilitate a further understanding of the invention, the following examples are presented primarily for the purpose of illustrating certain more specific details thereof. The invention is not to be deemed limited thereby except as defined in the claims. Unless otherwise noted, all parts are by weight.

Example 1

Preparation of 5,7-Dihydro-$\alpha$-isopropyl-$\alpha$-methyl-5,7-dioxo-6*H*-pyrrolo[3,4-b]pyridine-6-acetonitrile

To a stirred solution containing 212 g quinolinic anhydride in 950 ml methylene chloride is added at a moderate rate 167 g of 2-amino-2,3-dimethylbutyronitrile. The mixture had reached the boiling point of the solution after about one quarter of the aminonitrile had been added and the rate of addition is adjusted to maintain this temperature. After the addition the solution is heated under reflux for a further 4 hours. The solution is cooled, filtered and concentrated to a thick oil. This oil is dissolved in 950 ml acetic anhydride, 6 g anhydrous sodium acetate added and the mixture distilled until the vapor temperature reached 118°C when the heating was continued under reflux for 3 hours. The mixture is concentrated *in vacuo* the residue dissolved in 500 ml toluene and again concentrated. This is repeated. The residue is slurried with a mixture of ether and hexane and the crude product which crystallizes collected (349 g). This is dissolved in 700 ml methylene chloride and filtered through a column containing 700 g silica gel and the product eluted with methylene chloride. Concentration of the eluant gave 258 g of the desired product. An analytically pure sample with mp 95—96°C can be obtained by the recrystallization of the product from ether-methylene chloride.

Using the appropriate amino nitrile and quinolinic anhydride in the above procedure, the following pyrrolopyridines are prepared:

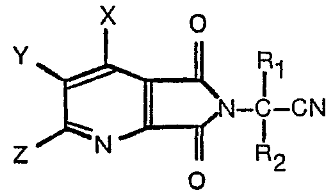

| $R_1$ | $R_2$ | X | Y | Z | mp°C |
|---|---|---|---|---|---|
| $CH_3$ | $CH_3$ | H | H | H | 119—123 |
| $CH_3$ | $C_2H_5$ | H | H | H | 95—97 |
| $CH_3$ | 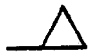 | H | H | H | 69—73 |
| $CH_3$ | $CH_2CH(CH_3)_2$ | H | H | H | oil |
| $—(CH_2)_5—$ | | H | H | H | 85—87 |
| $C_2H_5$ | $C_2H_5$ | H | H | H | 71—72.5 |
| $CH_3$ | $CH(CH_3)_2$ | $CH_3$ | H | H | 129.5—131.3 |
| $CH_3$ | $CH(CH_3)_2$ | H | H | $OCH_3$ | 108—110 |
| $CH_3$ | $CH(CH_3)_2$ | H | H | Cl | 94—96 |

Example 2

Preparation of 5,7-Dihydro-$\alpha$-isopropyl-$\alpha$-methyl-5,7-dioxo-6H-pyrrolo[3,4-b]pyridine-6-acetamide

To 330 ml concentrated sulfuric acid is added portion wise with thorough stirring 298 g finely divided nitrile so that the temperature did not go about 72°C. After the addition the temperature is adjusted to 60—65°C and maintained there for 1 1/2 hours. The mixture is cooled, quenched with ice and finally diluted to approximately 4 liters. After adding 454 g sodium acetate and cooling at 0°C for 2 hours the mixture is filtered, the solids collected and washed twice with 500 ml water containing sodium acetate followed by water to remove all the sulfuric acid. The solid is dried to give 289 g of product, mp 176—178°C. Material made in a similar way and analytically pure had mp 188—190°C.

Employing the appropriate pyrrolopyridineacetonitrile in the above procedure, the following pyrrolopyridineacetamides are prepared.

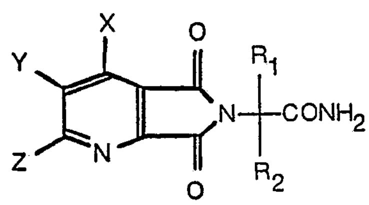

| $R_1$ | $R_2$ | X | Y | Z | mp°C |
|---|---|---|---|---|---|
| $CH_3$ | $CH_3$ | H | H | H | 203—5 |
| $CH_3$ | $C_2H_5$ | H | H | H | 158—161 |
| $CH_3$ | 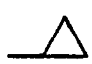 | H | H | H | 195—198 |

Example 3

Preparation of 3-Isopropyl-3-methyl-5*H*-imidazo-[1′,2′,:1,2]pyrrolo[3,4,*6*]pyridine-2-(3*H*), 5-dione

A mixture of 50 g amide and 450 ml toluene is heated under a Dean-Stark water separator to remove traces of water. To the cooled mixture is added 10.1 g of a 50% suspension of sodium hydride in mineral oil and the mixture heated under reflux for 23 hours. The hot solution is filtered, concentrated

*in vacuo* where upon the residue is crystallized. The mineral oil is removed by decantation and the solid washed with hexanes and dried *in vacuo* to give 45.5 g product which, by nmr analysis, is approximately 90% the desired isomer II and 10% the undesired isomer IIa.

A pure sample of isomer II can be obtained by recrystallizing the crude product from hexane-methylene chloride mp 107—115°C.

The cyclisation can be achieved by either the basic reagent sodium and potassium hydroxide, or the acidic reagent *p*-toluenesulfonic acid in a toluene solvent. It should be understood that a mixture of products corresponding to Structures II and IIa above is obtained and in general these are not purified but used directly for the preparation of the derived nicotinic acid esters.

Employing the appropriate pyrrolopyridine carboxamide, the following imidazopyrrolopyridines are prepared.

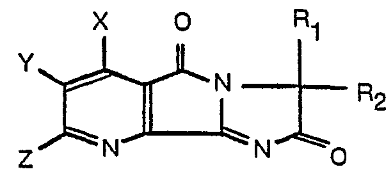

| $R_1$ | $R_2$ | X | Y | Z | mp°C |
|---|---|---|---|---|---|
| $CH_3$ | $CH_3$ | H | H | H | |
| $CH_3$ | $C_2H_5$ | H | H | H | |
| $CH_3$ | 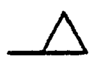 | H | H | H | |
| $—CH(CH_3)—CH_2CH_2CH_2CH_2—$ | | H | H | H | 125—130 |
| $CH_3$ | $CH(CH_3)_2$ | H | H | $OCH_3$ | 147—147.5 |

### Example 4

Preparation of 3-Isopropyl-5-H-imidazo[2′,2′:1,2]pyrrolo[3,4-6]pyridine-2(3H)-dione

A mixture containing 52 g of 3-[(1-Carbamoyl-1,2-dimethylpropyl)picolinate, 1.77 ml 1,5-diazabicyclo[5.4.0]-undec-5-ene (DBU) in 400 ml xylene is heated under reflux under a Dean-Stark water separator for 2 hours. The mixture is concentrated *in vacuo* and the residue is chromatographed on 400 g basic alumina. The mixture of desired products is eluted with methylene chloride and used without further purification.

### Example 5

Preparation of Methyl 2-(5-isopropyl-5-methyl-4-oxo-2-imidazolin-2-yl)nicotinate

To 20 ml dry methanol in which 10 mg sodium hydride had reacted is added 2.0 g of a mixture of the imidazopyrrolopyridines. After stirring for 16 hours, 0.03 g glacial acetic acid is added (to neutralize the base), the solution concentrated *in vacuo* and the residue chromatographed on silica gel in ether. The faster moving material, the desired ester, is obtained in several fractions, combined, concentrated and crystallized from acetonitrile to give the imidazolinyl nicotinate, mp 121—123.5°C. An analytically pure sample crystallized from methylene chloride hexane exhibits a melting point of from 121—122°C.

Example 6

Preparation of Methyl 2-(5-isopropyl-5-methyl-4-oxo-2-imidazolin-yl)nicotinate

This method involves the formation of the tricyclic compounds of Example 3 and 4, without isolation, directly forming the nicotinic acid esters:

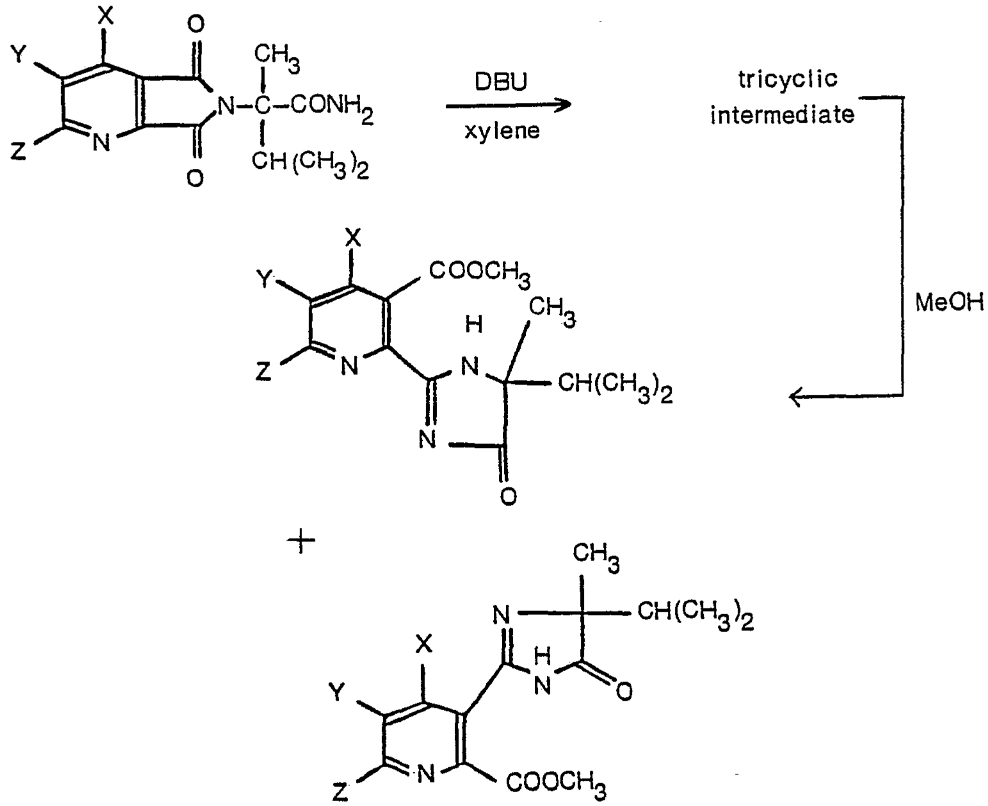


A mixture of 25 g amide and 1 ml 1,5-diazabicyclo-[5.4.0]undec-5-ene (DBU) in 500 ml xylene is heated under reflux for 1 hour under a Dean-Stark water separator. The mixture is cooled somewhat, the water separator removed, 100 ml anhydrous methanol added and the mixture heated under reflux for 1 hour. The solvents are then removed *in vacuo* and the product isolated by chromatography as described in Example 5 above to give 13.65 g product mp 120—122°C identical to that described in Example 5 above.

Example 7

Preparation of Methyl 2-(5-isopropyl 5-methyl-4-oxo-2-imidazolin-2-yl)nicotinate. Method A (Flow Diagram pg. 11)

A mixture of 13.65 g of the nicotinate and 9.69 g phosphorus pentachloride in 110 ml dry toluene is heated with stirring to 80°C. After 1 1/2 hours, the thick mixture is cooled, filtered and the solid washed with ether and dried. This is the hydrochloride salt of the desired product.

This salt is dissolved in 60 ml water; neutralized with sodium bicarbonate, the resulting precipitate removed by filtration, washed with water and air-dried to give the product identical to that prepared by the procedure of Example 5.

METHOD B

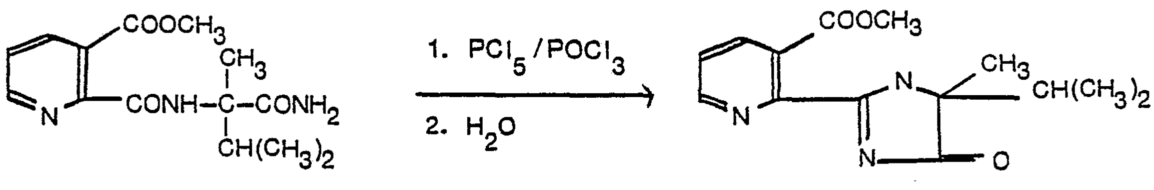


A mixture of 5.0 g nicotinate and 7.1 g phosphorus pentachloride in 40 ml phosphorus oxychloride is stirred at room temperature overnight. The phosphorus oxychloride is removed *in vacuo,* the residue suspended in 40 ml toluene and again concentrated. This is repeated. Water (40 ml) is

added to the residue and the mixture heated to reflux and held there for 1 hour. After cooling, the mixture is extracted with methylene chloride, the extract dried and concentrated to give 1.05 g of the desired product. The pH of the aqueous phase from the methylene chloride extraction is adjusted to 5—6 with sodium bicarbonate solution and the mixture extracted again with methylene chloride. The dried extract was concentrated and the residue crystallized to give a further 2.65 g of the desired product identical to that described in Example 5.

The following nicotinic acid esters are prepared by one or more of the methods described above:

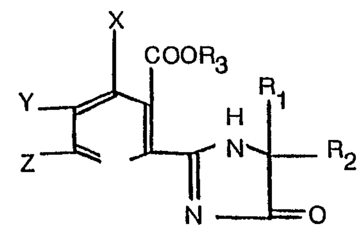

| $R_3$ | $R_1$ | $R_2$ | X | Y | Z | mp°C |
|---|---|---|---|---|---|---|
| $CH_3$ | $CH_3$ | $C_2H_5$ | H | H | H | 126.5 – 128.5 |
| $CH_2{\equiv}CH$ | $CH_3$ | $CH(CH_3)_2$ | H | H | H | 104 – 106 |
| $CH_3$ | $-CH(CH_3)-(CH_2)_4-$ | | H | H | H | 151 – 155.3 |
| $CH_2C{\equiv}CH$ | $-CH(CH_3)-(CH_2)_4-$ | | H | H | H | 117 – 120 |
| $CH_2C_6H_5$ | $-CH(CH_3)-(CH_2)_4-$ | | H | H | H | 148.5 – 151.3 |
| $CH_2C{\equiv}CH$ | $CH_3$ | $CH_3$ | H | H | H | 171 – 173 |
| $CH_3$ | $CH_3$ | $CH_3$ | H | H | H | 148 – 150 |
| $CH_2C_6H_5$ | $CH_3$ | $CH_3$ | H | H | H | 142 – 144 |
| $CH_2C_6H_5$ | $CH_3$ | $C_2H_5$ | H | H | H | 118 – 120 |
| $CH_2C{\equiv}CH$ | $CH_3$ | $C_2H_5$ | H | H | H | 138 – 140 |
| $-C_{12}H_{25}$-n | $CH_3$ | $CH(CH_3)_2$ | H | H | H | 55 – 57 |
| $-C_2H_5$ | $CH_3$ | $CH(CH_3)_2$ | H | H | H | 72 – 75 |
| $CH_2CH_2OCH_2C_6H_5$ | $CH_3$ | $CH(CH_3)_2$ | H | H | H | 90 – 92.5 |

| $R_3$ | $R_1$ | $R_2$ | X | Y | Z | mp°C |
|---|---|---|---|---|---|---|
| $-CH_2$ 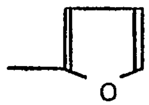 | $CH_3$ | $CH(CH_3)_2$ | H | H | H | 120.5 – 122 |
| $-CH(CH_3)_2$ | $CH_3$ | $CH(CH_3)_2$ | H | H | H | 94 – 97.5 |
| $-CH_2C_6H_5$ | $CH_3$ | $CH(CH_3)_2$ | H | H | H | 122 – 125 |
| $-CH_2-C\equiv C-C_7H_{15}$-n | $CH_3$ | $CH(CH_3)_2$ | H | H | H | oil |
| $CH_2CH_2OCH_3$ | $CH_3$ | $CH(CH_3)_2$ | H | H | H | 60 – 63 |
| $CH_2CH=CH_2$ | $CH_3$ | $CH(CH_3)_2$ | H | H | H | 81 – 84 |
| $-CH-CH=CH_2$<br>\|<br>$CH_3$ | $CH_3$ | $CH(CH_3)_2$ | H | H | H | oil |
| $-CH_2-C=CH_2$<br>\|<br>$CH_3$ | $CH_3$ | $CH(CH_3)_2$ | H | H | H | 98 – 100 |
| $CH-C\equiv CH$<br>\|<br>$CH_3$ | $CH_3$ | $CH(CH_3)_2$ | H | H | H | oil |
| $CH_2-CH=CHCH_3$ | $CH_3$ | $CH(CH_3)_2$ | H | H | H | 87 – 89 |
| $-C(CH_3)_3$ | $CH_3$ | $CH(CH_3)_2$ | H | H | H | 124 – 126 |

| $R_3$ | $R_1$ | $R_2$ | X | Y | Z | mp°C |
|---|---|---|---|---|---|---|
| 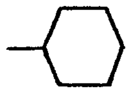 | $CH_3$ | $CH(CH_3)_2$ | H | H | H | 95.5 – 98 |
| $C_{18}H_{37}$-n | $CH_3$ | $CH(CH_3)_2$ | H | H | H | 77.3 – 79.2 |
| $CH_2C_6H_5$ | $CH_3$ | $CH(CH_3)_2$ | H | H | H | 116.5 – 119 |
| $-CH_2C_6H_5$ | $CH_3$ | $CH_2CH(CH_3)_2$ | H | H | H | 76 – 78.5 |
| $CH_3$ | $CH_3$ | $CH_2CH(CH_3)_2$ | H | H | H | 92 – 94 |
| $-C_4H_9$-n | $CH_3$ | $CH_2(CH_3)_2$ | H | H | H | 54 – 57 |
| $CH_2C{\equiv}CH$ | $CH_3$ | $CHCH(CH_3)_2$ | H | H | H | 128.5 – 131 |
| $CH_3$ | $CH_3$ | 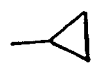 | H | H | H | 128 – 131 |
| $CH_2C_6H_5$ | $CH_3$ | 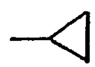 | H | H | H | 111 – 113 |
| $CH_3$ | $CH_3$ | $CH(CH_3)_2$ | H | H | $OCH_3$ | 154 – 155 |
| $CH_2-CH{=}CH-C_7H_{15}$-n | $CH_3$ | $CH(CH_3)_2$ | H | H | H | oil |
| $CH_2-C(Cl){=}CH_2$ | $CH_3$ | $CH(CH_3)_2$ | H | H | H | 73 – 77 |
| $C_6H_{13}$-n | $CH_3$ | $CH(CH_3)_2$ | H | H | H | oil |
| $CH(CH_3)CH{=}CH-CH_3$ | $CH_3$ | $CH(CH_3)_2$ | H | H | H | oil |

| R | R | R | X | Y | Z | mp°C |
|---|---|---|---|---|---|---|
| $CH_3$ | $-(CH_2)_5-$ | | H | H | H | 146 – 148 |
| $CH_2CH{=}(CH_3)_2$ | $CH_3$ | $CH(CH_3)_2$ | H | H | H | 77.5 – 79 |
| $CH_2C_6H_5$ | $-(CH_2)_5-$ | | H | H | H | 117 – 122 |
| $CH_2{\equiv}CCH_2OH$ | $CH_3$ | $CH(CH_3)_2$ | H | H | H | gum |
| $CH_2C_6H_5$ | $C_2H_5$ | $C_2H_5$ | H | H | H | 114.5 – 118 |
| $C(CH_3)C{\equiv}CH$ | $CH_3$ | $CH(CH_3)_2$ | H | H | H | 128 – 132 |
| $CH_2CH_2\overset{\oplus}{N}(CH_3)\overset{\ominus}{I}$ | $CH_3$ | $CH(CH_3)_2$ | H | H | H | 165 – 175 |
| $CH_3$ | $C_2H_5$ | $C_2H_5$ | H | H | H | 132.5 – 135.5 |
| $C(CH_3)_2C{\equiv}CH$ | $CH_3$ | $CH(CH_3)_2$ | H | H | H | 104 – 106 |
| $CH_2C{\equiv}CH$ | $CH_3$ | Δ | H | H | H | 122 – 124 |
| $CH_2C{\equiv}CH$ | $(-CH_2)-$ | | H | H | H | 164.5 – 166.5 |
| $CH_3$ | $CH_3$ | $CH(CH_3)_2$ | $CH_3$ | H | H | 114 – 115.5 |
| $CH_2C{\equiv}CH$ | $C_2H_5$ | $C_2H_5$ | H | H | H | 135.5 – 137 |
| $CH_2$ 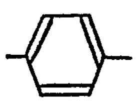 $OCH_3$ | $CH_3$ | $CH(CH_3)_2$ | H | H | H | 111 – 113 |

| $R_3$ | $R_1$ | $R_2$ | X | Y | Z | mp°C |
|---|---|---|---|---|---|---|
| 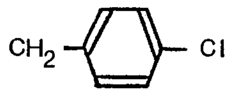 | $CH_3$ | $CH(CH_3)_2$ | H | H | H | 136 – 138 |
| 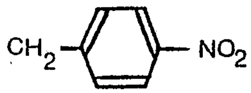 | $CH_3$ | $CH(CH_3)_2$ | H | H | H | 131.5 – 133 |
| $CH_2COOCH_3$ | $CH_3$ | $CH(CH_3)_2$ | H | H | H | 104 – 108 |
| $CH_2CH_2CH_2COOC_2H_5$ | $CH_3$ | $CH(CH_3)_2$ | H | H | H | oil |
| $CH(CH_3)COOCH_3$ | $CH_3$ | $CH(CH_3)_2$ | H | H | H | 133 – 135 |
| $CH_3$ | $CH_3$ | $CH(CH_3)_2$ | H | Br | H | 122.5 – 126 |
| $CH_2CH{=}CH{-}COOC_2H_5$ | $CH_3$ | $CH(CH_3)_2$ | H | H | H | oil |
| $(CH_2)_4COOCH_3$ | $CH_3$ | $CH(CH_3)_2$ | H | H | H | oil |
| 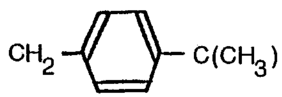 | $CH_3$ | $CH(CH_3)_2$ | H | H | H | 108 – 111 |
| 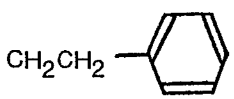 | $CH_3$ | $CH(CH_3)_2$ | H | H | H | 107 – 109 |
| 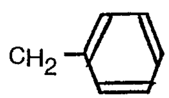 | $CH_3$ | $CH(CH_3)_2$ | $CH_3$ | H | H | 130 – 132 |

| $R_3$ | $R_1$ | $R_2$ | X | Y | Z | mp°C |
|---|---|---|---|---|---|---|
| $CH_2CH=CH-$ 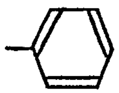 | $CH_3$ | $CH(CH_3)_2$ | H | H | H | 113 – 115 |
| $CH_2CH=C(CH_3)-$ $CH_2CH_2CH=C(CH_3)_2$ | $CH_3$ | $CH(CH_3)_2$ | H | H | H | oil |
| $(CH_2)_3C\equiv CH$ | $CH_3$ | $CH(CH_3)_2$ | H | H | H | 73 – 75 |
| $CH(C_6H_5)COOCH_3$ | $CH_3$ | $CH(CH_3)_2$ | H | H | H | oil |
| $CH_2CH_2-C(CH_3)=CH_2$ | $CH_3$ | $CH(CH_3)_2$ | H | H | H | oil |
| $(CH_2)_9CH=CH_2$ | $CH_3$ | $CH(CH_3)_2$ | H | H | H | oil |
| $CH(CH_3)C_6H_5$ | $CH_3$ | $CH(CH_3)_2$ | H | H | H | oil |
| $CH_3$ | 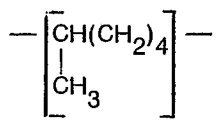 | | H | H | H | 122 – 124 |
| $CH_2-$ 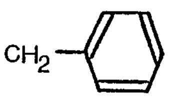 | 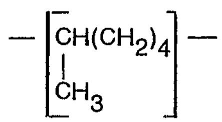 | | H | H | H | 123 – 125 |
| $CH_2C\equiv CH$ | 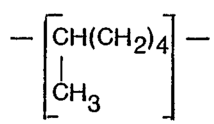 | | H | H | H | 132 – 134.5 |

| $R_3$ | $R_1$ | $R_2$ | X | Y | Z | mp°C |
|---|---|---|---|---|---|---|
| $CH_3$ | $CH_3$ | $CH(CH_3)_2$ | H | H | Cl | 102.5 – 104.5 |
| $CH_2COOCH_2CH_3$ | $CH_3$ | $CH(CH_3)_2$ | H | H | H | 86 – 90 |
| $CH_2COOH$ | $CH_3$ | $CH(CH_3)_2$ | H | H | H | 187 – 189 |
| $CH_2COOCH_2C_6H_5$ | $CH_3$ | $CH(CH_3)_2$ | H | H | H | 121.5 – 123 |
| $CH_2COOH$ | $CH_3$ | $CH(CH_3)_2$ | H | H | H | 106 – 110 |
| $CH_3$ | $CH_3$ | $CH(CH_3)_2$ | H | H | H | 110 – 112<br>$[\alpha]_D = +27.41°$ |
| $CH_3$ | $CH_3$ | $CH(CH_3)_2$ | H | H | H | 110.5 – 114<br>$[\alpha]_D = +27.28°$ |
| $CH_2C_6H_5$ | $CH_3$ | $CH(CH_3)_2$ | H | H | H | 102 – 105<br>$[\alpha]_D = +13.08°$ |
| $CH_2C_6H_5$ | $CH_3$ | $CH(CH_3)_2$ | H | H | H | 104 – 107<br>$[\alpha]_D = +12.76°$ |
| $CH_3$ | $CH_3$ | $CH(CH_3)_2$ | H | H | $N(CH_3)_2$ | 184.5 – 185.5 |

Example 8

Preparation of the Hydrochloride salt of methyl-2-(5-isopropyl-5-methyl-4-oxo-2-imidazolin-2-yl)nicotinate

To a stirred suspension of 3.0 g of the ester of Example 5 in 40 ml ether is added enough methylene chloride to obtain a solution. Dry hydrogen chloride is then passed into the solution for about 20 minutes. After 1 hour, the mixture is filtered to remove the product which is washed with ether and dried to give 1.90 g of analytically pure hydrochloride salt and melting point equal to 195—196°C.

Example 9

Preparation of 2-(5-Isopropyl-5-methyl-4-oxo-2-imidazolin-2-yl)nicotinic acid

To 22.63 g ester of Example 5 in 100 ml water is added a solution containing 3.29 g sodium hydroxide in 25 ml water and the mixture heated under reflux with stirring for 1.5 hours. After standing at room temperature overnight, 6.8 ml concentrated hydrochloric acid is added causing a heavy precipitate to form. This is removed by filtration, washed with 20 ml water, followed by 30 ml ether and dried to give 19.27 g acid, mp 168—170°C. This material is dissolved in 350 ml methylene chloride, filtered (to remove a small amount of the isomeric 2-acid) and concentrated to give 17.91 g of pure acid, mp 170—172°C The analytically pure sample is prepared by recrystallization of the material from acetonehexane, mp 170—172.5°C.

Example 10

Preparation of 2-(5-Isopropyl-5-methyl-4-oxo-2-imidazolin-2-yl)nicotinic acid

To 1.0 g of the benzyl ester in 2 ml ethanol is added 50 mg 5% palladium on carbon catalyst and the mixture shaken in an atmosphere of hydrogen until one equivalent of hydrogen has been absorbed. The catalyst is removed by filtration, the solvent removed *in vacuo* and the residue crystallized from acetone-hexane to give the acid as described in Example 9.

The following acids are made by the above methods:

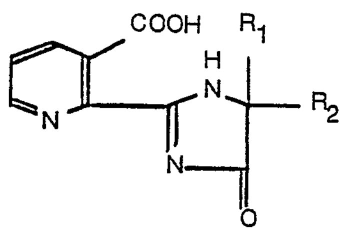

| $R_1$ | $R_2$ | mp°C |
|---|---|---|
| $CH_3$ | $C_2H_5$ | 124 – 126° |
| $-\underset{CH_3}{CH}-(CH_2)_4-$ | | 180 – 183° |
| $CH_3$ | $CH_3$ | 204 – 205.5° |
| $CH_3$ | Δ | 198 – 200° |

Example 11

Preparation of Calcium 2-(5-isopropyl-5-methyl-4-oxo-2-imidazolin-2-yl)nicotinate

To 0.98 g of the acid of Example 9 partially dissolved in 10 ml water is added, with stirring 0.18 g calcium carbonate. After 10 minutes, the solution is filtered, the filtrate concentrated and the residue treated with ether to give a crystalline product which is dried at 40°C and 25 mm pressure to give 0.88 g of the calcium salt mp 265°C.

The sodium, diisopropylammonium, and triethylammonium salts are prepared in a similar manner.

The following salts can be prepared by the above procedure using the appropriate acid and the oxide, carbonate, bicarbonate or hydroxide of the selected metal, alkali metal, alkaline earth metal, ammonia or aliphatic amine.

| $Z^+$ | mp°C |
|---|---|
| $NH_3CH(CH_3)C_6H_5$ | gum |
| $NH_4$ | sublines >168 |
| Ba/2 | >225 |
| Cu/2 | >225 |
| K | >225 |
| Li | >225 |
| Mg/2 | >225 |
| 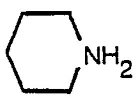 | – |
| 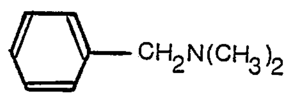 | oil |
| $H_2N-(CH_2)_6NH_3$ | oil |
| $C_{18}H_{35}NH_3$ | wax |
| $n-C_{12}H_{25}NH_3$ | 150 – 153 |
| $(CH_3)_3CCH_2C(CH_3)_2NH_3$ | – |
| $(n-C_4H_9)_2NH_2$ | – |
| $HOCH_2CH_2NH_2CH_3$ | – |
| 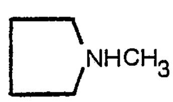 | – |
| $n-C_8H_{17}NH_3$ | – |
| 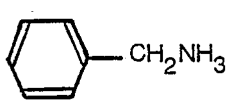 | – |
| 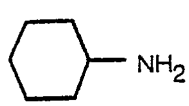 | – |
| 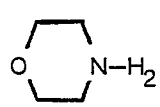 | – |

| $Z^+$ | mp°C |
|---|---|
| 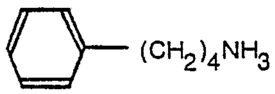 | – |
| 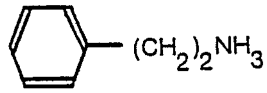 | – |
| $(CH_3O)_2CHCH_2NH_3$ | – |
| $(C_2H_5OCH_2CH_2)NH_3$ | – |
| $CH_3O(CH_2)_3NH_3$ | – |
| $(HOCH_2CH_2)_2NH_2$ | – |
| Fe/2 | >225 |
| Fe/3 | 155 – 158 |
| $HOCH_2CH_2NH_3$ | – |
| 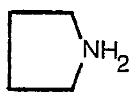 | – |
| $(C_2H_5)_2NH_2$ | – |
| $(CH_3)_2CHNH_3$ | – |
| $CH_2{=}C(CH_3)CH_2NH_3$ | – |
| $(CH_3)_2CHCH_2NH_3$ | – |
| $CH_3OCH_2CH(CH_3)NH_3$ | – |
| $(CH_3)_3CNH_3$ | – |

Example 12

Preparation of Methyl 2-[(carbamoyl-1,2-dimethylpropyl)carbamoyl]nicotinate

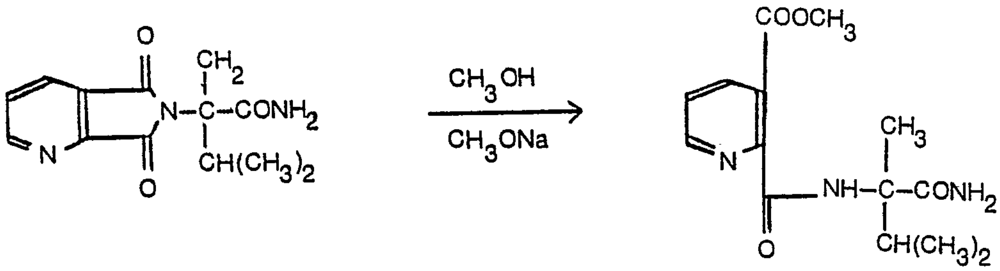


Sodium hydride (0.47 g of a 50% suspension in mineral oil) is reacted with 500 ml dry methanol under nitrogen. To this is added 51.4 g of the amide of Example 2 and the mixture stirred at room temperature overnight. The mixture is concentrated, the residue dissolved in methylene chloride and the solution washed first with 150 ml water followed by 150 ml brine. After drying ($Na_2SO_4$), the organic phase is concentrated and the residue crystallized from ether to give 47.85 g of product which is analytically pure mp 108—145°C with decomposition.

Example 13

Preparation of Methyl 3-[1-carbamoyl-1,2-dimethylpropyl)caramoyl]picolinate

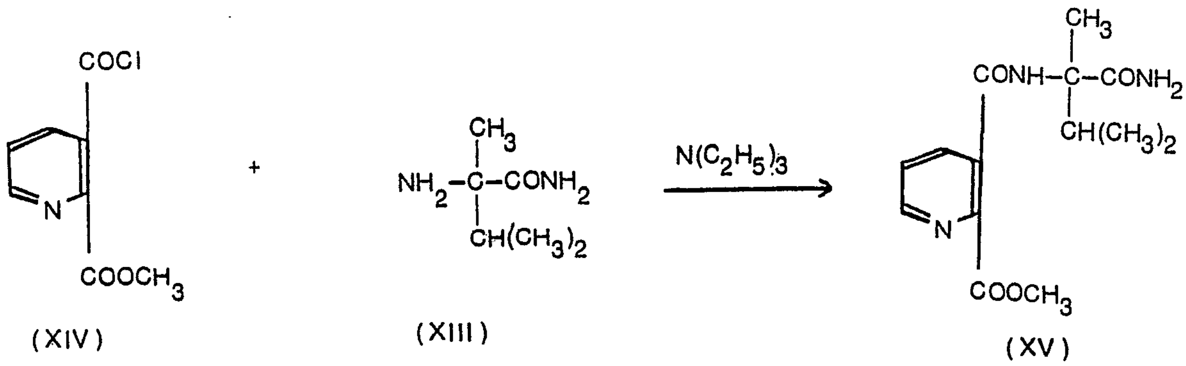


(XIV) (XIII) (XV)

To a stirred mixture containing 25.5 g acid chloride [(Helv. Chem. Acta, *34*, 488 (1951)] and 29.7 ml triethylamine in 200 ml methylene chloride is added, under nitrogen, dropwise a solution containing 13.93 g amino amide as disclosed in (U.S. 4,017,510) at such a rate that the temperature of the reaction mixture remains below 30°C. After 1 hour, the mixture is filtered, the solid washed with methylene chloride and dried to give 19.8 g product, mp 176—177°C (decomp). A sample recrystallized from nitromethane had mp 196—196.5°C (decomp) and analytically pure.

Example 14

Preparation of 5,7-Dihydro-$\alpha$-isopropyl-$\alpha$-methyl-5,7-dioxo-6-*H*-pyrrolo[3,4-*b*]pyridine-6-acetic acid (-isomer)

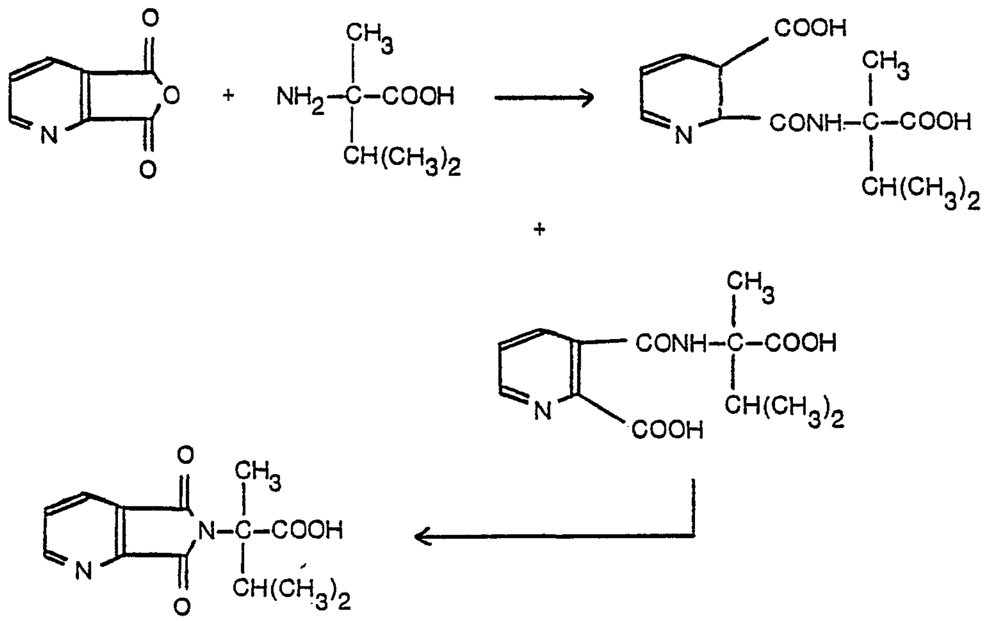

To a stirred suspension of 18.4 g of the anhydride in 760 ml of dry acetone is added, under nitrogen, 16.2 g of (+) -methylvaline. After stirring at room temperature for 48 hours, the mixture is filtered and the filtrate concentrated to give the crude intermediate. This material is dissolved in 500 ml acetic anhydride, a catalytic quantity of sodium acetate added and the mixture stirred at room temperature for 5 hours. After heating under reflux for 1.5 hours, the mixture is concentrated. The residue is dissolved in ethyl acetate and washed with water. The dried extract is concentrated to give a dark syrup. A sample is dissolved in ethyl acetate, treated with charcoal, filtered and concentrated. The residue is crystallized from methylene chloride to give the product, mp 122—125°C $[\alpha]_D^{25} = -7.73$ (c = 0.100, THF).

By essentially the same procedure and using the appropriate starting quinolinic anhydride and amino acid the following amides are prepared

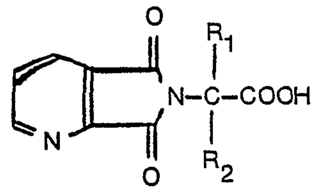

| $R_1$ | $R_2$ | mp°C |
|---|---|---|
| $—CH_3$ | $—CH(CH_3)_2$ | 126—127<br>$[\alpha]_D^{25} = +6.93$<br>(c = 0.100, THF) |
| $CH_3$ | $CH_2CH(CH_3)_2$ | 174—176 |
| $CH_3$ | $CH(CH_3)_2$ | 196.5—198.5 |
| $—\underset{\displaystyle CH_3}{\overset{|}{C}}HCH_2CH_2CH_2CH_2—$ | | 183—186 |

Example 15

Preparation of 5,7-Dihydro-$\alpha$-isopropyl-$\alpha$-methyl-5,7-dioxo-6*H*-pyrrolo[3,4-*b*]pyridine-6-acetamide

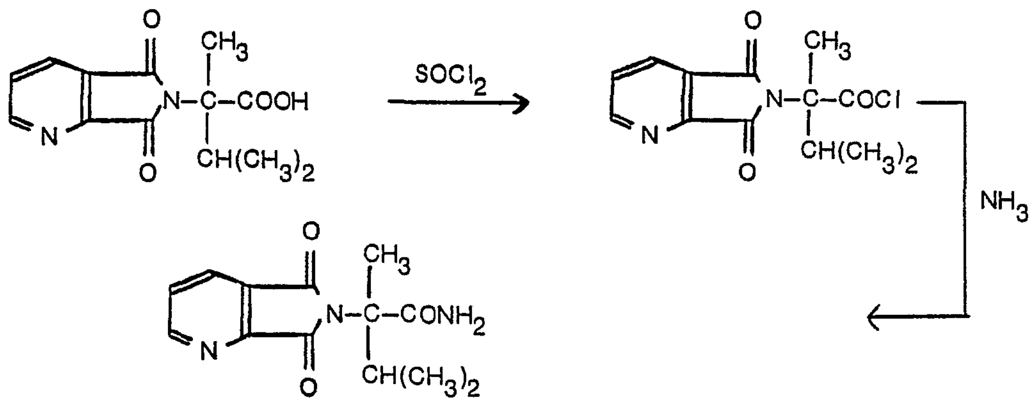


To a mixture containing 32 g of (—)-acid in 375 ml of toluene is added 2 ml of dimethylformamide by 13 ml of thionyl chloride. After heating at reflux for 1.25 hours, the mixture is concentrated *in vacuo*. The residue is dissolved in 350 ml of tetrahydrofuran, cooled to 0°C and a slight excess of gaseous ammonia bubbled through the mixture. The solvent is removed *in vacuo* to leave a solid which is washed with water and air-dried. A portion of this solid is crystallized twice from ethyl acetate (with charcoal treatment) to give the desired product as a white crystalline solid, mp 188—189°C $[\alpha]_D^{25} = +3.59$ (c = 0.791, DMSO).

By essentially the same procedure and using the appropriate acid, the following amides are prepared.

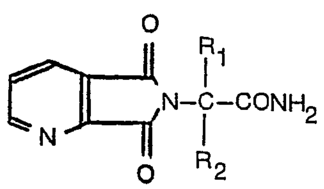

| $R_1$ | $R_2$ | mp°C |
|---|---|---|
| $CH_3$ | $CH(CH_3)_2$ | 189.5—192<br>$[\alpha]_D^{25} = -3.02$<br>(c = 0.0744, DMSO) |
| $CH_3$ | $CH_2CH(CH_3)_2$ | 176—178 |
| $—\underset{\displaystyle CH_3}{\overset{|}{C}}HCH_2CH_2CH_2CH_2—$ | | 186—188 |

Example 16

Preparation of 5-n-Butyl-N-(1-carbamoyl-1,2-dimethyl-propyl)picolinamide

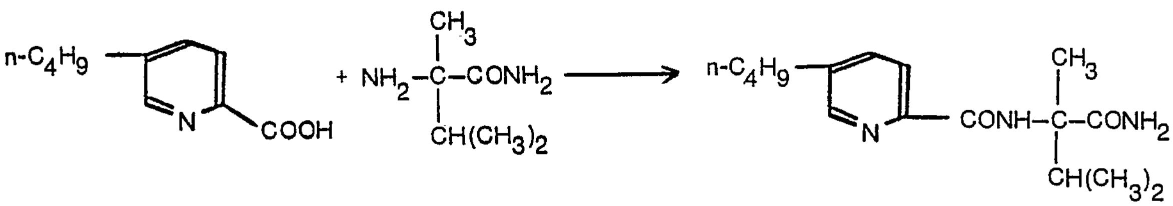

To a suspension of 20 g of acid in 200 ml of dry tetrahydrofuran is added with stirring 10.7 ml of ethyl chlorformate. The mixture is cooled to −10°C and 17.1 ml of triethylamine added dropwise so that the temperature does not exceed 0°C. After 10 minutes, a solution containing 14.3 g of the amino amide in 150 ml of dry tetrahydrofuran is added dropwise at 0°C with stirring. The mixture is allowed to reach toom temperature and after 2 hours, enough water added to dissolve the solid. The tetrahydrofuran is removed in vacuo. The aqueous residue is extracted with ethyl acetate, and after saturating with salt, extracted again. The organic phases are combined, washed with brine dried and concentrated. The residual oil crystallized. A portion was crystallized first from methylene chloride-hexane followed by ether-hexane to give analytically pure product mp 83—86°C.

Using essentially the sme procedures described above the following picolinic acids are prepared.

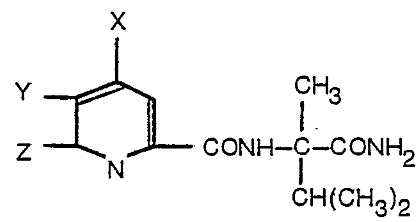

| X | Y | Z | mp°C |
|---|---|---|---|
| H | $CH_3$ | H | 126—127.5° |
| H | H | $CH_3$ | |
| H | $C_6H_5$ | H | |
| H | $No_2$ | H | |

Example 17

Preparation of 2-(5-n-Butyl-2-pyridyl)-5-isopropyl-5-methyl-2-imidazolin-4-one

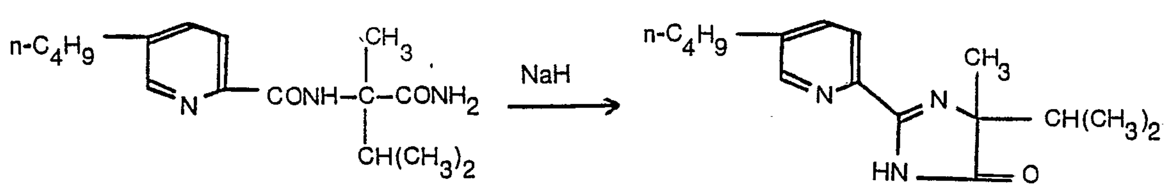


A stirred suspension of sodium hydride (2.4 g) in 250 ml of dry toluene is heated with stirring, under reflux under a Dean-Stark water Separator. To this mixture is added slowly 26.52 g of diamide. After the addition, heating is continued for 1.5 hours. After standing overnight, the reaction is quenched with water, the pH adjusted to 5 with hydrochloric acid and the phases separated. The aqueous phase is further extracted twice with ethyl acetate, the organic extracts combined, washed with brine, dried and concentrated.

The residue is recrystallized from hexane to give the pure product mp 60—62°C.

Using essentially the same procedure, the following imidazolinones are prepared.

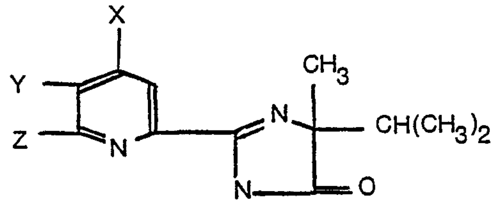

| X | Y | Z | mp°C |
|---|---|---|---|
| H | $C_6H_5$ | H | |
| H | H | $CH_3$ | |
| H | $CH_3$ | H | |
| H | $NO_2$ | H | |

Example 18

Preparation of 5-n-Butyl-2-(5-isopropyl-5-methyl-4-oxo-2-imidazolin-2-yl)nicotinic acid

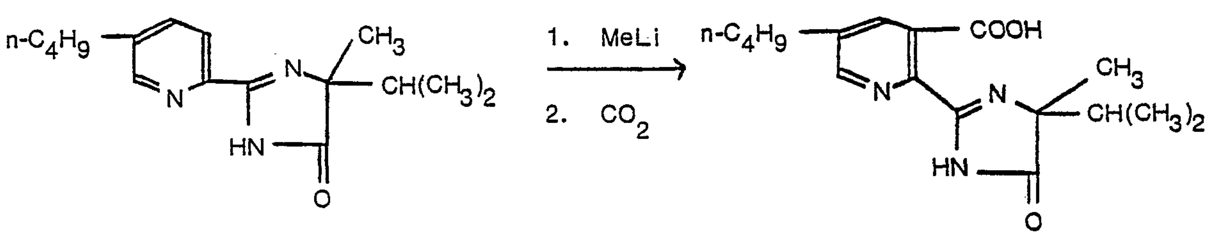


To a stirred solution containing 10.0 g imidazolinone in 100 ml dry tetrahydrofuran at −76° under nitrogen is added dropwise 47.3 ml of a 1.7 M solution of methyl lithium in ether. The mixture becomes very thick and 2 ml hexamethylphosphoramide and about 150 ml tetrahydrofuran is added. The mixture is allowed to warm to −10°C and held at this temperature for 0.75 hour. The mixture is cooled to −70° and added to slurry of carbon dioxide in tetrahydrofuran. After stirring for 0.5 hour, water is added to the mixture, the pH adjusted to 2 with dilute sulfuric acid, and the product extracted into methylene chloride. The extract is washed with brine, dried and concentrated to give the product as a yellow solid. Recrystallization from methylene-chloride-hexane gave an analytically pure sample, mp 152—154°.

Using essentially the same procedure as described above but substituting the appropriate imidazolinone for 2-(5-butyl-2-pyridyl)-5-isopropyl-5-methyl-2-imidazolinone, and using dimethylformamide and methyl iodide as well as carbon dioxide as electrophiles, the following imidazolinones are prepared:

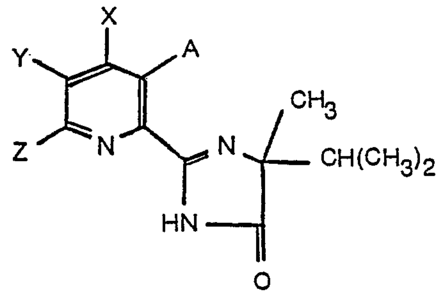

| A | X | Y | Z | mp°C |
|---|---|---|---|---|
| COOH | Cl | H | H | 184—186 |
| COOH | H | $CH_3$ | H | 203.5—204.5 |
| COOH | H | $C_6H_5$ | H | 150—151.5 |
| $CH_3$ | H | H | H | 93—96 |
| CHO | H | H | H | 223—225 |
| COOH | H | H | $C_6H_5$ | 252—254 |
| COOH | H | 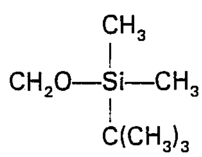 | H | 82—85 |
| COOH | H | $C_2H_5$ | H | |

Example 19

Preparation of 2-(5-Isopropyl-5-methyl-4-oxo-2-imidazolin-2-yl)-3-pyridineacetic acid

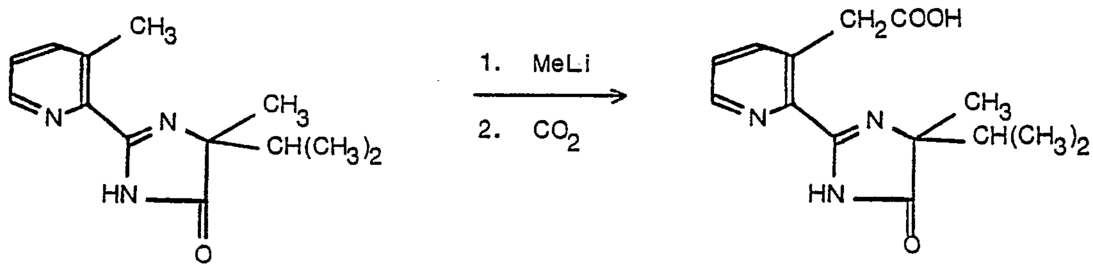


Using essentially the same procedure as described in Example 18 but substituting 5 - isopropyl - 5 - methyl - 2 - (3 - methyl - 2 - pyridyl) - 2 - imidazolin - 4 - one for 5 - isopropyl - 5 - methyl - 2 - (5 - n - butyl - 2 - pyridyl) - 2 - imidazolin - 4 - one, there is obtained the desired pyridineacetic acid, mp 173 (dec.).

Example 20

Preparation of Methyl 2-(5-isopropyl-5-methyl-4-oxo-2-imidazolin-2-yl)-6-phenoxynicotinate

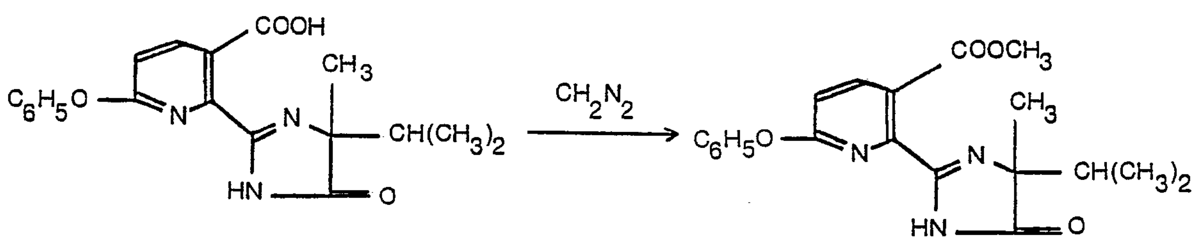


A solution of the acid in ether is treated with excess diazomethane. After a few minutes excess

diazomethane is removed by warming. The solvent is removed and the residue crystallized from ether hexane to give the desired methyl ester mp 128—131°C.

Using essentially the same conditions as those described above, the following methyl esters are prepared starting with the appropriate acid.

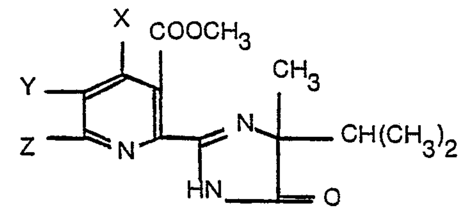

| X | Y | Z | mp°C |
|---|---|---|---|
| H | H | $—OC_6H_5$ | 128—131 |
| H | $—C_4H_9$-n | H | 69—71.5 |
| Cl | H | H | 110—113 |
| H | H | $OCH_2C_6H_5$ | 187—188 |
| H | H | $OC_2H_5$ | 126—129 |
| $OC_6H_5$ | H | H | 175—177 |
| H | $CH_3$ | H | 129—130.5 |
| H | $C_6H_5$ | H | |
| H | H | $C_6H_5$ | |
| $OCH_2C_6H_5$ | H | H | 164—171 |
| H | $C_2H_5$ | H | |
| H | $CH_2OH$ | H | 146—147 |

Example 21

Preparation of 4 [2-(5-Isopropyl-5-methyl-4-oxo-2-imidazolin-2-yl)nicotinoyl]morpholine

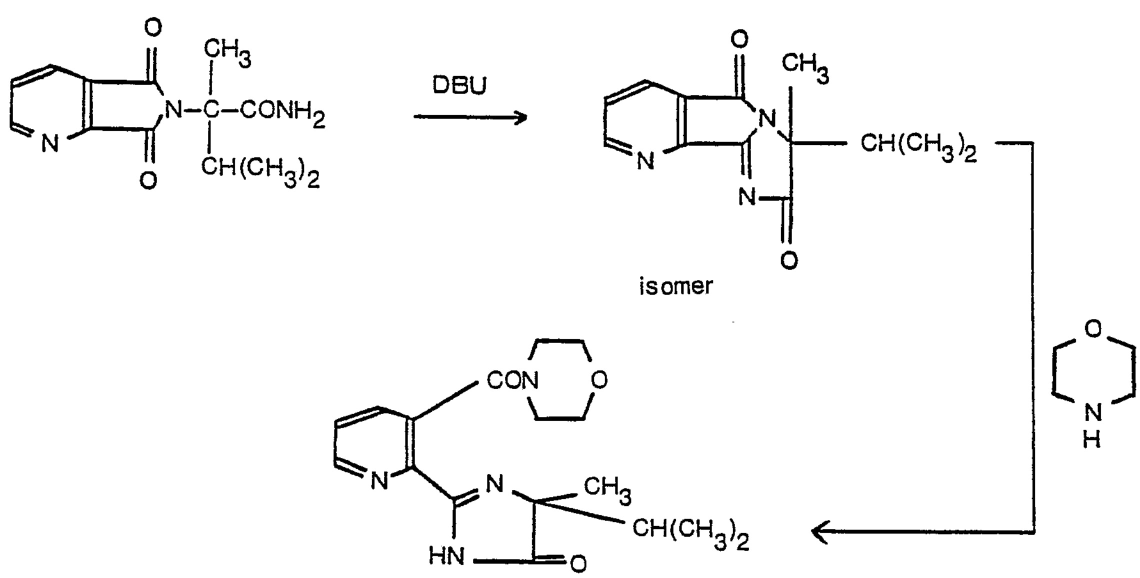


The cyclization of the amide is accomplished by heating 7.83 g of amide in 150 ml of toluene and 0.45 ml of 1,8-diazabicyclo[5,4,0]undec-7-ene under a Dean-Stark water Separator for 2 hours as

described in Example 4. The separator is removed, 4 ml of morpholine is added and heating continued for 3 hours. The mixture is concentrated and the residue chromatographed on silica gel in ethyl acetate. The product is eluted first and this material is recrystallized from ether-hexane to give pure amide mp 143—145.5°C.

By substituting the appropriate amine for morpholine, the following amides are prepared.

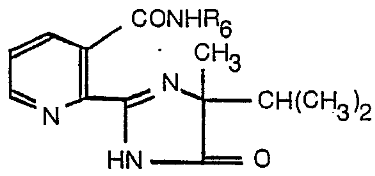

| $R_6$ | mp°C |
|---|---|
| $—CH_2C{\equiv}CH$ | 171—173.5 |
| 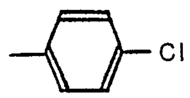 | 227.5—228.5 |
| $—CH_2CH_2OH$ | 174.5—175.5 |

### Example 22

Preparation of N-(2-chloroethyl)-2-(5-isopropyl-5-methyl-4-oxo-2-imidazolin-2-yl)nicotinamide

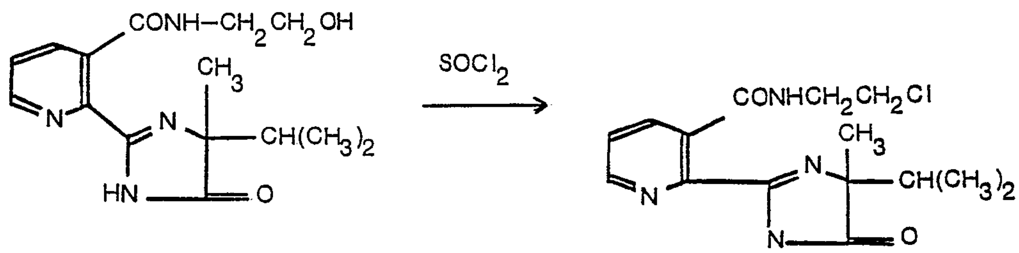


A mixture containing 4.04 g of hydroxyethylamide and 8.2 ml of thionyl chloride in 250 ml of methylene chloride is heated at reflux for 3.5 hours. The mixture is cooled, poured into water and the aqueous phase made basic with sodium carbonate. The mixture is shaken, the organic phase separated, washed with water, dried and concentrated to leave a white solid which is recrystallized from toluene to give the desired chloroethylamide as a white crystalline solid, melting partially at 128.5°C with complete melting at 157°C.

### Example 23

Preparation of 2-(5-Isopropyl-5-methyl-4-oxo-2-imidazolin-2-yl)nicotinamide

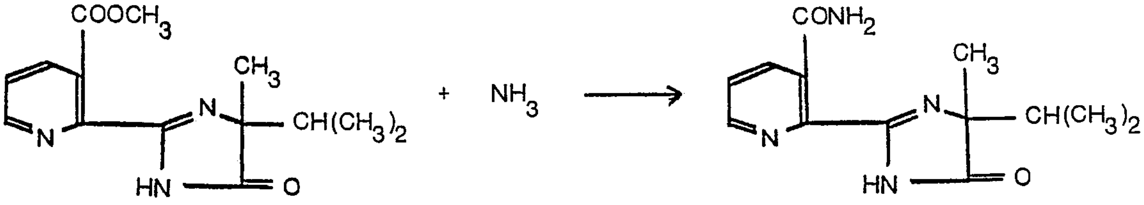


A solution containing 10.0 g of ester in 50 ml of tetrahydrofuran is added to 100 ml of liquid ammonia in a glass bomb. The bomb is sealed and the contents heated at 100°C for 16 hours. After cooling, the ammonia is evaporated and the residue concentrated. This residue is combined with material from similar reactions using 5 g and 7 g of the ester. These are crystallized from ethyl acetate to give 5 g of product. The filtrate is concentrated after treatment with charcoal to give a further 15.7 g of product.

Two recrystallizations of a sample from ethyl acetate gives the pure nicotinamide as a white crystalline solid mp 178—182°C.

## Example 24

Preparation of 2-(5-Isopropyl-5-methyl-4-oxo-2-imidazolin-2-yl)nicotinonitrile

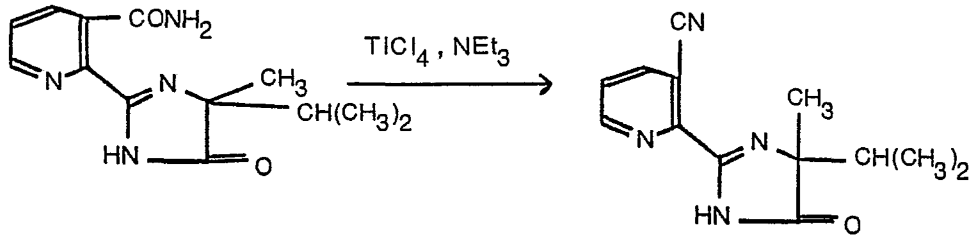


To 75 ml of ice-cold tetrahydrofuran under nitrogen is added with stirring 12 ml of titanium tetrachloride in 20 ml of carbon tetrachloride at such a rate that the temperature does not exceed 5°C. This is followed by the addition of 5.2 g of the amide in 75 ml of tetrahydrofuran again maintaining a temperature <5°C. Finally, 17 ml of triethylamine in 5 ml of tetrahydrofuran is added to the mixture under the same conditions. After 1.5 hours at 5°C, the mixture is stirred overnight at room temperature. Water (100 ml) is cautiously added at 0°C, the upper organic phase is separated and the aqueous phase extracted with methylene chloride (4 x 100 ml). The combined extracts are washed with brine, dried and concentrated. The solid residue is recrystallized from hexane-methylenechloride to give the nicotinonitrile as a tan solid, mp 144—148°C. The analytically pure compound has mp 148—150°C.

## Example 25

Preparation of 2-[5-(hydroxymethyl)-2-pyridyl]-5-isopropyl-5-methyl-2-imidazolin-4-one

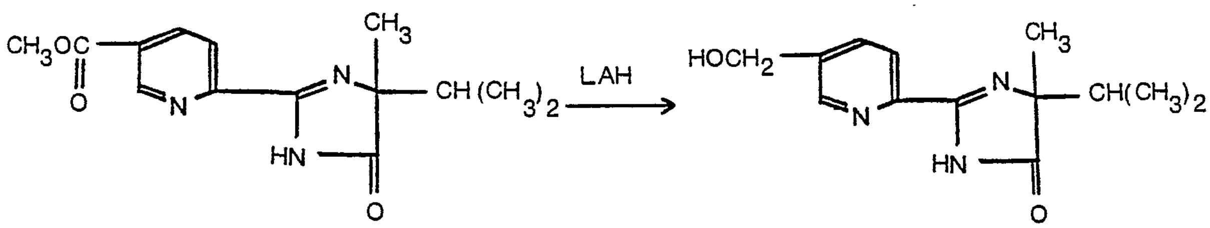


To a stirred slurry of 23 g lithium aluminum hydride in 250 ml tetrahydrofuran under nitrogen at —70°C is added dropwise 46.8 g of the ester in 350 ml tetrahydrofuran. The mixture is warmed to room temperature, 73 ml of a saturated ammonium chloride solution added cautiously with vigorous stirring, the mixture filtered and the solid washed with tetrahydrofuran. The filtrate is concentrated to leave a gum. This is chromatographed on silica gel and the product eluted by ethyl acetate, mp 101—104°C.

## Example 26

Preparation of 2 - [5(*tert* - butyldimethylsiloxy) - methyl - 2 - pyridyl] - 5 - isopropyl - 5 - methyl - 2 - imidazolin - 4 - one

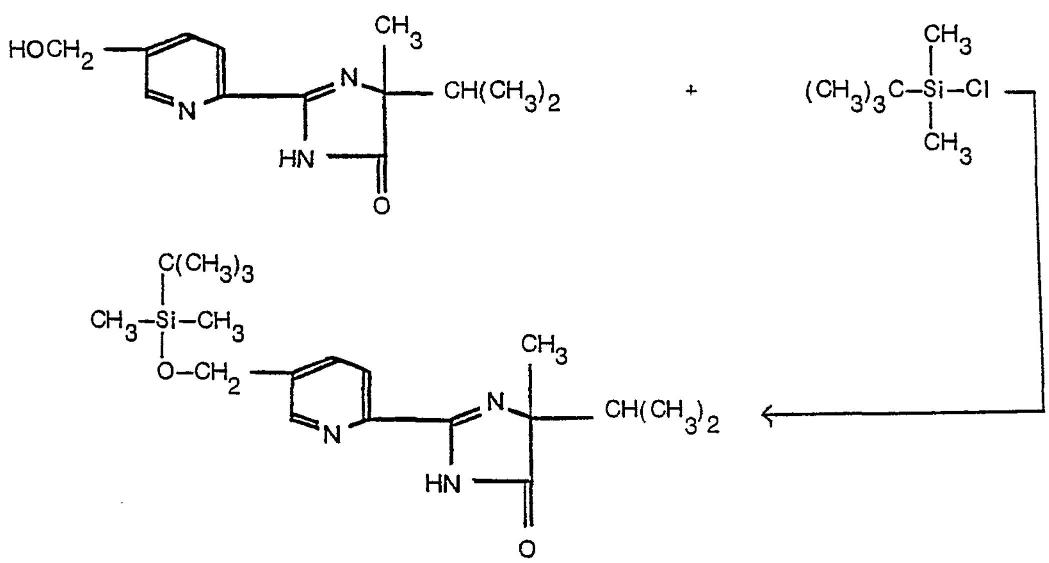

To a stirred solution containing 2.03 g alcohol in 3.5 ml dimethylformamide under nitrogen is added 0.68 g imidazole followed by 3.1 g *t*-butyldimethylsilyl chloride. The mixture is kept at 35°C for 10 hours and room temperature for 10 hours. Saturated sodium sulfate is added and the aqueous mixture extracted with ether. The extract is washed with brine, dried and evaporated. The pure product is isolated as a gum by chromatography of the crude product on silica gel and elution with methylene chloride followed by ether.

## Example 27

Preparation of 5-(Hydroxymethyl)-2-(5-isopropyl-5-methyl-4-oxo-2-imidazolin-2-yl)nicotinic acid

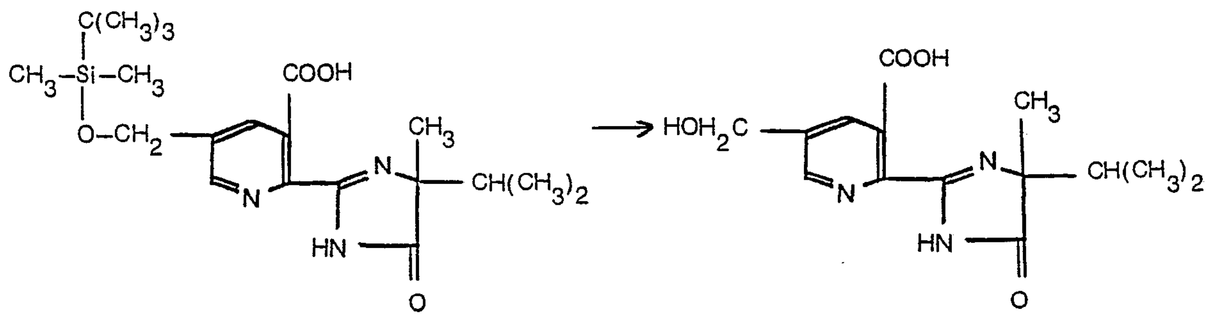

A solution containing 0.29 g silyl ether in 10 ml 80% aqueous acetic acid is heated on the steam bath for 0.5 hours. The mixture is concentrated and the residue dried azeotropically with toluene. The residue, a gum, is crystallized from methylene chloride-hexane. The pure product has mp 170—171.5°C.

## Example 28

Preparation of Methyl 2 - (1 - acetyl - 4 - isopropyl - 4 - methyl - 5 - oxo - 2 - imidazolin - 2 - yl)nicotinate

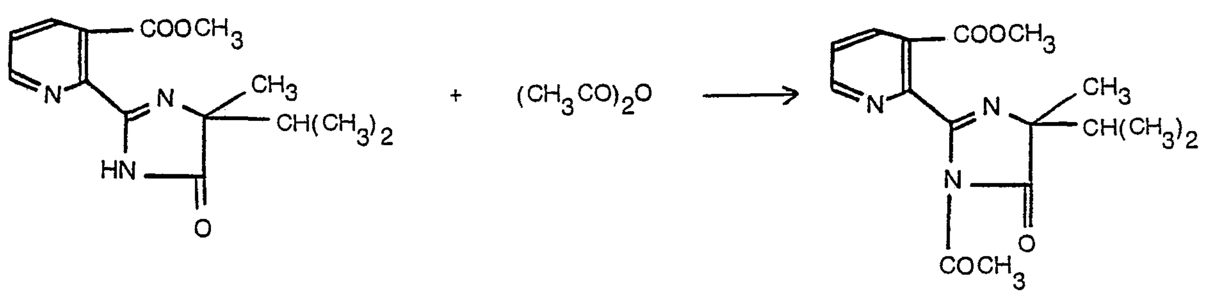

A solution containing 10 g methyl 2-(5-isopropyl-5-methyl-4-oxo-2-imidazolin-2-yl)nicotinate in 100 ml acetic anhydride is heated under reflux for 16 hours. The mixture is concentrated and the residue crystallized from ether-hexane to give the N-acetyl derivative, mp 88—90°C. This is the mp of analytically pure material.

Using essentially the same conditions as those described above, the following N-substituted imidazolinones are prepared by reacting the appropriate imidazolinyl nocotinate with the appropriate acyl anhydride, acyl halide, sulfonyl halide, alkyl halide or sulfate either alone or in a solvent such as pyridine or toluene.

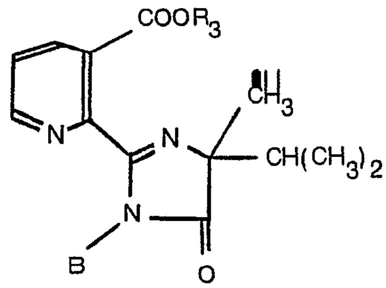

| $R_3$ | B | mp°C |
|---|---|---|
| $CH_3$ | $CH_3$ | oil |
| $CH_3$ | $COC(CH_3)_3$ | 85 – 87 |
| $CH_3$ | $COC_{11}H_{23}$-n | oil |
| $CH_3$ | $COC_6H_5$ | 104 – 107 |
| $CH_3$ | $COC_2H_5$ | 90 – 92.5 |
| $CH_3$ | $COCH_2Cl$ | 98 – 100 |
| $CH_2C_6H_5$ | $COC_2H_5$ | oil |
| $CH_2C_6H_5$ | $COC(CH_3)_3$ | oil |
| $CH_2C_6H_5$ | $COCH_2Cl$ | oil |
| $CH_3$ | $SO_2CH_3$ | 115 – 118 |
| $CH_2{\equiv}CH$ | $COCH_3$ | 125 – 127 |
| $CH_2C{\equiv}CH$ | $COCH_2Cl$ | 118 – 122 |
| $CH_2C{\equiv}CH$ | $COC_6H_5$ | 118 – 120 |
| $CH_2C{\equiv}CH$ | $COC(CH_3)_3$ | 101 – 104 |
| $CH_3$ | $COOC_2H_5$ | oil |
| $CH_3$ | 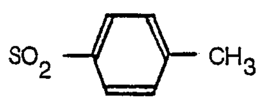 | 114 – 118 |
| $CH_2C_6H_5$ | $COC_6H_5$ | 117 – 125 |
| $CH_2C{\equiv}CH$ | $COC_2H_5$ | 85 – 88 |
| $CH_3$ | 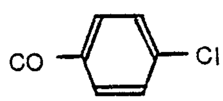 | 122 – 125 |
| $CH_3$ | 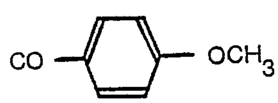 | 119.5 – 121.5 |
| $CH_3$ | 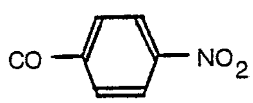 | 148 – 151 |

Example 29

Preparation of Methyl 2-(1-acetyl-4-isopropyl-4-methyl-5-oxo-2-imidazolin-2-yl)nicotinate-1-oxide

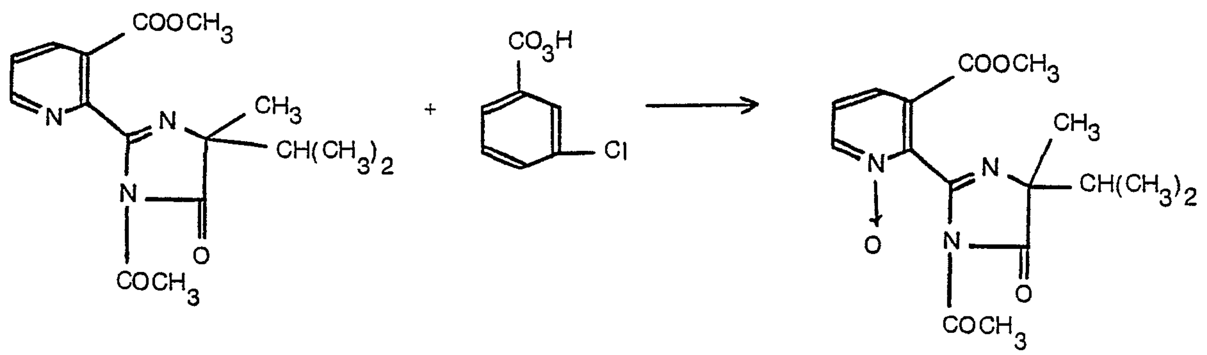

To a solution containing 40 g (126 mmoles) of the nicotinate in 500 ml methylene chloride is added 30 g of 80—90% pure (139 mmoles based on 80% purity) m-chloroperbenzoic acid. After heating at reflux overnight, excess peracid is destroyed by the addition of excess 1-hexene. The solution is washed with saturated sodium bicarbonate solution, dried and concentrated. The residue is crystallized from methylene chloride-hexane-ether to give 18.3 g of the desired N-oxide, mp 92—100°C. The analytically pure N-oxide has mp 95—99°C.

Example 30

Preparation of Methyl 2-(5-isopropyl-5-methyl-4-oxo-2-imidazolin-2-yl)nicotinate-1-oxide

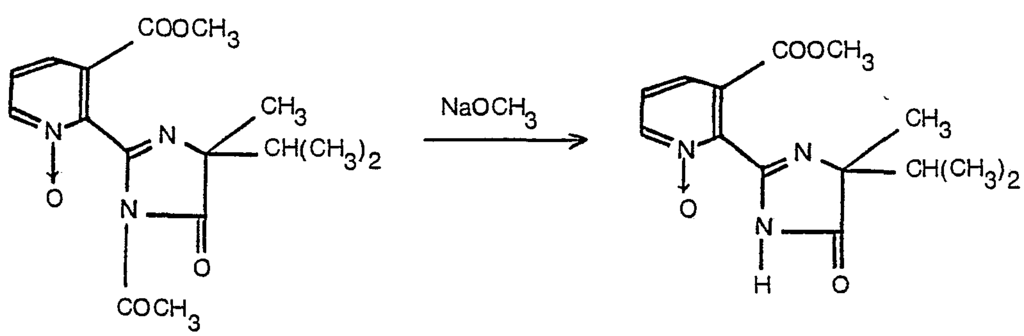


To a solution of 30 g of the N-acetyl compound in 200 ml methanol is added approximately 0.5 g sodium methoxide. After stirring for two hours, the product is removed by filtration and air dried, mp 197—201°C. The analytically pure sample which had been recrystallized from acetone-hexane has mp 200—201°C.

Example 31

Preparation of Methyl 6-chloro-2-(5-isopropyl-5-methyl-4-oxo-2-imidazolin-2-yl)nicotinate

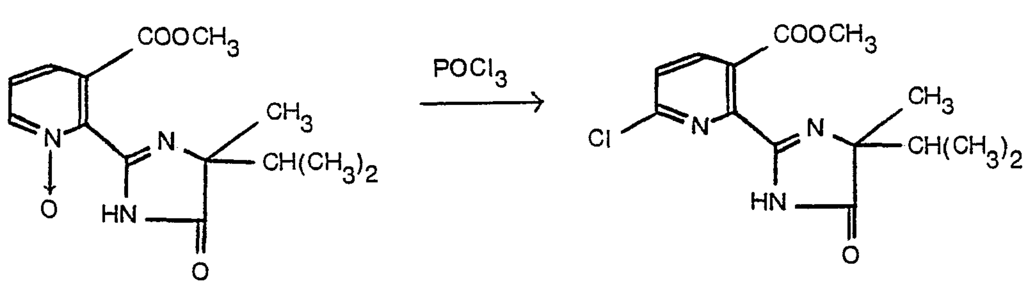


A solution containing 22.0 g N-oxide in 135 ml phosphorus oxychloride is heated under reflux for four hours. After standing at room temperature overnight, excess phosphorus oxychloride is removed *in vacuo* and the residue treated with xylene and again concentrated. The residue is dissolved in methylene chloride and treated with water, the pH adjusted to 5 with sodium carbonate and ether added to make the organic layer the upper layer. The layers are separated and the aqueous phase reextracted twice with ether. The combined organic extracts are washed with brine, dried and concentrated. The residue is chromatographed on 250 g of silica gel in a mixture of ether and hexane to

give 10.6 g of the desired product. This is recrystallized from ether-hexane to give 8.95 g of the 6-chloro derivative, mp 104—106°C. The analytically pure sample melted at 102.5—104.5°C.

Example 32

Preparation of 6-Chloro-2-(5-isopropyl-5-methyl-4-oxo-2-imidazolin-2-yl)nicotinic acid

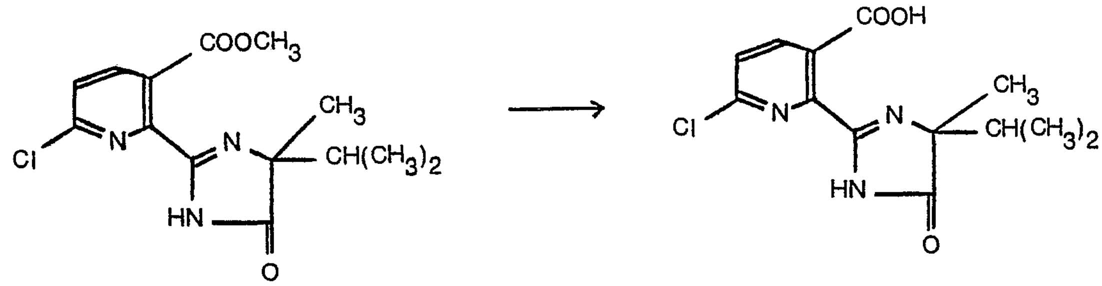

A suspension of 3.0 g of the ester in 5.8 ml of 2N sodium hydroxide, 5 ml water and 3 ml methanol is warmed to 35°C to obtain a clear solution. After stirring the solution for three hours, it is cooled, extracted with ether and the organic phase discarded. The pH of the aqueous phase is adjusted to 2 with 6N hydrochloric acid and then sodium bicarbonate solution added to bring the pH to 4. The aqueous phase is extracted twice with methylene chloride, the pH of the aqueous phase adjusted to 2 and again extracted twice with methylene chloride. The organic phases are combined, dried and concentrated and the residue crystallized from methylene chloride-hexane to give the analytically pure acid, mp 154—157°C.

Following the above procedure but substituting the 5-bromo ester for the 6-chloro ester yields 5 - bromo - 2 - (5 - isopropyl - 5 - methyl - 4 - oxo - 2 - imidazolin - 2 - yl)nicotinic acid, mp 211—213°C.

Example 33

Preparation of 6-(Benzyloxy)-2-(5-isopropyl-5-methyl-4-oxo-2-imidazolin-2-yl)nicotinic acid

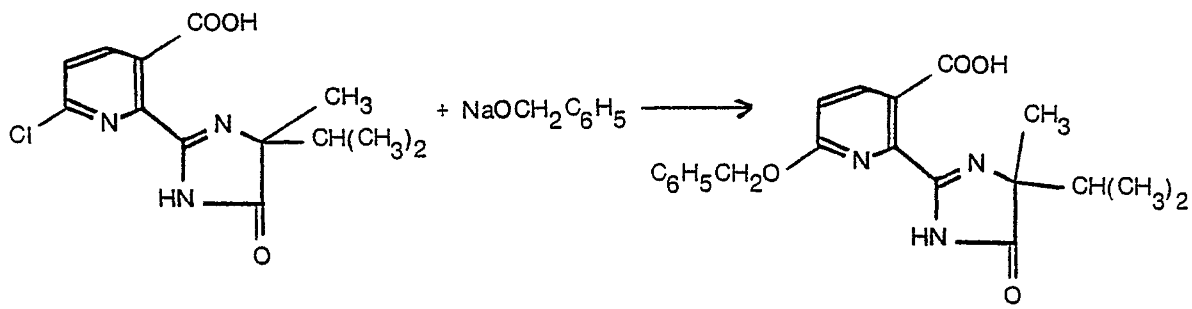


To sodium hydride (from 0.34 g 50% sodium hydride in oil) in 2 ml of N-methyl pyrolidone is added with stirring and under nitrogen 2 ml benzyl alcohol. After the formation of the alkoxide is complete, 0.6 g of the chloro acid is added and the mixture heated at 165—175° for five hours.

After cooling, the mixture is diluted with water, the pH thereof adjusted to 1 with 1N hydrochloric acid and then back to pH 8 with saturated sodium bicarbonate. The mixture is extracted twice with ether and the ether discarded. The pH of the aqueous phase is adjusted to 5 and extracted several times with methylene chloride. The extracts are combined, dried and concentrated. Crystallization from ether-hexane gives the 6-benzyloxy derivative mp 205—207°C.

Using essentially the same conditions as described above, and using the appropriate 4- or 6-chloro-2-(5-isopropyl-5-methyl-4-oxo-2-imidazolin-2-yl)nicotinic acid and appropriate sodium alkoxide, phenoxide or thioalkoxide, the following imidazolinyl nicotinic acids are prepared.

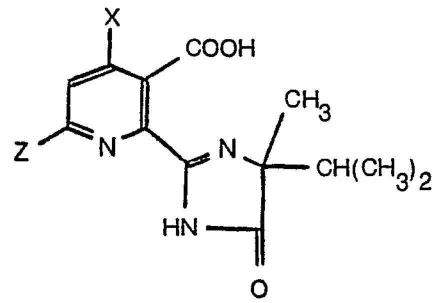

| X | Z | Mp°C |
|---|---|---|
| H | $OCH_3$ | 190—191.5 |
| $OC_6H_5$ | H | 196—198 |
| H | $OC_6H_5$ | 182.5—185.5 |
| H | $OC_2H_5$ | 190—191.5 |
| H | $SCH_3$ | 188.5—190 |
| $OCH_2C_6H_5$ | H | 172—174 |
| H | $OCH_2C_6H_5$ | 205—207 |

Example 34

Preparation of 4-Hydroxy-2(5-isopropyl-5-methyl-4-oxo-2-imidazolin-2-yl)nicotinic acid

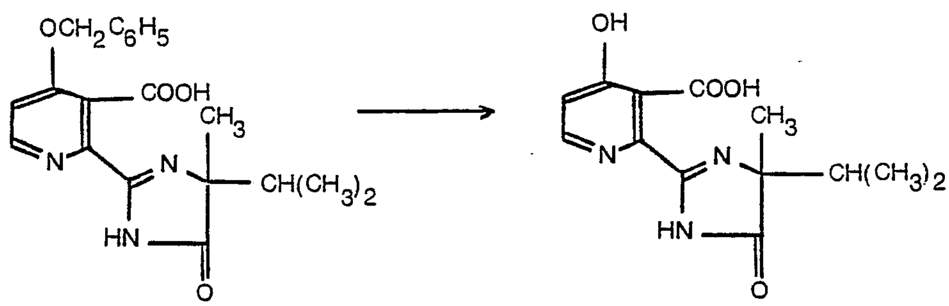

To 15 ml concentrated sulfuric acid is added slowly, with stirring 1.55 g of the benzyloxy derivative. To this mixture is added 7 ml ethylenedichloride. After 16 hours at room temperature, the mixture is poured over ice, the pH adjusted to 4 with dilute sodium hydroxide and extracted with ethyl acetate. The extract was dried and concentrated to leave a tan solid which is recrystallized from methylene chloride-ether, mp 210—211°C.

Example 35

Preparation of 2 - Isopropyl - 2 - methyl - 5*H* - imidazo - [1′,2′:1,2]pyrrolo[3,4 - *b*]pyridine - 3(2*H*),5 - dione

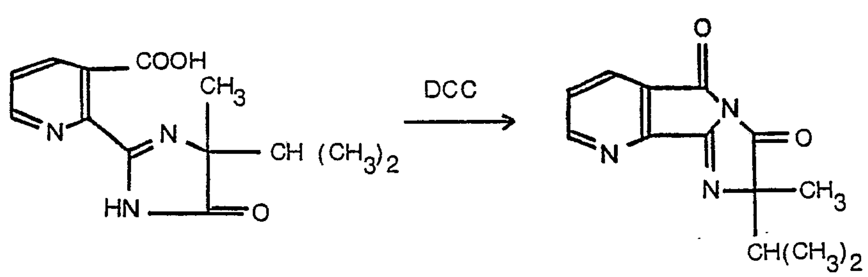


To a solution containing 50.9 g of dicyclohexylcarbodiimide in 600 ml of dry methylene chloride is added, while stirring, 60 g of the acid at such a rate that the temperature does not exceed 32°C. After stirring at room temperature for 2.5 hours, the mixture is filtered and the filtrate concentrated to give a

white solid. This solid is recrystallized from methylene chloride to give 57.4 g of the dione, mp 125—128.5°C. The analytically pure dione melts at 132—134°C.

Example 36

Preparation of the Acetone oxime ester of 2-(5-isopropyl-5-methyl-4-oxo-2-imidazolin-2-yl)nicotinic acid

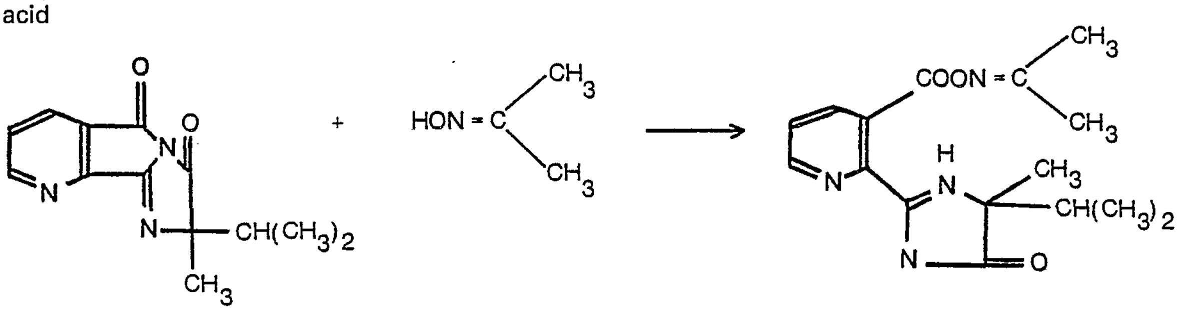

To a solution containing 2.0 g of the 3,5-dione in 15 ml of toluene is added 0.6 g of acetone oxime. The mixture is heated and stirred at 50—60°C for 2.75 hours. After stirring overnight at room temperature, the solvent is removed and the residue chromatographed on silica gel using 10% acetonitrile in methylene chloride followed by 30% acetonitrile in methylene chloride as the eluent. Toluene is removed from the fractions containing the product and the product collected. This is recrystallized from methylenechloride hexane to give analytically pure oxime ester mp 117—119.5°C. The ester from 2,2,2-trichloroethanol mp 114—116°C is prepared in essentially the same manner.

Example 37

Preparation of 2-(3-Acetyl-2-pyridyl)-5-isopropyl-5-methyl-2-imidazolin-4-one

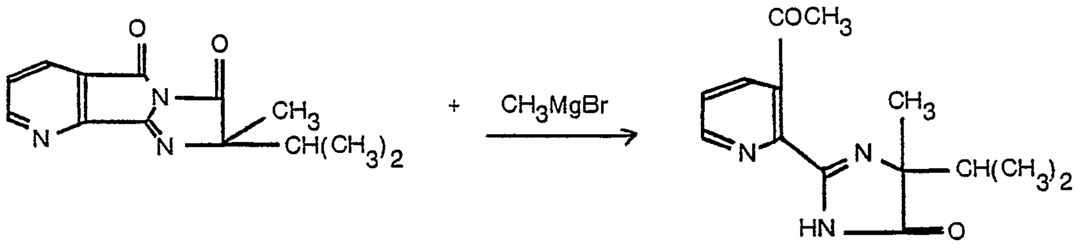


To a stirred solution containing 10.0 g of the dione in 100 ml of dry tetrahydrofuran under nitrogen and −78°C is added dropwise 15.1 ml of a 3M solution of methyl magnesium bromide in ether. A temperature of <−60°C is maintained during the addition. After the addition, stirring is continued at −78°C and then the mixture warmed slowly to room temperature. The mixture is diluted with an equal volume of water, the pH adjusted to 4 with glacial acetic acid and extracted three times with methylene chloride. The combined extracts are dried and concentrated. The residue is chromatographed on silica gel with ether. Concentration of the appropriate fractions give 6.1 g of product as a crystalline solid mp 104—108°C. An analytically pure sample has mp 103—105°C.

Using essentially the same procedure as described above but substituting phenyl lithium or sodium trimethyl phosphonoacetate for methyl magnesium bromide, the following imidazolinones are prepared.

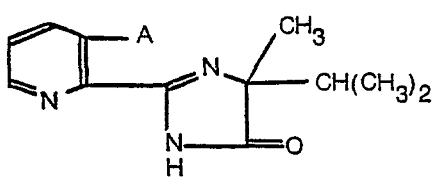

| A | mp°C |
|---|---|
| $COC_6H_5$ | 138—140.5 |
| 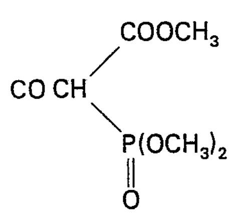 | 131.5—134 |

Example 38

Preparation of 2 - [3 - (Hydroxymethyl) - 2 - pyridyl] - 5 - isopropyl - 5 - methyl - 2 - imidazolin - 4 - one

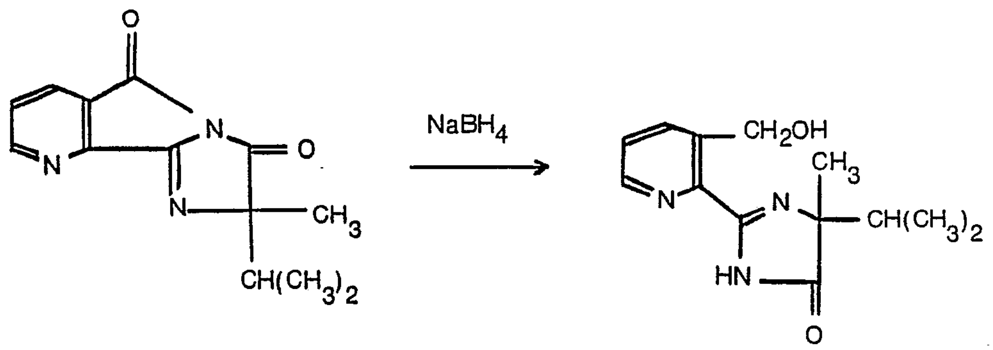


To a stirred solution of 0.32 g sodium borohydride in 25 ml absolute ethanol at 0°C is added during 10 minutes with stirring a solution containing 2.0 g dione in 25 ml dry tetrahydrofuran. The mixture is then stirred a further three hours at room temperature. The mixture is poured into 200 ml ice water, extracted with methylene chloride, the extract dried and concentrated. The residue is crystallized from methylenechloride-hexane to give the desired product. The analytically pure sample has mp 145—149°C.

Example 39

Preparation of 1,3-Dihydro-$\alpha$-isopropyl-$\alpha$-methyl-1,3-dioxo-2-H-pyrrolo-[3,4-$\beta$] quinoline-2-acetonitrile

Procedure A

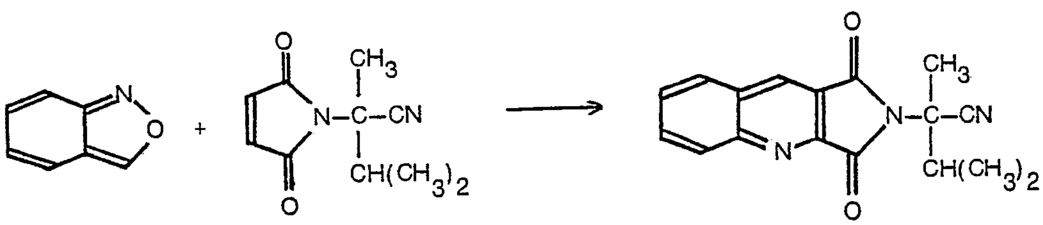

Anthranil (59.6 g, 0.5 mol) is added dropwise under nitrogen, with stirring, over a 45 minute period, to a refluxing solution of $\alpha$-isopropyl-$\alpha$-methyl-2,5-dioxo-3-pyrroline-1-acetonitrile in *o*-dichlorobenzene (450 ml). After 18 hours the reaction mixture is cooled and methylene chloride added. This solution is passed through a 3 inch silica gel column, by eluting with methylene chloride. The eluate is concentrated to 500 ml and hexane added. A precipitate forms and is filtered off and air dried, to yield the product 110.6 g, (75%) as a light brown solid. Crystallization from ethyl acetate-hexane gives pale yellow crystals, mp 195—196°C. Anal. calcd. for $C_{17}H_{15}N_3O_2$: C, 69.61; H, 5.15; N, 14.33. Found: C, 69.37; H, 5.15; N, 14.43.

Employing similar conditions the compounds of Table I are prepared.

Procedure B

Cyclization of o-formylanilino-maleimides

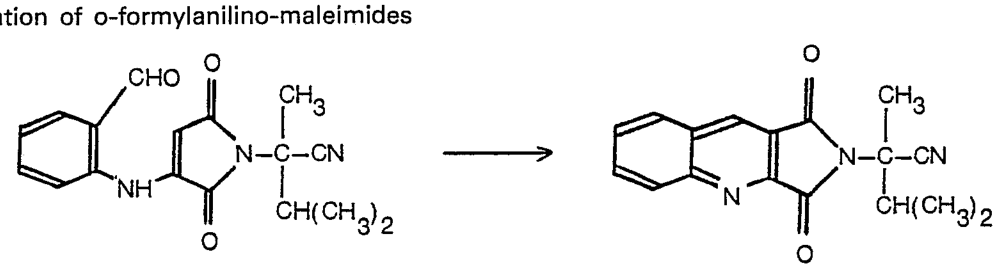

A solution of N-(1-cyano-1,2-dimethylpropyl)-2-(*o*-formylanilino) maleimide (7.19 g, 0.023 mol) in xylene (300 ml) containing *p*-toluenesulfonic acid (0.3 g, 0.0016 mol) is heated at reflux for 4 hours using a Dean-Stark trap to collect the eliminated water. The reaction is cooled, evaporated under reduced pressure and dissolved in hot ethyl acetate which is passed through a 3 inch silica gel column. The ethyl acetate fractions eluted are combined to give a solid, mp 195—195.5°C, 5.51 g, (81%) of 1,3-dihydro-$\alpha$-isopropyl-$\alpha$-methyl-1,3-dioxo-2-*H*-pyrrolo [3,4-*b*] quinoline-2-acetonitrile.

Other compounds prepared by this procedure are listed in Table I.

Example 40

Procedure C

Preparation of 1,3-Dihydro-α-isopropyl-α-methyl-1,3-dioxo-2-H-pyrrolo[3,4-b] 4-acetoxyquinoline-2-acetonitrile

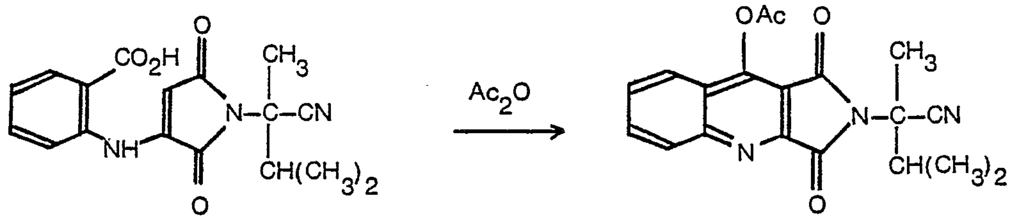


A solution of N-[1-(1-cyano-1,2-dimethylpropyl)-2,5-dioxo-3-pyrrolin-3-yl anthranilic]acid (3.27 g, 0.01 mol) in acetic anhydride (20 ml) is treated all at once with triethylamine (10 ml) and dimethylaminopyridine (0.122 g, 0.001 mol). After stirring under nitrogen for 1 hour at 25° the reaction is poured into ice-water. A solid forms and is filtered off. Purification is achieved by re-suspension in ether, filtering and drying. Yield 2.54 g (72%) of product, mp 145—151°C,

$$\frac{m+I}{e} = 352.$$

TABLE I

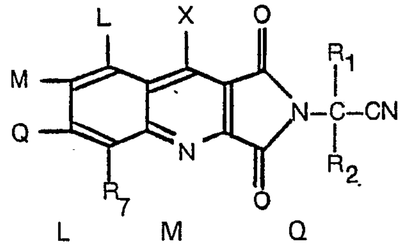

| $R_1$ | $R_2$ | X | L | M | Q | $R_7$ | Procedure | mp°C |
|---|---|---|---|---|---|---|---|---|
| $CH_2$ | $CH(CH_3)_2$ | H | H | $NO_2$ | H | H | A | 230—232 |
| $CH_3$ | $CH(CH_3)_2$ | H | H | H | $NO_2$ | H | A | 260—261 |
| $CH_3$ | $CH(CH_3)_2$ | H | $NO_2$ | H | H | H | | |
| $CH_3$ | $CH(CH_3)_2$ | H | H | H | H | $NO_2$ | A | |
| $CH_3$ | $CH(CH_3)_2$ | H | Br | H | H | H | A | |
| $CH_3$ | $CH(CH_3)_2$ | H | Cl | H | H | H | A,B | 139.5—142 |
| $CH_3$ | $CH(CH_3)_2$ | H | H | $CF_3$ | H | H | B | |
| $CH_3$ | $CH(CH_3)_2$ | H | H | H | Cl | H | B | 188 |
| $CH_3$ | $CH(CH_3)_2$ | H | H | $CH_3$ | H | H | B | |
| $CH_3$ | $CH(CH_3)_2$ | H | H | H | $CH_3$ | H | B | 186—190 |
| $CH_3$ | $CH(CH_3)_2$ | H | H | H | H | $CH_3$ | B | |
| $CH_3$ | $CH(CH_3)_2$ | H | H | $OCH_3$ | H | H | B | |
| $CH_3$ | $CH(CH_3)_2$ | H | H | H | $CH_3$ | $CH_3$ | B | |
| $CH_3$ | $CH(CH_3)_2$ | H | H | $CH_3$ | H | $CH_3$ | B | |
| $CH_3$ | $CH(CH_3)_2$ | H | $CH_3$ | H | H | $CH_3$ | B | |
| $CH_3$ | $CH(CH_3)_2$ | H | H | H | Cl | $CH_3$ | B | |
| $CH_3$ | $CH(CH_3)_2$ | H | Cl | H | H | $CH_3$ | B | |
| $CH_3$ | $CH(CH_3)_2$ | H | H | Cl | H | $CH_3$ | B | |
| $CH_3$ | $CH(CH_3)_2$ | H | Cl | H | H | $OCH_3$ | B | |
| $CH_3$ | $C_3H_7$ | H | H | H | H | H | A | |
| $CH_3$ | $C_2H_5$ | H | H | H | H | H | A | |
| $CH_3$ | *n*-$C_4H_9$ | H | H | H | H | H | A | |
| $CH_3$ | *s*-$C_4H_9$ | H | H | H | H | H | A | |
| $CH_3$ | *i*-$C_4H_9$ | H | H | H | H | H | A | |
| $CH_3$ | *t*-$C_4H_9$ | H | H | H | H | H | A | |
| $CH_3$ | cyclopropyl | H | H | H | H | H | A | |
| $CH_3$ | $CH_2CH{=}CH_2$ | H | H | H | H | H | A | |

TABLE I (continued)

| $R_1$ | $R_2$ | X | L | M | Q | $R_7$ | Procedure | mp°C |
|---|---|---|---|---|---|---|---|---|
| $CH_3$ | cyclohexyl | H | H | H | H | H | A | |
| | $(CH_2)_5$ | H | H | H | H | H | A | |
| $CH_3$ | $CH(CH_3)_2$ | Cl | H | H | H | H | | |
| $CH_3$ | $CH(CH_3)_2$ | $CH_3$ | H | H | H | H | | |
| $CH_3$ | $CH(CH_3)_2$ | F | H | H | H | H | | |
| $CH_3$ | $CH(CH_3)_2$ | $OCH_3$ | H | H | H | H | | |
| $CH_3$ | $CH(CH_3)_2$ | OH | H | H | H | H | | |
| $CH_3$ | $CH(CH_3)_2$ | H | H | Cl | | H | A | 202.5—203.5 |

Example 41

Preparation of 1,3-Dihydro-$\alpha$-isopropyl-$\alpha$-methyl-1,3-dioxo-2-*H*-pyrrolo [3,4-*b*]quinoline-2-acetamide

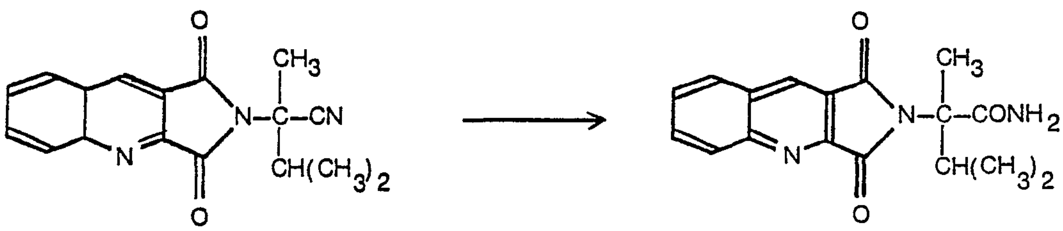

1,3-Dihydro-$\alpha$-isopropyl-$\alpha$-methyl-1,3-dioxo-2-*H*-pyrrolo [3,4-*b*]quinoline-2-acetonitrile (0.44 g, 0.0015 mol) is dissolved in conc. sulfuric acid (5 ml) at room temperature and stirred overnight. The reaction mixture is poured onto crushed ice (50 ml) and a white precipitate forms and is filtered off, washed with water, aqueous sodium bicarbonate and water and then vacuum dried. This gives 0.34 g (74%) of product, mp 237—239°C (dec.). Anal. calcd. for $C_{17}H_{17}N_3O_3$: C, 65.58; H, 5.50; N, 13.50. Found: C, 65.03; H, 5.63; N, 13.19.

The following compounds are prepared in the same manner as described above.

TABLE II

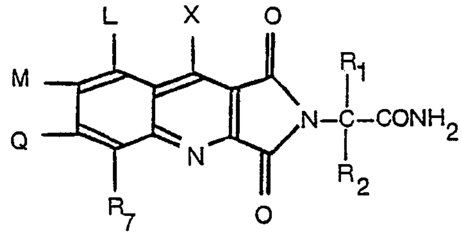

| $R_1$ | $R_2$ | X | L | M | Q | $R_7$ | mp°C |
|---|---|---|---|---|---|---|---|
| $CH_3$ | $CH(CH_3)_2$ | H | H | $NO_2$ | H | H | 225—227 (dec.) |
| $CH_3$ | $CH(CH_3)_2$ | H | H | H | $NO_2$ | H | 221—224 |
| $CH_3$ | $CH(CH_3)_2$ | H | $NO_2$ | H | H | H | |
| $CH_3$ | $CH(CH_3)_2$ | H | Br | H | H | H | |
| $CH_3$ | $CH(CH_3)_2$ | H | H | Cl | H | H | |
| $CH_3$ | $CH(CH_3)_2$ | H | H | $CF_3$ | H | H | |
| $CH_3$ | $CH(CH_3)_2$ | H | H | H | $CF_3$ | H | |
| $CH_3$ | $CH(CH_3)_2$ | H | H | H | Cl | H | 232—234 |
| $CH_3$ | $CH(CH_3)_2$ | H | H | H | H | Cl | |
| $CH_3$ | $CH(CH_3)_2$ | H | H | $CH_3$ | H | H | |
| $CH_3$ | $CH(CH_3)_2$ | H | H | H | $CH_3$ | H | 223—227(dec.) |
| $CH_3$ | $CH(CH_3)_2$ | H | H | H | H | $CH_3$ | |
| $CH_3$ | $CH(CH_3)_2$ | H | H | $OCH_3$ | H | H | |
| $CH_3$ | $CH(CH_3)_2$ | H | H | H | $CH_3$ | $CH_3$ | |
| $CH_3$ | $CH(CH_3)_2$ | H | H | $CH_3$ | H | $CH_3$ | |
| $CH_3$ | $CH(CH_3)_2$ | OH | H | H | H | H | |
| $CH_3$ | $CH(CH_3)_2$ | OAc | H | H | H | H | |
| $CH_3$ | $CH(CH_3)_2$ | H | Cl | H | H | H | 198—199 (dec.) |

Example 42

Preparation of 2-(5-Isopropyl-5-methyl-4-oxo-2-imidazolin-2-yl)-3-quinolinecarboxylic acid

Procedure A

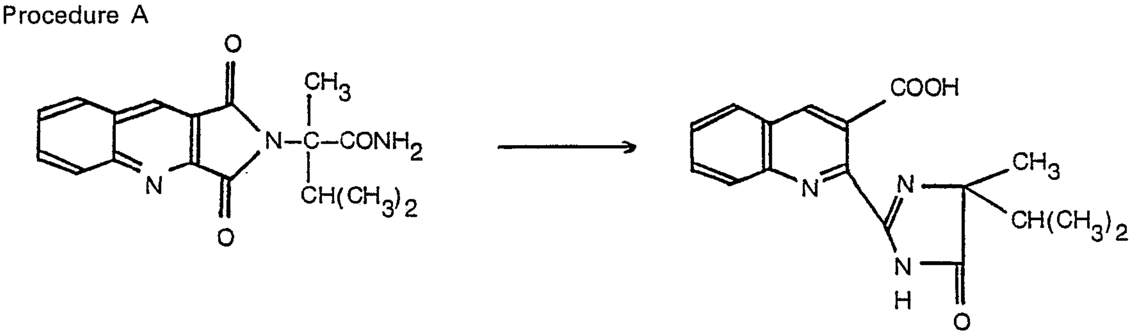

To a slurry of 1,3-dihydro-$\alpha$-isopropyl-$\alpha$-methyl-1,3-dioxo-2-H-pyrrolo [3,4-b] quinoline-2-acetamide (5.76 g, 0.0185 mol) in dry xylene (600 ml) is added a 50% oil dispersion of sodium hydride (1.33 g, 0.0278 mol) and the mixture is heated to reflux, whereupon the reaction mixture becomes homogeneous. After 3 hours at reflux the reaction is set aside at room temperature overnight and then methanol (15 ml) containing sodium methoxide (0.1 g) is slowly added and warmed at reflux for 1 hour. The mixture is filtered while hot and the organic solvents stripped to give an oil and solid. A methylene chloride-water mixture is shaken with the above residues, until they dissolve. The aqueous layer (200 ml) is separated and slowly acidified with acetic acid (5 ml). A precipitate of the product is formed and is collected by filtration to give 3.91 g (72%) of mp 219—224°C. Recrystallization from hexane-ethyl acetate gives mp 219—222°C (dec.). Anal. calcd. for $C_{17}H_{17}N_3O_3$: C, 65.58; H, 5.50; N, 13.50. Found: C, 65.09: H, 5.50; N, 13.59.

This example is used to prepare the following examples. However to avoid ester formation, rather than filter off the insolubles and strip down the xylene one may simply add methanol followed by water (caution hydrogen may be evolved) to the xylene layer.

TABLE III

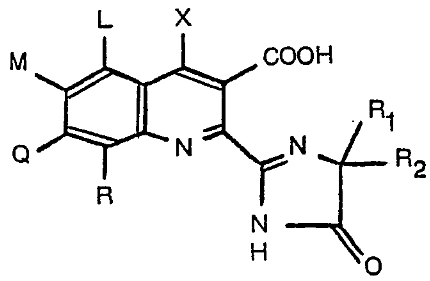

| $R_1$ | $R_2$ | X | L | M | Q | R | Procedure | mp°C |
|---|---|---|---|---|---|---|---|---|
| $CH_3$ | $CH(CH_3)_2$ | H | H | H | $NO_2$ | H | A | 247—251 |
| $CH_3$ | $CH(CH_3)_2$ | H | H | $NO_2$ | H | H | A | 241.5—242 |
| $CH_3$ | $CH(CH_3)_2$ | H | $NO_2$ | H | H | H | A | |
| $CH_3$ | $CH(CH_3)_2$ | H | H | H | H | $NO_2$ | A | |
| $CH_3$ | $CH(CH_3)_2$ | H | Br | H | H | H | A | |
| $CH_3$ | $CH(CH_3)_2$ | H | H | Cl | H | H | A | |
| $CH_3$ | $CH(CH_3)_2$ | H | H | H | Cl | H | A | 238—240 |
| $CH_3$ | $CH(CH_3)_2$ | H | H | H | H | Cl | A | |
| $CH_3$ | $CH(CH_3)_2$ | Cl | H | H | H | H | A | |
| $CH_3$ | $CH(CH_3)_2$ | H | Cl | H | H | H | A | 255—256 (dec.) |
| $CH_3$ | $CH(CH_3)_2$ | H | H | $CF_3$ | H | H | A | |
| $CH_3$ | $CH(CH_3)_2$ | H | H | H | $CF_3$ | H | A | |
| $CH_3$ | $CH(CH_3)_2$ | H | H | F | H | H | A | |
| $CH_3$ | $CH(CH_3)_2$ | H | H | $CH_3$ | H | H | A | |
| $CH_3$ | $CH(CH_3)_2$ | H | H | H | $CH_3$ | H | A | |
| $CH_3$ | $CH(CH_3)_2$ | H | H | H | H | $CH_3$ | A | |
| $CH_3$ | $CH(CH_3)_2$ | $CH_3$ | H | H | H | H | A | |
| $CH_3$ | $CH(CH_3)_2$ | H | H | $OCH_3$ | H | H | A | |
| $CH_3$ | $CH(CH_3)_2$ | H | H | $SCH_3$ | H | H | A | |
| $CH_3$ | $CH(CH_3)_2$ | H | H | $SO_2CH_3$ | H | H | (peracid on above) | |
| $CH_3$ | $CH(CH_3)_2$ | F | H | H | H | H | A | |
| $CH_3$ | $CH(CH_3)_2$ | H | H | H | $CH_3$ | $CH_3$ | A | |

Example 43

Preparation of Ethyl-2-(5-isopropyl-5-methyl-4-oxo-2-imidazolin-2-yl)-3-quinolinecarboxylic acid

Procedure A

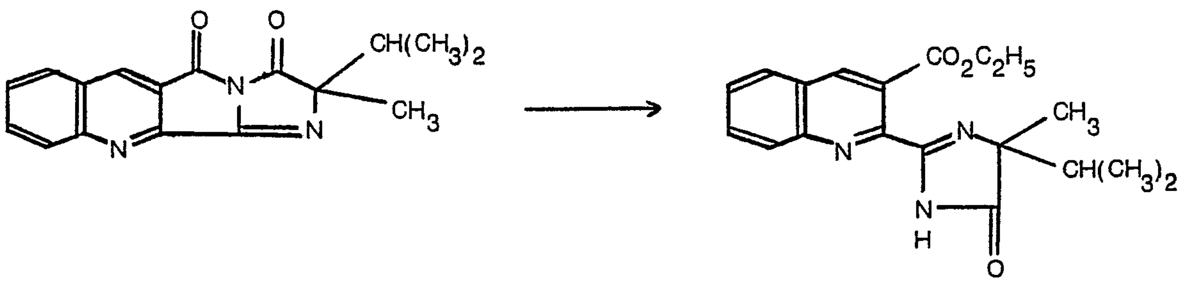

To 2-isopropyl-2-methyl-5-*H*-imidazo[1',2':1,2]pyrazolo [3,4-*b*]quinoline-3H(2H),5-dione (2 g, 0.0068 mol) in absolute ethanol (40 ml) under nitrogen is added 50% sodium hydride (0.34 g, 0.00716 mol) with ice-cooling. Gas evolution is observed. After 10 minutes the reaction is neutralized with aqueous ammonium chloride, stripped and partitioned between water and ethyl acetate. The organic layer is separated and dried over anhydrous magnesium sulfate, filtered, stripped and the residue crystallized from ethyl acetate-hexane to give 1.38 g (60%) of a white solid, mp 146—147.5°C.

In a similar manner, the following esters in Table IV may be prepared by Procedure A.

Example 44

Preparation of Methyl-2-(5-isopropyl-5-methyl-4-oxo-2-imidazolin-2-yl)-3-quinolinecarboxylic acid

Procedure B

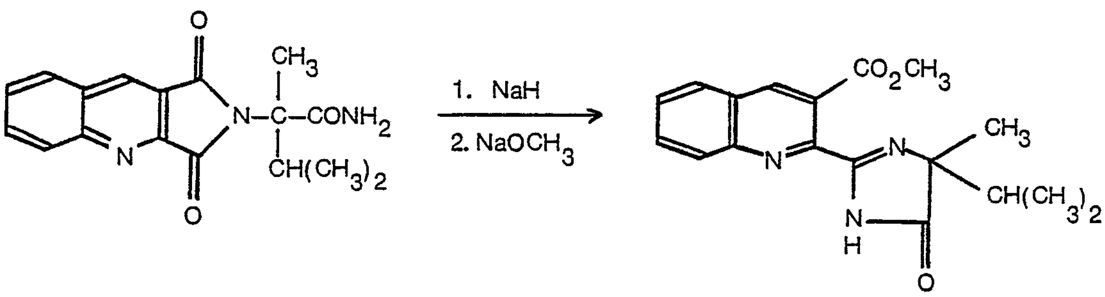


A 50% sodium hydride oil dispersion (1.4 g, 0.0292 mol) is added to azeotropically dried 1,3-dihydro-$\alpha$-isopropyl-$\alpha$-methyl-1,3-dioxo-2-H-pyrrolo [3,4-*b*] quinoline-2-acetamide (6 g, 0.0193 mol) under nitrogen. The mixture is heated and stirred under reflux for 6 hours, cooled and slowly quenched with a solution of sodium methoxide (0.1 g) in methanol (20 ml). After heating at 60°C for 3 hours the mixture is filtered and the filtrate stripped to give a white solid, which is dissolved in a methylene chloride-water mixture. Separation of the organic layer and stripping afforded a solid of 0.48 g, which is purified by passing through a silica gel pad with ethyl acetate as solvent. After removal of the solvent, the solid residue is crystallized from ethyl acetate-hexane to give white needles of the required ester, 0.4 g, mp 145—154°C. Anal. calcd. for $C_{18}H_{19}N_3O_3$: C, 66.44; H, 5.89; N, 12.92. Found: C, 66.35; H, 5.93; N, 12.83.

TABLE IV

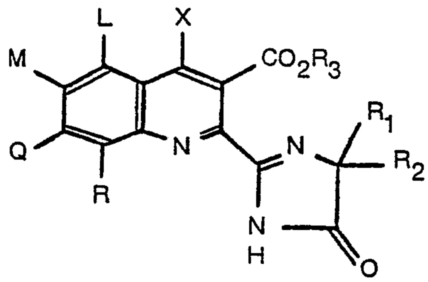

| $R_3$ | $R_1$ | $R_2$ | X | L | M | Q | R | mp°C | Example Procedure |
|---|---|---|---|---|---|---|---|---|---|
| $CH_3$ | $CH_3$ | $CH(CH_3)_2$ | H | H | H | H | H | 145—154 | B |
| $C_2H_5$ | $CH_3$ | $CH(CH_3)_2$ | H | H | H | H | H | 146—147.5 | A |
| $CH(CH_3)_2$ | $CH_3$ | $CH(CH_3)_2$ | H | H | H | H | H | | |
| $CH_2CH{=}CH_2$ | $CH_3$ | $CH(CH_3)_2$ | H | H | H | H | H | | |
| $CH_2$ 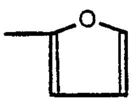 | $CH_3$ | $CH(CH_3)_2$ | H | H | H | H | H | | |
| $CH_2C_6H_5$ | $CH_3$ | $CH(CH_3)_2$ | H | H | H | H | H | | |
| $C_6H_5$ | $CH_3$ | $CH(CH_3)_2$ | H | H | H | H | H | | |
| $C_8H_{17}$ | $CH_3$ | $CH(CH_3)_2$ | H | H | H | H | H | | |
| $CH_3$ | $CH_3$ | $C_2H_5$ | H | H | H | H | H | | |
| $CH_2$ 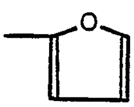 | $CH_3$ | $C_2H_5$ | H | H | H | H | H | | A |
| $CH_3$ | $CH_3$ | $CH(CH_3)_2$ | H | H | $CH_3$ | H | H | | |
| $CH_3$ | $CH_3$ | $CH(CH_3)_2$ | H | H | H | Cl | H | | |
| $CH_3$ | $CH_3$ | $CH(CH_3)_2$ | H | H | $N(CH_3)_2$ | H | H | | |
| $C_4H_9$ | $CH_3$ | $CH(CH_3)_2$ | H | H | H | H | H | 133.5—134.5 | A |
| $CH_3$ | $CH_3$ | $C_2H_5$ | H | H | H | H | H | | |
| $CH_3$ | $CH_3$ | 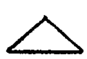 | H | H | H | H | H | | |
| $CH_3$ | $CH_3$ | *sec*-$C_4H_9$ | H | H | H | H | H | | |
| $CH_3$ | $-(CH_2)_5-$ | | H | H | H | H | H | | |

Example 45

Acid Salts of Quinolinecarboxylic acid esters:

Preparation of the hydrochloride salt of methyl 2-(5-isopropyl-5-methyl-4-oxo-2-imidazolin-2-yl)-3-quinoline carboxylic acid

A solution of the ester is dissolved in an ether-methylene chloride solution and dry hydrogen chloride is passed through the solution until all of the solid hydrochloride salt forms and is filtered off, ether washed and vacuum dried, with mp 226—270°C. The following salts may be prepared in a similar manner, substituting the appropriate acid HX, although some are hygroscopic oils, and employing ethyl acetate as a preferred solvent for acid salts.

TABLE V

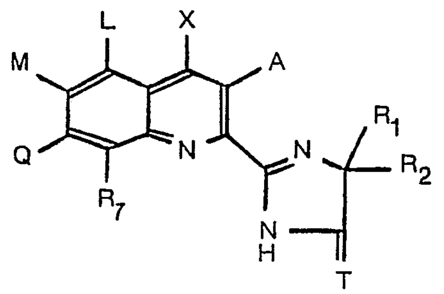

H acid

| $R_1$ | $R_2$ | T | A | L | M | Q | $R_7$ | H Acid | mp°C |
|---|---|---|---|---|---|---|---|---|---|
| $CH_3$ | $CH(CH_3)_2$ | O | $CO_2CH_3$ | H | H | H | H | HCl | |
| $CH_3$ | $CH(CH_3)_2$ | S | $CO_2CH_3$ | H | H | H | H | HCl | |
| $CH_3$ | $CH(CH_3)_2$ | O | $CO_2CH_3$ | H | H | H | H | HBr | |
| $CH_3$ | $CH(CH_3)_2$ | O | $CO_2CH_3$ | H | H | H | H | $HNO_3$ | |
| $CH_3$ | $CH(CH_3)_2$ | O | $CO_2CH_3$ | H | H | H | H | $H_2SO_4$ | |
| $CH_3$ | $CH(CH_3)_2$ | O | $CO_2H$ | H | H | H | H | HCl | 266—270 |
| $CH_3$ | $CH(CH_3)_2$ | O | CHO | H | H | H | H | HCl | |
| $CH_3$ | $CH(CH_3)_2$ | O | $CH_2OH$ | H | H | H | H | HCl | |
| $CH_3$ | $CH(CH_3)_2$ | O | $CO_2CH_3$ | H | H | Cl | H | HCl | |

Example 46

Preparation of Sodium 2-(5-isopropyl-5-methyl-4-oxo-2-imidazolin-2-yl)-3-quinolinecarboxylic acid

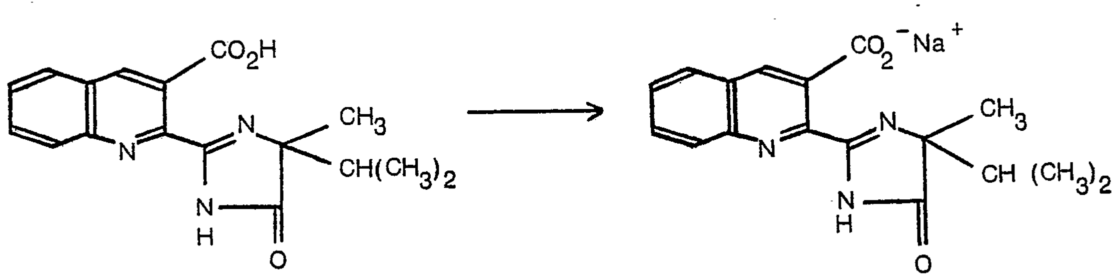

A solution of 2-(5-isopropyl-5-methyl-4-oxo-2-imidazolin-2-yl)-3-quinolinecarboxylic acid (2.33 g, 0.0075 mol) in water (22 ml) containing sodium hydroxide (0.3 g, 0.0075 mol) is stirred at room temperature overnight, then washed with methylene chloride and the aqueous layer separated and evaporated to an orange solid, which is washed with ether and air dried. The product as a dihydrate is obtained as a cream solid, mp 235—250°C (dec.). Anal. calcd. for $C_{17}H_{16}N_3O_3Na+2\ H_2O$: C, 55.27; H, 5.45; N, 11.37; Na, 6.22. Found: C, 55.56; H, 5.31; N, 11.35; Na, 6.30.

Substituting for sodium hydroxide the following salts are prepared in a similar manner. Compounds prepared in this manner are described in Table VI.

TABLE VI

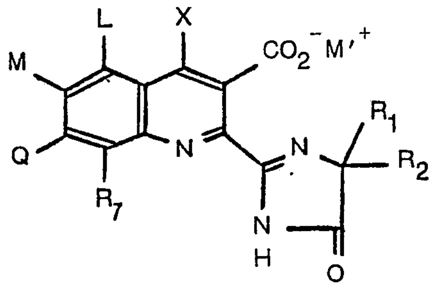

| $R_1$ | $R_2$ | X | L | M | Q | $R_7$ | M′ | mp°C |
|---|---|---|---|---|---|---|---|---|
| $CH_3$ | $(CH_3)_2CH$ | H | H | H | H | H | Na | 235—250 |
| $CH_3$ | $(CH_3)_2CH$ | | | | | | K | |
| $CH_3$ | $(CH_3)_2CH$ | H | H | H | H | H | $NH_4$ | 128—137 |
| $CH_3$ | $(CH_3)_2CH$ | H | H | H | H | H | $H_3NCH_3$ | |
| $CH_3$ | $(CH_3)_2CH$ | H | H | H | H | H | $H_2N(CH_3)_2$ | |
| $CH_3$ | $(CH_3)_2CH$ | H | H | H | H | H | $HN(CH_3)_3$ | |
| $CH_3$ | $(CH_3)_2CH$ | H | H | H | H | H | $H_2N(C_3H_7\text{-}i)_2$ | 237—239 |
| $CH_3$ | $(CH_3)_2CH$ | H | H | H | H | H | $NH_3CH_2CH{=}CH_2$ | |
| $CH_3$ | $(CH_3)_2CH$ | H | H | H | H | H | $NH_3CH_2C{\equiv}CH$ | |
| $CH_3$ | $(CH_3)_2CH$ | H | H | H | H | H | $NH_3C_6H_5$ | |
| $CH_3$ | $(CH_3)_2CH$ | H | H | H | H | H | $NH_3C_8H_{17}$ | |
| $CH_3$ | $(CH_3)_2CH$ | H | H | H | H | H | $NH_3C_{18}H_{37}$ | |
| $CH_3$ | $(CH_3)_2CH$ | H | H | H | H | H | $NH_3C_3H_7\text{-}i$ | |
| $CH_3$ | $(CH_3)_2CH$ | H | H | H | H | H | $NH_3$—$CH_2$ 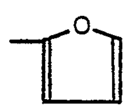 | |
| $CH_3$ | $(CH_3)_2CH$ | H | H | H | H | H | Ca | 270—290 |
| $CH_3$ | $(CH_3)_2CH$ | H | H | H | Cl | H | Ca | |
| $CH_3$ | $(CH_3)_2CH$ | H | H | H | $CH_3$ | H | $NH_2(C_3H_7i)_2$ | |
| $CH_3$ | $C_2H_5$ | H | H | H | H | H | $NH_3C_8H_{17}$ | |
| —$(CH_2)_5$— | | H | H | H | H | H | $NH_3C_{18}H_{37}$ | |

Example 47

Preparation of 2-Isopropyl-2-methyl-5-H-imidazo[1',2':1,2]-pyrazolo 3,4-b quinoline 3(2H),5-dione, 2-isopropyl-2-methyl

Procedure A

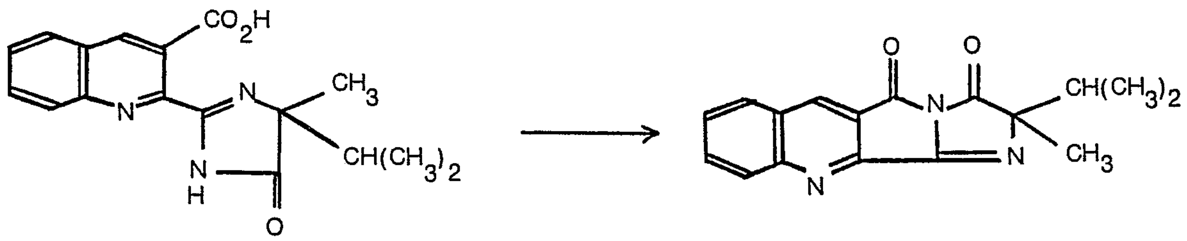

Dicyclohexylcarbodiimide (3.47 g, 0.0168 mol) in methylene chloride under nitrogen is added to a stirred suspension of 2-(5-isopropyl-5-methyl-4-oxo-2-imidazolin-2-yl)-3-quinolinecarboxylic acid, (5.24 g, 0.0168 mol) in methylene chloride at room temperature overnight. Since reaction was incomplete a further 0.3 g of dicyclohexylcarbodiimide was added and the mixture stirred for a further 48 hours. The reaction mixture is evaporated to a yellow solid and is purified by chromatography on a silica gel column. The product elutes with acetonitrile-methylene chloride as a white solid, which is crystallized from toluene as mp 225—227°C. Anal. calcd. for $C_{17}H_{15}N_3O_2$: C, 69.61; H, 5.15; N, 14.33. Found: C, 69.76; H, 5.31; N, 14.13.

Example 48

Procedure B

Preparation of cis and trans 1,11b-Dihydro-11b-hydroxy-3-isopropyl-3-methyl-5-H-imidazol[1',2':1,2]-pyrrolo[3,4-b]quinoline-2(3H),5-dione

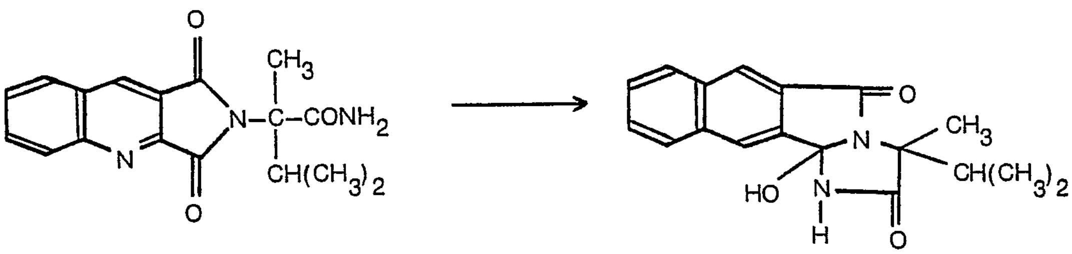

A solution of 1,3-dihydro-$\alpha$-isopropyl-$\alpha$-methyl-1,3-dioxo-2-H-pyrrolo[3,4-*b*]quinoline-2-acetamide (0.5 g, 0.0016 mol) was heated under reflux in xylene for 23 hours. On cooling, a white solid 0.17 g, mp 191—192°C precipitates and a further crop of 0.1 g, mp 187—189°C is formed by dilution of the filtrate by hexane. Anal. calcd. for $C_{17}H_{17}N_3O_3$: C, 65.58; H, 5.50; N, 13.50. Found: C, 66.08; H, 5.65; N, 13.00.

Other tricycles are obtained by procedures similar to Procedures A and B above.

Example 49

Preparation of N-(1-cyano-1,2-dimethylpropyl-2-(o-formylanilino)maleimide

Procedure A

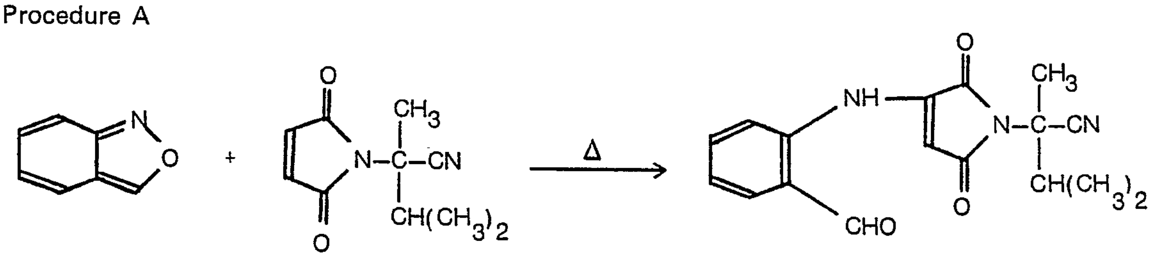


A solution of anthranil (3.55 g, 0.0298 mol) and $\alpha$-isopropyl-$\alpha$-methyl-2,5-dioxo-3-pyrroline-1-acetonitrile (5.73 g, 0.0298 mol) in xylene (20 ml) is heated at reflux for 39 hours under nitrogen. On cooling a yellow precipitate forms and is filtered off to give 2.78 g of mp 191—192°C product. Anal calcd. for $C_{17}H_{17}N_3O_3$: C, 65.58; H, 5.50; N, 13.50. Found: C, 65.33; H, 5.44; N, 13.36.

Example 50

Preparation of N-(1-cyano-1,2-dimethylpropyl)-2-(2-formyl-5-chloroanilino)maleimide

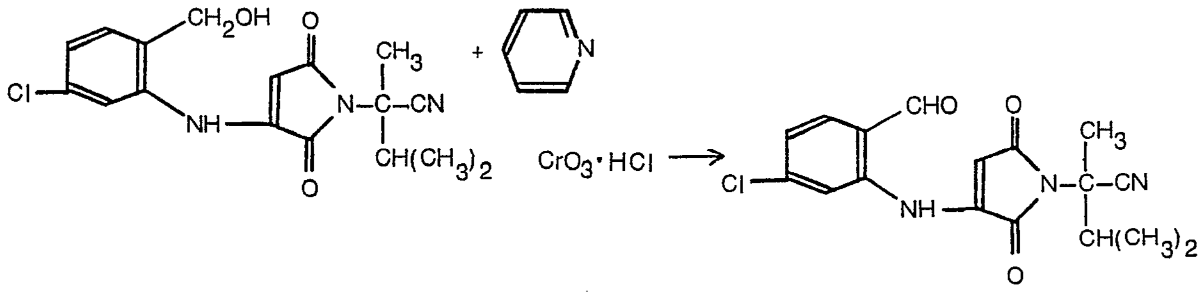

Pyridinium chlorochromate (4.4 g, 0.0204 mol) in methylene dichloride (20 ml) is added rapidly to a methylene chloride (20 ml) solution of N-(1-cyano-1,2-dimethylpropyl)-2-(5-chloro-2-hydroxymethylanilino)maleimide (4.75, g, 0.0136 mol). After 2 hours the dark reaction mixture is diluted with ether (20 ml) and a yellow precipitate is formed and is filtered off. This solid is redissolved in ethyl acetate:methylene chloride (1:1) and is passed through a silica gel column to give a yellow solid 4.31 g, (92%) with mp 80°C (dec.).

The following aldehydes are prepared according to Procedures A or B as shown in Table VII.

TABLE VII

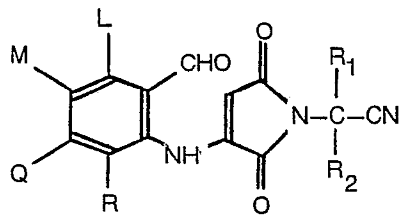

| $R_1$ | $R_2$ | L | M | Q | R | Example Method | mp°C |
|---|---|---|---|---|---|---|---|
| $CH_3$ | $CH(CH_3)_2$ | Cl | H | H | H | | |
| $CH_3$ | $CH(CH_3)_2$ | H | Cl | H | H | 50B | |
| $CH_3$ | $CH(CH_3)_2$ | H | H | Cl | H | 49A | 80 |
| $CH_3$ | $CH(CH_3)_2$ | H | H | H | Cl | | |
| $CH_3$ | $CH(CH_3)_2$ | $CH_3$ | H | H | H | | |
| $CH_3$ | $CH(CH_3)_2$ | H | $CH_3$ | H | H | 50B | |
| $CH_3$ | $CH(CH_3)_2$ | H | H | $CH_3$ | H | 50B | 205—212 |
| $CH_3$ | $CH(CH_3)_2$ | H | H | H | $CH_3$ | | |
| $CH_3$ | $CH(CH_3)_2$ | H | H | H | $OCH_3$ | | |
| $CH_3$ | $CH(CH_3)_2$ | H | H | Cl | $CH_3$ | | |
| $CH_3$ | $CH(CH_3)_2$ | Cl | H | H | $CH_3$ | | |
| $CH_3$ | $CH(CH_3)_2$ | H | Cl | H | $CH_3$ | | |
| $CH_3$ | $CH(CH_3)_2$ | Cl | H | H | $OCH_3$ | | |
| $CH_3$ | $CH(CH_3)_2$ | $CH_3$ | H | H | $CH_2$ | | |
| $CH_3$ | $CH(CH_3)_2$ | H | $CH_3$ | H | $CH_3$ | | |

Example 51

Preparation of N-(1-cyano-1,2-dimethylpropyl)-2-(2-hydroxymethylanilino)maleimide

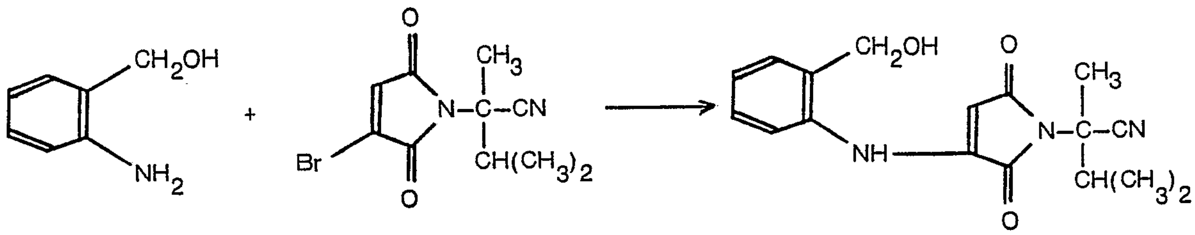

To o-aminobenzyl alcohol, (2 g, 0.0125 mol) and 3-bromo-$\alpha$-isopropyl-$\alpha$-methyl-2,5-dioxo-3-pyrroline-1-acetonitrile (2.7 g, 0.01 mol) is added absolute ethanol (100 ml) containing 3 g of 5A pulverized sieves. The mixture is stirred for 20 hours at room temperature. The solvent is removed and the residue is purified through a silica gel dry column, eluant ether-hexane (2:1). Starting bromomaleimide is first recovered, followed by a bright yellow solid 1.89 g (60%), mp 39—45°C. Anal. calcd. for $C_{17}H_{19}N_3O_3$: C, 65.16; H, 6.11; N, 13.41. Found: C, 65.94; H, 6.21; N, 12.87.

Other compounds are prepared by the above procedure with variously substituted *o*-aminobenzyl-alcohols. Employing *i*-propanol or *t*-butanol for ethanol generally improves the product yield and bases as acid acceptors may also be employed.

TABLE VIII

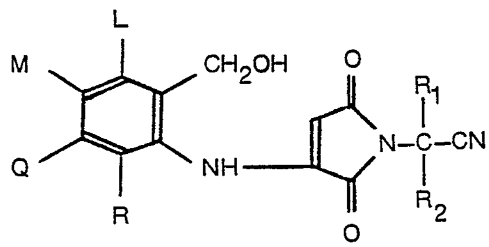

| $R_1$ | $R_2$ | L | M | Q | R | mp°C |
|---|---|---|---|---|---|---|
| $CH_3$ | $CH(CH_3)_2$ | H | H | H | $CH_3$ | |
| $CH_3$ | $CH(CH_3)_2$ | H | H | $CH_3$ | H | gum |
| $CH_3$ | $CH(CH_3)_2$ | H | $CH_3$ | H | H | |
| $CH_3$ | $CH(CH_3)_2$ | $CH_3$ | H | H | H | |
| $CH_3$ | $CH(CH_3)_2$ | H | H | H | Cl | |
| $CH_3$ | $CH(CH_3)_2$ | H | H | Cl | H | 98—100 |
| $CH_3$ | $CH(CH_3)_2$ | H | Cl | H | H | |
| $CH_3$ | $CH(CH_3)_2$ | Cl | H | H | H | |
| $CH_3$ | $CH(CH_3)_2$ | H | H | H | $OCH_3$ | |
| $CH_3$ | $CH(CH_3)_2$ | H | H | Cl | $CH_3$ | |
| $CH_3$ | $CH(CH_3)_2$ | H | Cl | H | $CH_3$ | |
| $CH_3$ | $CH(CH_3)_2$ | Cl | H | H | $OCH_3$ | |
| $CH_3$ | $CH(CH_3)_2$ | $CH_3$ | H | H | $CH_3$ | |
| $CH_3$ | $CH(CH_3)_2$ | H | $CH_3$ | H | $CH_3$ | |
| $CH_3$ | $CH(CH_3)_2$ | H | H | $CH_3$ | $CH_3$ | |

Example 52

Preparation of 3-Bromo-$\alpha$-isopropyl-$\alpha$-methyl-2,5-dioxo-3-pyrroline-1-acetonitrile

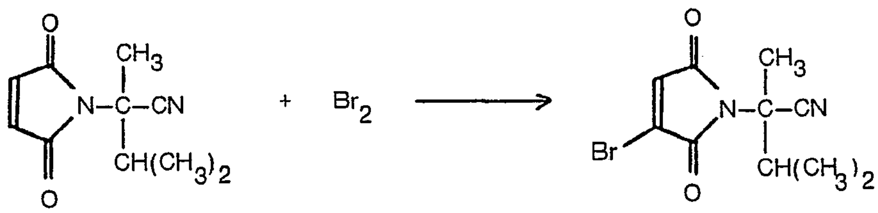

To a solution of $\alpha$-isopropyl-$\alpha$-methyl-2,5-dioxo-3-pyrroline-1-acetonitrile (50 g, 0.25 mol) in acetic acid (500 ml) heated at 75°C is added bromine (40.76 g, 0.255 mol) in acetic acid (80 ml) dropwise with stirring. The reaction is maintained at 85° overnight and evaporated to a syrup, which is dissolved in methylene chloride (300 ml), is cooled to 5°C to which triethylamine (34.78 ml) is added. After stirring for 2 hours the brown methylene chloride solution is diluted with ether and a white precipitate is formed. This is extracted with water (400 ml) and the organic layer is dried over anhydrous magnesium sulfate, then passed through a 2 inch bed of silica gel with methylene chloride to elute. The eluate is obtained as a dark brown oil. Anal. calcd. for $C_{10}H_{10}N_2O_2Br$: C, 44.29; H, 4.09; N, 10.33. Found: C, 43.37; H, 4.05; N, 10.07.

Example 53

Preparation of $\alpha$-isopropyl-$\alpha$-methyl-2,5-dioxo-3-pyrroline-1-acetonitrile

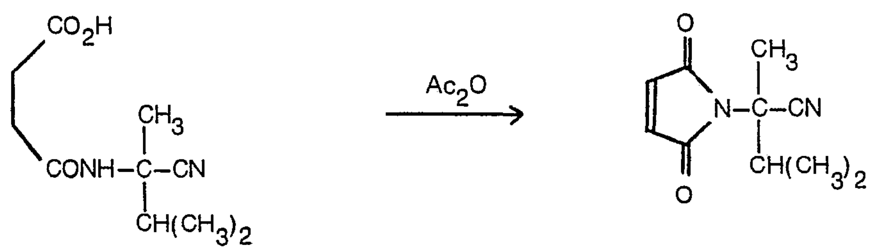


A solution of N-(1-cyano-1,2-dimethylpropyl)-maleamic acid (595 g, 2.83 mol) in acetic anhydride (3.96 liters) containing sodium acetate (13.72 g, 0.167 mol) is heated under reflux for one hour, cooled and the solvent removed in vacuo. The product is distilled* at 120—130°C/0.1 mm to give 337 g (63%) of product. *The pot temperature should not exceed 200°. Anal. calcd. for $C_{10}H_{12}N_2O_2$: C, 62.49; H, 6.29; N, 14.57. Found: C, 62.32; H, 6.36; N, 14.59.

Example 54

Preparation of N-[1-(1-cyano-1,2-dimethylpropyl)-2,5-dioxo-3-pyrrolin-3-yl] anthranilic acid

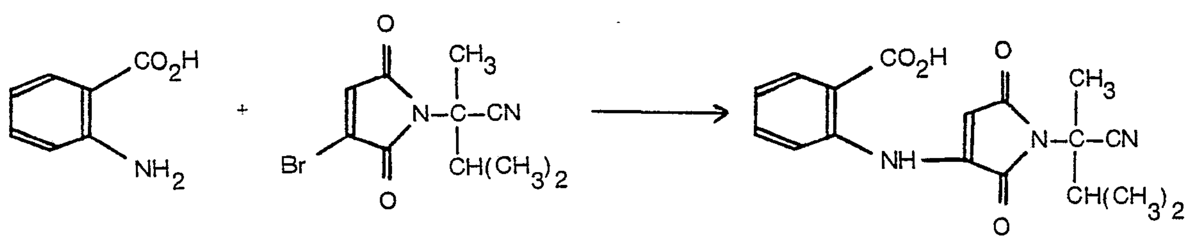

A mixture of anthranilic acid, (13.7 g, 0.1 mol), 3-bromo-$\alpha$-isopropyl-$\alpha$-methyl-2,5-dioxo-3-pyrroline-1-acetonitrile (27 g, 0.1 mol), isopropanol (200 ml) and sodium acetate (8.2 g) is stirred at room temperature 3 days and then is heated at reflux 1 hour. On cooling, and with the addition of ether, a yellow solid 31.6 g, (97.7%) is obtained, mp 262—266°C after crystallizing from acetic acid. Anal. calcd. for $C_{17}H_{17}N_3O_4$: C, 62.37; H, 5.24; N, 12.84. Found: C, 62.24; H, 5.19; N, 12.70.

Example 55

Preparation of N-(1-carbamoyl-1,2-dimethylpropyl)-quinaldamide

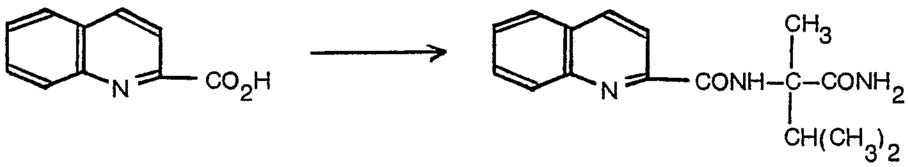

To a solution of quinaldic acid (20 g, 0.116 mol) in tetrahydrofuran (500 ml) cooled to −9°C is added methyl chloroformate (8.92 ml, 0.116 mol) followed by triethylamine (18.4 ml, 0.139 mol). After 20 minutes α-isopropyl-α-methyl-3-pyrroline-1-acetamide (15.1 g, 0.116 mol) is added and the mixture stirred overnight at room temperature. Water is added and the solution is reduced to 200 ml on a rotorvap. A white solid separates and is filtered off, water washed and dried. Recrystallization from absolute ethanol gives the product mp 179—180°C, 26.86 g (87%). Anal. calcd. for $C_{16}H_{19}N_3O_2$: C, 67.34; H, 6.73; N, 14.72. Found: C, 67.14; H, 6.17; N, 14.72.

Example 56

Preparation of α-Isopropyl-α-methyl-2,5-dioxo-3-pyrroline-1-acetamide

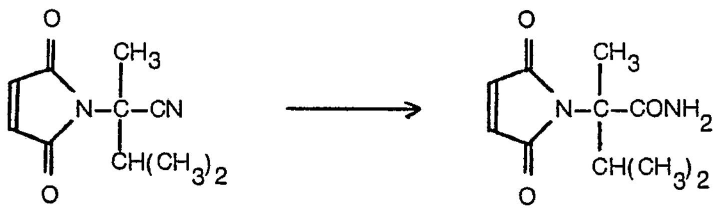

A solution of α-isopropyl-α-methyl-2,5-dioxo-3-pyrroline-1-acetonitrile (2.0 g, 0.104 mol) in methylene chloride (30 ml) is added in a fine stream to concentrated sulfuric acid at room temperature. After stirring overnight at room temperature for 16 hours the mixture is poured onto ice, containing sodium chloride and ethyl acetate. The organic layer is washed with aqueous sodium bicarbonate, brine and dried. Evaporation after washing with ether-pentane gives a solid (72%), mp 138.5—140°C. Anal. calcd. for $C_{10}H_{14}N_2O_3$: C, 57.13; H, 6.71; N, 13.33. Found: C, 56.89; H, 6.64; N, 13.16.

Example 57

Preparation of 5-Isopropyl-5-methyl-2-(2-quinolyl)-2-imidazolin-4-one

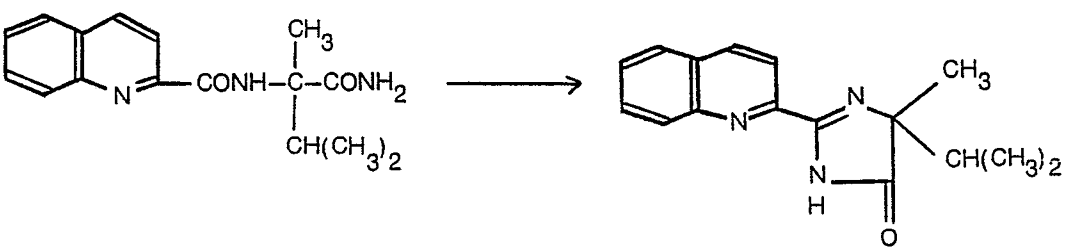

To a slurry of N-(1-carbamoyl-1,2-dimethylpropyl)-2-quinolinecarboxamide (16.04 g, 0.0562 mol) in xylene (610 ml) under nitrogen is added a 50% oil dispersion of sodium hydride (2.7 g, 0.056 mol) at 20°C. The reaction is heated to reflux for 2 hours, cooled and water (50 ml) added. The aqueous layer is extracted with methylene chloride and the organic layers combined and evaporated to give a yellow oil, 17 g. Purification is accomplished by passing through a silica gel column using hexane-ethyl acetate as solvent. A pale yellow solid is obtained and is recrystallized from ethyl acetate to give 11.77 g (78%) of white product, mp 112—117°C. Anal. calcd. for $C_{16}H_{17}N_3O$: C, 71.88; H, 6.41; N, 15.72. Found: C, 71.91; H, 6.47; N, 15.70.

Example 58

Preparation of 2-(5-isopropyl-5-methyl-4-oxo-2-imidazolin-2-yl)-3-quinolinecarboxaldehyde

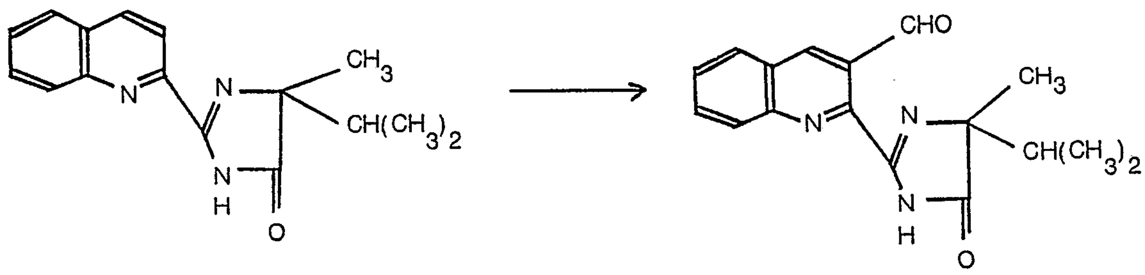

To a mixture of 5-isopropyl-5-methyl-2-(2-quinolyl)-2-imidazolin-4-one (3 g, 0.0112 mol) in ether (150 ml) is added tetramethylethylenediamine (3.4 g, 0.00225 mol). *n*-Butyllithium (17 ml, 0.027 mol) in hexane is added dropwise to the reaction mixture cooled to —63°C. An intense red color is generated and after the addition the mixture was maintained at —10 to —20°C for 2—1/2 hours. Dry DMF (5 ml) was added at —10°C and the mixture is allowed to attain room temperature, while stirring overnight. The mixture is diluted with water (75 ml) and is neutralized with acetic acid. A pale yellow solid 2.57 g (78%) is obtained with mp 226—227°C after crystallization* from 95% ethanol. Anal. calcd. for $C_{17}H_{17}N_3O_2$: C, 69.13; H, 5.80; N, 14.23. Found: C, 68.98; H, 5.88; N, 14.25.

*Dilution of the 95% ethanol with 5—10 ml of water gives a new solid which is filtered off. Washing the solid with 95% ethanol removes the yellow color and gives a material mp 168—169°C. An

$$\frac{m+l}{e}$$

confirms either tricyclic structure A or B.

Example 59

Preparation of 2- 3-(Hydroxymethyl)-2-quinolyl-5-isopropyl-5-methyl-2-imidazolin-4-one

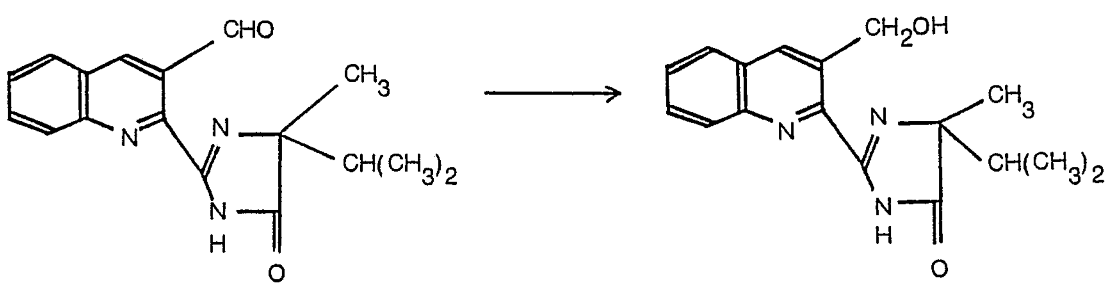

Powdered sodium borohydride (0.5 g, 0.013 mol) is added to 2-(5-isopropyl-5-methyl-4-oxo-2-imidazolin-2-yl)-3-quinolinecarboxaldehyde (0.78 g, 0.00264 mol) which is suspended in ethanol (150 ml) under nitrogen. A clear yellow solution results. After 20 minutes the reaction is concentrated to 40 ml, and is diluted with water (75 ml). Methylene chloride extraction and evaporation gives a solid which is crystallized from hexane-ethyl acetate to give pale yellow crystals, mp 138—149°C, M/e 298.

Other compounds may be prepared by the above procedure using the appropriately substituted quinoline-carboxaldehyde.

Example 60

Evaluation of the maturation effect on Barley caused by the compounds of the invention

In the following tests, the appropriate compounds are dissolved or dispersed in acetone-water (1:1) mixtures at the final concentration corresponding to the kg/ha rates indicated in the tables below. The solutions also contain 0.1% to 0.25% v/v colloidal BIOFILM® (a product of Colloidal Products Corp.) which is a mixture of alkyl aryl polyethoxyethanol, free and combined fatty acids, glycol ethers, dialkylbenzene carboxylate and 2-propanol.

The plant species used in these tests is barley (*Hordeum vulgare,* var. Mexico).

The solution or dispersion of the compound under test is sprayed at a rate of 40 ml per pot applied to the foliage.

In the postemergence tests, the plants are seedlings at the two leaf stage (19 plants per pot; pot size; 8.9 cm x 6.3 cm x 6.3 cm).

The pots were watered immediately before treatment and placed on benches in a random arrangement in the greenhouse. Normal watering and fertilizing practices are followed (pesticides are applied to the plants as needed). Minimum day and night temperatures of 18.3°C are maintained during cooler periods of the year. Normal daily fluctuations occur during the summer season.

Plants are sprayed to provide the kg/ha rates indicated in the tables below. Each treatment is replicated 6 times.

*Data Recording*

Periodic observations are made after treatment and morphological changes are noted. From these observations as compared to the untreated controls, the maturation effect of the instant compounds on barley can be determined.

The data thus obtained are averaged and summarized in Tables Ia to Ic inclusive.

TABLE Ia

Evaluation of the maturation effect of the compounds of the invention on barley

| Compound | Rate kg/ha | Results |
|---|---|---|
| Triethylammonium-2-(5-isopropyl-5-methyl-4-oxo-2-imidazolin-2-yl)-nicotinate | 0.01 | slight increase in tillering |
| | 0.002 | slight increase in tillering |
| Methyl-2-(5-isopropyl-5-methyl-4-oxo-2-imidazolin-2-yl)nicotinate | 0.01 | great increase in tillering |
| | 0.002 | earlier heading increased tillering |
| | 0.004 | earlier heading increased tillering |
| Calcium-2-(5-isopropyl-5-methyl-4-oxo-2-imidazolin-2-yl)nicotinate | 0.01 | Phytotoxic |
| | 0.002 | increased tillering |
| 2-(5-ethyl-5-methyl-4-oxo-2-imidazolin-2-yl)nicotinate | 0.01 | delayed heading increased tillering |

TABLE Ib

Evaluation of the maturation effect of the compounds of the invention on barley

| Compound | Rate kg/ha | Results |
|---|---|---|
| Methyl-2-(5-isopropyl-5-methyl-4-oxo-2-imidazolin-2-yl)nicotinate | 0.01 | slight increase in tillering |
| | 0.005 | slight increase in tillering |

TABLE Ic

Evaluation of the maturation effect of the compounds of the invention on barley

| Compound | Rate kg/ha | Results |
|---|---|---|
| Methyl-2-(5-isopropyl-5-methyl-4-oxo-2-imidazolin-2-yl)nicotinate | 0.1 | Phytotoxic |
| | 0.05 | Phytotoxic |
| | 0.01 | great increase in tillering with earlier head maturation |

## Example 61

Evaluation of the increased branching and flowering effect of the compounds of the invention.

By the method of Example 60, the effect of the compounds of the invention is evaluated on soybeans (*Glycine max*, var. ADELPHI). Each test is replicated six times. There is one plant per pot and the plants are treated at the 2nd to 3rd trifoliate stage.

The data obtained are averaged and summarized in Tables IIa to IIc inclusive.

TABLE IIa

Evaluation of the increased branching effect on soybeans of the compounds of the invention

| Compound | Rate kg/ha | Results |
|---|---|---|
| Methyl-2-(5-isopropyl-5-methyl-4-oxo-2-imidazolin-2-yl)nicotinate | 0.01 | greatly increased branching, blossoming delayed, greener leaves |
| | 0.002 | increased branching |
| Calcium-2-(5-isopropyl-5-methyl-4-oxo-2-imidazolin-2-yl)niconate | 0.01 | greatly increased branching, delayed blossoming, greener leaves |
| 2-(5-ethyl-5-methyl-4-oxo-2-imidazolin-2-yl)nicotinic acid | 0.01 | moderately increased branching |
| | 0.0004 | moderately increased branching |

TABLE IIb

Evaluation of increased branching and flowering effect on soybeans of the compounds of the invention

| Compound | Rate kg/ha | Results |
|---|---|---|
| Methyl-2-(5-isopropyl-5-methyl-4-oxo-2-imidazolin-2-yl)nicotinate | 0.01 | greatly increased branching; increased number of flowers, flowering delayed slightly |
| | 0.005 | greatly increased branching; moderately increased flowering |
| | 0.0025 | moderately increased branching; increased flowering |

TABLE IIc

Evaluation of increased tillering effect on soybeans of the compounds of the invention

| Compound | Rate kg/ha | Results |
|---|---|---|
| Methyl-2-(5-isopropyl-5-methyl-4-oxo-2-imidazolin-2-yl)nicotinate | 0.1 | Phytotoxic |
| | 0.05 | Phytotoxic |
| | 0.01 | greatly increased branching darker green leaves |

## Example 62

Evaluation of the increased yield effect of the compounds of the invention

By the method of Example 60, the effect of the compounds of the invention is evaluated on soybeans (*Glycine max.* var. ADELPHI). Each test is replicated 6 times. There is one plant per pot and the plants are treated at the 6th trifoliate stage. The data obtained are averaged and summarized in Table IX below.

TABLE IX

Evaluation of the increased yield effect of the compounds of the invention on soybeans (Average of 6 replicates)

| Compound | Rate kg/ha | No. of pods | % Increase (+) Decrease (–) | Fresh pod wt in g | % Increase (+) Decrease (–) | Dry pod wt in g | % Increase (+) Decrease (–) |
|---|---|---|---|---|---|---|---|
| Control | – | 99.7 | – | 126.2 | – | 32.4 | – |
| Methyl-2-(5-isopropyl-5-methyl-4-oxo-2-imidazolin-2-yl)nicotinate | 0.01<br>0.002<br>0.0004 | 27.3<br>115.0<br>122.8 | –73<br>+15<br>+23 | 28.5<br>126.3<br>149.6 | –77<br>0<br>+19 | 7.7<br>29.5<br>38.2 | –76<br>– 9<br>+18 |

## Example 63

Evaluation of the maturation effect on wheat caused by the compounds of the invention applied postemergence

By the method of Example 60, wheat (*Triticum aestivum,* var. Era) is treated at the early tillering stage at the kg/ha rates indicated in Table XII below. Each treatment is replicated 6 times.

*Data recording*

The plants are harvested five weeks post treatment, the number of heads, fresh and dry head weight and straw weight are determined and the % changes (±) indicated. The data obtained are averaged and summarized in Table X below.

TABLE X

Evaluation of the maturation effect on wheat, caused by the compounds of the invention, applied postemergence

| Compound | Rate kg/ha | Heads; No.; wt in g; % change | | | Straw; wt in g; % change |
|---|---|---|---|---|---|
| | | No. of heads; % change | Fresh wt in g; % change | Dry wt in g; % change | |
| Control | – | 30.2 | 64.3 | 30.1 | 100.1 |
| | | – | – | – | – |
| Methyl-2-(5-isopropyl-4-oxo-2-imidazolin-2-yl)-nicotinate | 0.01 | 37.0 | 77.3 | 30.8 | 173.5 |
| | | 22.5 | 20.2 | 2.3 | 73.3 |
| | 0.002 | 38.2 | 88.0 | 38.4 | 160.7 |
| | | 26.5 | 36.8 | 27.6 | 60.5 |
| | 0.004 | 31.5 | 62.2 | 28.2 | 110.8 |
| | | 4.3 | –3.1 | –6.3 | 10.7 |

## Example 64

Evaluation of the maturation effect on wheat caused by the compounds of the invention applied preemergence

The seeds of wheat (*Triticum aestivum,* var. Era) are mixed with potting soil and planted on top of approximately 2.5 cm of soil in 12.5 cm diameter fiber pots. After planting, the pots are sprayed with the aqueous acetone solution containing the test compound at the kg/ha rates given in Table XI below. The treated cups are then placed on greenhouse benches, watered and cared for in accordance with conventional greenhouse procedures. Each treatment is replicated six times.

*Data recording*

The plants are harvested 11 weeks post treatment, the number of heads, fresh and dry head weight and straw weight are determined and the % changes (±) indicated. The data obtained are averaged and summarized in Table XI below.

TABLE XI

Evaluation of the maturation effect on wheat, caused by the compounds of the invention, applied preemergence

| Compound | Rate kg/ha | Heads; No.; wt in g; % change | | | Straw wt in g; % change |
|---|---|---|---|---|---|
| | | No. of heads; % change | Fresh wt in g; % change | Dry wt in g; % change | |
| Control | – | 25.7 | 47.2 | 15.7 | 145.3 |
| | | – | – | – | – |
| Methyl-2-(5-iso-propyl-5-methyl-4-oxo-2-imidazolin-2-yl)-nicotinate | 0.1 | – | – | – | 101.6 |
| | | – | – | – | –30.1 |
| | 0.01 | 29.3 | 50.9 | 17.0 | 139.8 |
| | | 14.0 | 7.8 | 8.3 | –3.8 |
| | 0.001 | 28.8 | 53.0 | 18.0 | 135.3 |
| | | 12.1 | 12.3 | 14.6 | –6.9 |

Example 65

Evaluation of test compounds for increasing soybean yield

In the following tests, seeds of either *Era* variety wheat or *Adelphi* variety soybeans, are planted in 13 cm fiber pots. The test compounds are then dispersed in acetone-water (1:1) mixtures containing 0.1% to 0.25% v/v colloidal BIOFILM® which is a mixture of alkyl aryl polyethoxyethanol, free and combined fatty acids, glycol ethers, dialkylbenzene carboxylate and 2-propanol. The planted seeds are sprayed with a sufficient amount of the mixture to provide each pot with 0.1, 0.01, or 0.001 kg/ha of test compound. The treated cups are then placed on greenhouse benches and cared for in accordance with greenhouse procedures. Three months after treatment the soybeans are harvested, dried and weighed. Six replicates per treatment are used and averaged. Data obtained are reported below.

TABLE XII

Soybean yield enhancement evaluation

| Compound | Rate kg/ha | Dry Pod wt (g) | % Increase over controls |
|---|---|---|---|
| Untreated controls | — | 13 | — |
| Methyl 2-(5-isopropyl-5-methyl-4-oxo-2-imidazolin-2-yl)-nicotinate | 0.1 | 4.8 | |
| | 0.01 | 14.9 | +15 |
| | 0.001 | 15.3 | +18 |

Wheat yield enhancement evaluation

| Compound | Rate kg/ha | Dry Head wt (g) | % Increase over controls |
|---|---|---|---|
| Untreated controls | — | 15.7 | — |
| Methyl 2-(5-isopropyl-5-methyl-4-oxo-2-imidazolin-2-yl)-nicotinate | 0.1 | — | |
| | 0.01 | 17.0 | +8.2 |
| | 0.001 | 18.0 | +14.6 |
| Untreated control | — | 19.3 | — |
| Methyl 2-(5-isopropyl-5-methyl-4-oxo-2-imidazolin-2-yl)-nicotinate)·HCl | 0.016 | 11.3 | |
| | 0.008 | 21.5 | +11.4 |
| | 0.004 | 23.2 | +14.5 |

Example 66

Wheat yield enhancement evaluation, test compound applied post-emergence

The following tests were conducted using the procedure of Example 60, excepting that the plant species used is *Era* wheat and the crop is harvested nine weeks after treatment, the following results are obtained.

Wheat Yield Enhancement Evaluation

| Compound | Rate kg/ha | Dry Head wt (g) | % Increase over controls |
|---|---|---|---|
| Untreated controls | — | 30.1 | — |
| Methyl 2-(5-isopropyl-5-methyl-4-oxo-2-yl-nicotinate | 0.01 | 30.8 | +2.3 |
| | 0.002 | 38.4 | +28.1 |
| | 0.0004 | 28.2 | −6.3 |

**Claims**

1. A method to increase axillary branching, tillering, flowering and yield of agronomic and horticultural crops and accelerate maturation of cereal crops comprising: applying to said crops plants or to soil containing the seeds or other propagating organs of said plants, a compound having the structure:

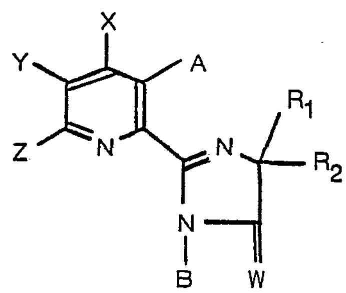

(I)

wherein

$R_1$ is $C_1$—$C_4$ alkyl;

$R_2$ is $C_1$—$C_4$ alkyl or $C_3$—$C_6$ cycloalkyl; and when $R_1$ and $R_2$ are taken together they may represent $C_3$—$C_6$ cycloalkyl optionally substituted with methyl;

A is $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

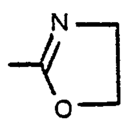

OR

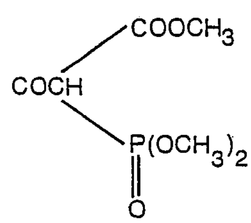

(II)

;

$R_3$ is hydrogen,

lower alkylidene amino

$C_1$—$C_{12}$ alkyl optionally substituted with one of the following groups: $C_1$—$C_3$ alkoxy, halogen, hydroxyl, $C_3$—$C_6$ cycloalkyl, benzyloxy, furyl, phenyl, halophenyl, loweralkylphenyl, loweralkoxyphenyl, nitrophenyl, carboxyl, loweralkoxycarbonyl, cyano or triloweralkylammonium;

$C_3$—$C_{12}$ alkenyl optionally substituted with one of the following groups: $C_1$—$C_3$ alkoxy, phenyl, halogen or loweralkoxycarbonyl or with two $C_1$—$C_3$ alkoxy groups or two halogen groups;

$C_3$—$C_6$ cycloalkyl optionally substituted with one or two $C_1$—$C_3$ alkyl groups;

$C_3$—$C_{10}$ alkynyl optionally substituted with one or two $C_1$—$C_3$ alkyl groups; or,

A cation selected from the group consisting of alkali metals, alkaline earth metals, manganese, copper, iron, zinc, cobalt, lead, silver, nickel, ammonium and organic ammonium;

$R_6$ is hydrogen, hydroxyl, $C_3$-alkenyl, $C_3$-alkynyl or $C_1$—$C_4$ alkyl optionally substituted with one hydroxyl or one chlorine group;

B is H, $COR_4$ or $SO_2R_5$, provided that when B is $COR_4$ or $SO_2R_5$; A is $COOR_3$ in which $R_3$ is other than H, or a salt-forming cation, $CH_3$ or CN; W is O; and Y and Z are not alkylamino, hydroxyl, or hydroxyloweralkyl;

$R_4$ is $C_1$—$C_{11}$ alkyl, chloromethyl or phenyl optionally substituted with one chloro, one nitro or one methoxy group;

$R_5$ is $C_1$—$C_4$ alkyl or phenyl optionally substituted with one methyl group;
W is O or S;
$R_8$ is $C_1$—$C_4$-alkyl or phenyl;
X is hydrogen, halogen, hydroxyl or methyl, with the proviso that when Y and Z are taken together to form a ring and YZ is represented by the structure: $-(CH_2)_n-$, where n is 3 or 4, X is hydrogen;
Y and Z each represent members selected from the group consisting of hydrogen, halogen, $C_1$—$C_6$ alkyl, hydroxy-loweralkyl, $C_1$—$C_6$ alkoxy, $C_1$—$C_4$ alkylthio, phenoxy, $C_1$—$C_4$-haloalkyl, nitro, cyano, $C_1$—$C_4$ alkylamino, diloweralkylamino or $C_1$—$C_4$ alkylsulfonyl group, or phenyl optionally substituted with one $C_1$—$C_4$ alkyl, $C_1$—$C_4$ alkoxy or halogen; and, when taken together, Y and Z may form a ring in which YZ are represented by the structure: $-(CH_2)_n-$, where n is an integer selected from 3 and 4, provided that X is hydrogen; or

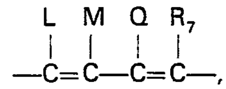

where L, M, Q and $R_7$ each represent members selected from the group consisting of hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $C_1$—$C_4$ alkylamino, diloweralkylamino, chlorophenyl, methylphenyl, or phenoxy substituted with one Cl, $CF_3$, $NO_2$ or $CH_3$ group, with the proviso that only one of L, M, Q or $R_7$, may represent a substituent other than hydrogen, halogen, $C_1$—$C_4$ alkyl or $C_1$—$C_4$-alkoxy.
and when W is O and A is CN, $CH_3$ or $COOR_3$, provided that $R_3$ cannot be unsaturated alkyl and Y and Z cannot be alkylamino, dialkylamino or alkylthio, and the N-oxides thereof, and when $R_1$ and $R_2$ are not the same, the optical isomers thereof, and, except when $R_3$ is a salt-forming cation, the acid addition salts thereof.

2. The method according to Claim 1, to induce tillering, accelerate the maturation and increase the yield of cereal crops comprising: applying to said plants or to the soil containing seeds thereof from 0.0001 kg/ha to 0.01 kg/ha of a compound of Claim 1.

3. The method according to Claim 1, to induce axillary branching, tillering and to increase the flowering, pod set and yield of legumes comprising: applying to said plants or to soil containing the seeds thereof from 0.0001 kg/ha to 0.01 kg/ha of a compound of Claim 1.

4. The method according to Claim 1, to increase axillary branching and flowering of herbaceous broadleaf ornamentals comprising: applying to the foliage of said plants or soil containing seeds or other propagating organs thereof, from 0.0001 kg/ha to 0.01 kg/ha of a compound of Claim 1.

5. A method according to Claim 1, wherein said compound has the structure:

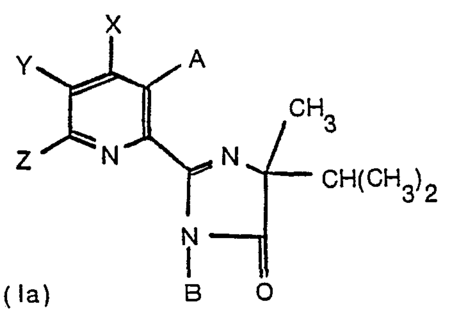
(Ia) ;

wherein B is hydrogen, CO-alkyl, $C_1$—$C_6$ or CO-phenyl; A is $COOR_3$ where $R_3$ is as described in formula (I) above; X is hydrogen and Y and Z each represent hydrogen, $C_1$—$C_6$ alkyl, $C_1$—$C_4$-alkoxy, halo, $C_1$—$C_4$-haloalkyl, or phenyl and, when taken together, YZ represent $-(CH_2)_4-$.

6. A method according to Claim 1, wherein said compound has the structure:

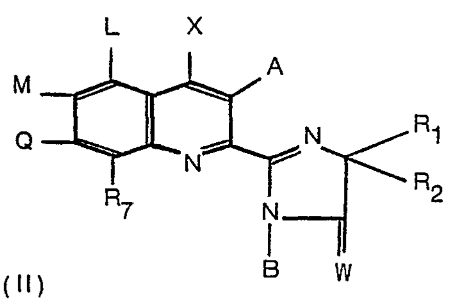
(II) ;

wherein $R_1$, $R_2$, W, B, A, X, L, M, Q and $R_7$ are as defined in reference to formula (I) above, but especially those wherein $R_1$ is methyl; $R_2$ is methyl, ethyl, isopropyl or cyclopropyl; W is oxygen; B is hydrogen, CO-alkyl $C_1$—$C_6$, CO-phenyl optionally substituted with one chloro, nitro or methoxy group; A is $COOR_3$,

$CH_2OH$ or CHO; $R_3$ is as defined in formula (I); X is hydrogen and L, M, Q and $R_7$ are each selected from the group consisting of hydrogen, halogen, methoxy, nitro, alkyl $C_1$—$C_4$, $CF_3$, CN, $N(CH_3)_2$, $NH_2$, $SCH_3$, or $SO_2CH_3$, provided that only one of L, M, Q or $R_7$ may be nitro, $CF_3$, CN, $N(CH_3)_2$, $NH_2$, $SCH_3$ or $SO_2CH_3$.

7. The method according to Claim 2, wherein said cereal crops are barley, oats, rice, rye, wheat and corn.

8. The method according to Claim 3, wherein said legumes are soybeans, beans, peas and lentils.

9. The method according to Claim 4, wherein said herbaceous ornamentals are colei, dahlias, tulips, daffodils, crocuses, chrysanthemums and roses.

10. A composition for the treatment of a one hectare plot, to increase the crop yield of cereal grains or legumes grown thereon, comprising: from 100 to 500 liters of water; from 0.01 to 0.001 kg of a compound having the structure:

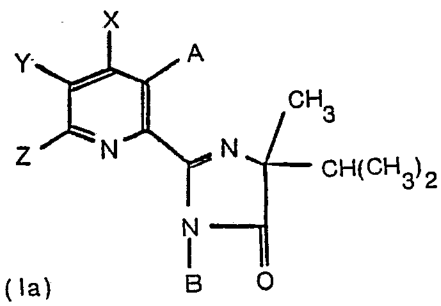

(Ia) ;

wherein B is hydrogen, CO-alkyl $C_1$—$C_6$ or CO-phenyl; A is $COOR_3$ where $R_3$ is as described in formula (I) above; X is hydrogen and Y and Z each represent hydrogen, $C_1$—$C_6$ alkyl, $C_1$—$C_4$-alkoxy, halo, $C_1$—$C_4$-haloalkyl, or phenyl and, when taken together, YZ represent —$(CH_2)_4$—; and, from 0.01% to 3% by weight of a dispersing agent and/or non-ionic surfactant.

**Revendications**

1. Procédé pour accroître la ramification axillaire, le tallage, la floraison et le rendement des cultures agronomiques et horticoles et pour accélérer la maturation des céréales cultivées comprenant: l'application auxdites plantes cultivées ou au sol contenant les semences ou d'autres organes de propagation desdites plantes d'un composé ayant la structure:

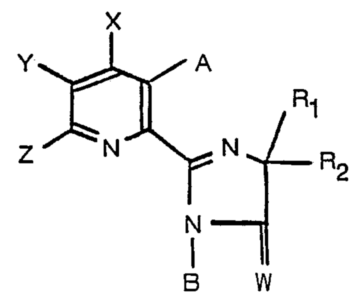

(I)

dans laquelle

$R_1$ est un alkyle en $C_1$—$C_4$;

$R_2$ est un alkyle en $C_1$—$C_4$ ou un cycloalkyle en $C_3$—$C_6$;

et lorsque $R_1$ et $R_2$ sont pris ensemble, ils peuvent représenter un cycloalkyle en $C_3$—$C_6$ éventuellement substitué par un méthyle;

A est $COOR_3$, $CONHR_6$, CHO, $CH_2OH$, $COCH_3$, $COC_6H_5$, CB, $CH_3$, CH=NOH, $CH_2COOH$, CONHOH, $CH_2CH_2COOH$, $CHR_8OH$,

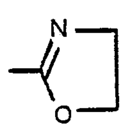

ou

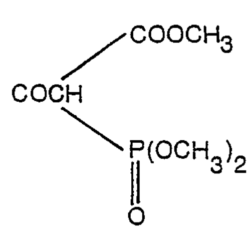

;

$R_3$ est un hydrogène.

un alkylidèneamino inférieur,

un alkyl en $C_1$—$C_{12}$ éventuellement substitué par un des groupes suivants: alcoxy en $C_1$—$C_3$,

halogéno, hydroxy, cycloalkyle en $C_3$—$C_6$, benzyloxy, furyle, phényle, halogénophényle, alkyle(inférieur)phényle, alcoxy(inférieur)phényle, nitrophényle, carboxy, alcoxy(inférieur)-carbonyle, cyano ou trialkyl(inférieur)ammonium;

un alcényle en $C_3$—$C_{12}$ éventuellement substitué par un des groupes suivants; alcoxy en $C_1$—$C_3$, phényle, halogéno ou alcoxy(inférieur)carbonyle ou par deux groupes alcoxy en $C_1$—$C_3$ ou deux groupes halogéno;

un cycloalkyle en $C_3$—$C_6$ éventuellement substitué par un ou deux groupes alkyle en $C_1$—$C_3$; un alcynyle en $C_3$—$C_{10}$ éventuellement substitué par n ou deux groupes alkyle en $C_1$—$C_3$; ou un cation choisi parmi le groupe constitué par les métaux alcalins, les métaux alcalino-terreux, le manganèse, le cuivre, le fer, le zinc, le cobalt, le plomb, l'argent, le nickel, l'ammonium et un ammonium organique;

$R_6$ est un hydrogène, un hydroxy, un alcényle en $C_3$, un alcynyle en $C_3$ ou un alkyle en $C_1$—$C_4$ éventuellement substitué par un groupe hydroxy ou un groupe chloro;

B est H, $COR_4$ ou $SO_2R_5$, sous réserve que lorsque B est $COR_4$ ou $SO_2R_5$: A est $COOR_3$ où $R_3$ est autre que H, ou un cation formant un sel, $CH_3$ ou CN; W est O; et Y et Z ne sont pas un alkylamino, un hydroxy ou un hydroxyalkyle inférieur;

$R_4$ est un alkyle en $C_1$—$C_{11}$, un chlorométhyle ou un phényle éventuellement substitué par un groupe chloro, un groupe nitro ou un groupe méthoxy;

$R_5$ est un alkyle en $C_1$—$C_4$ ou un phényle éventuellement substitué par un groupe méthyle;

W est O ou S;

$R_8$ est un alkyle en $C_1$—$C_4$ ou un phényle;

X est un hydrogène, un halogéno, un hydroxy ou un méthyle, sous réserve que lorsque Y et Z sont pris ensemble pour former un cycle et que YZ est représenté par la structure: $-(CH_2)_n-$, où n est 3 ou 4, X est un hydrogène;

Y et Z représentent chacun des composants choisis dans le groupe constitué par un hydrogène, un halogéno, un alkyle en $C_1$—$C_6$, un hydroxyalkyle inférieur, un alcoxy en $C_1$—$C_6$, un alkylthio en $C_1$—$C_4$, u phénoxy, un halogénoalkyle en $C_1$—$C_4$, un nitro, un cyano, un alkylamino en $C_1$—$C_4$, un dialkyle(inférieur)amino ou un alkylsulfonyle en $C_1$—$C_4$, ou un phényle éventuellement substitué par un alkyle en $C_1$—$C_4$, un alcoxy en $C_1$—$C_4$ ou un halogéno; et lorsqu'ils sont pris ensemble, Y et Z peuvent former un cycle dans lequel YZ sont représentés par la structure: $-(CH_2)_n-$ où n est un entier choisi parmi 3 et 4, sous réserve que X soit un hydrogène; ou

$$\begin{array}{cccc} L & M & Q & R_7 \\ | & | & | & | \\ -C= & C- & C= & C-, \end{array}$$

où L, M, Q et $R_7$ représentent chacun des composants choisis parmi le groupe constitué par un hydrogène, un halogéno, un alkyle en $C_1$—$C_4$, un alcoxy en $C_1$—$C_4$, un alkylthio en $C_1$—$C_4$, un alkylsulfonyle en $C_1$—$C_4$, un halogénoalkyle en $C_1$—$C_4$, $NO_2$, CN, un phényle, un phénoxy, un amino, un alkylamino en $C_1$—$C_4$, un dialkyle(inférieur)amino, un chlorophényle, un méthylphényle, ou un phénoxy substitué par un groupe, Cl, $CF_3$, $NO_2$ ou $CH_3$, sous réserve qu'un seul de L, M, Q ou $R_7$ peut représenter un substituant autre qu'un hydrogène, un halogéno, un alkyle en $C_1$—$C_4$ ou un alcoxy en $C_1$—$C_4$; et lorsque W est O et A est CN, $CH_3$ ou $COOR_3$, sous réserve que $R_3$ ne peut pas être un alkyle insaturé, et Y et Z ne peuvent pas être un alkylamino, un dialkylamino ou un alkylthio, et leurs N-oxydes, et lorsque $R_1$ et $R_2$ ne sont pas semblables, leurs isomères optiques, et, sauf lorsque $R_3$ est un cation formant un sel, leurs sels d'addition d'acides.

2. Procédé selon la revendication 1, pour induire le tallage, accélérer la maturation et accroître le rendement des céréales cultivées comprenant l'application auxdites plantes ou au sol contenant leurs semences de 0,0001 kg/ha à 0,01 kg/ha d'un composé de la revendication 1.

3. Procédé selon la revendication 1, pour induire la ramification axillaire, le tallage et pour accroître la floraison, le nombre des gousses et le rendement des légumes comprenant: l'application auxdites plantes ou au sol contenant leurs semences de 0,0001 kg/ha à 0,01 kg/ha d'un composé de la revendication 1.

4. Procédé selon la revendication 1, pour accroître la ramification axillaire et la floraison des plantes ornementales herbacées et latifoliées comprenant l'application au feuillage desdites plantes ou au sol contenant leurs semences ou d'autres organes de leur propagation de 0,0001 kg/ha à 0,01 kg/ha d'un composé de la revendication 1.

5. Procédé selon la revendication 1, dans lequel ledit composé a la structure:

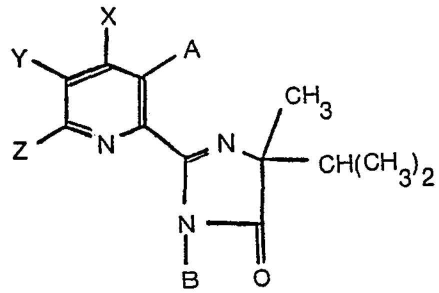

;

dans laquelle B est un hydrogène, un CO-alkyle en $C_1$—$C_6$ ou un CO-phényle; A est $COOR_3$ où $R_3$ est comme décrit pour la formule (I) ci-dessus; X est un hydrogène et Y et Z représentent chacun un hydrogène, un alkyle en $C_1$—$C_6$, un alcoxy en $C_1$—$C_4$, un halogéno, un halogénoalkyle en $C_1$—$C_4$ ou un phényle et lorsqu'ils sont pris ensemble, YZ représente —$(CH_2)_4$—.

6. Procédé selon la revendication 1, dans laquelle ledit composé a la structure:

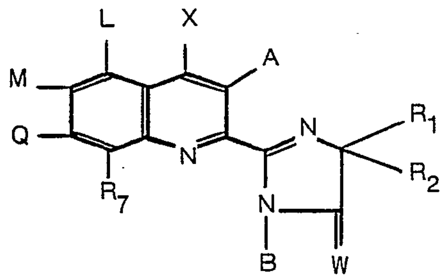

;

dans laquelle $R_1$, $R_2$, W, B, A, X, L, M, Q et $R_7$ sont comme défini relativement à la formule (I) ci-dessus, en particulier ceux dans lesquels $R_1$ est un méthyle; $R_2$ est un méthyle, un éthyle, un isopropyle ou un cyclopropyle; W est un oxygène; B est un hydrogène, un CO-alkyle en $C_1$—$C_6$, un CO-phényle éventuellement substitué par un groupe chloro, nitro ou méthoxy; A est $COOR_3$, $CH_2OH$ ou CHO; $R_3$ est comme défini dans la formule (I); X est un hydrogène et L, M, Q et $R_7$ sont choisis chacun parmi le groupe constitué par un hydrogène, un halogéno, un méthoxy, un nitro, un alkyle en $C_1$—$C_4$, $CF_3$, CN, $N(CH_3)_2$, $NH_2$, $SCH_3$ ou $SO_2CH_3$, sous réserve qu'un seul de L, M, Q ou $R_7$ puisse être un nitro, $CF_3$, CN, $N(CH_3)_2$, $NH_2$, $SCH_3$ ou $SO_2CH_3$.

7. Procédé selon la revendication 2, dans laquelle lesdites céréales cultivées sont l'orge, l'avoine, le riz, le seigle, le blé et le maïs.

8. Procédé selon la revendication 3, dans laquelle lesdits légumes sont les sojas, les haricots, les pois et les lentilles.

9. Procédé selon la revendication 4, dans laquelle lesdites plantes ornementales herbacées sont les Coleus, les dahlias, les tulipes, les jonquilles, les crosus, les chrysanthèmes et les roses.

10. Composition pour le traitement d'une parcelle d'un hectare, pour accroître le rendement des cultures de céréales ou de légumes qu'on y effectue comprenant: de 100 à 500 l d'eau, de 0,01 à 0,001 kg d'un composé ayant la structure:

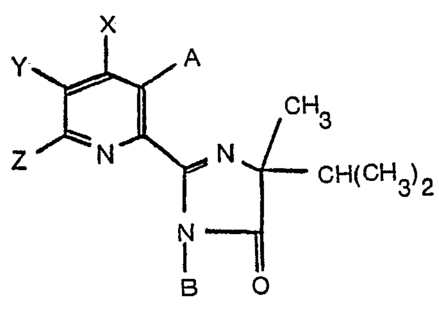

;

dans laquelle B est un hydrogène, un CO-alkyle en $C_1$—$C_6$ ou un CO-phényle; A est $COOR_3$ où $R_3$ est comme décrit dans la formule (I) ci-dessus; X est un hydrogène et Y et Z représentent chacun un hydrogène, un alkyle en $C_1$—$C_6$, un alcoxy en $C_1$—$C_4$, un halogéno, un halogénoalkyle en $C_1$—$C_4$ ou un phényle et, lorsqu'ils sont pris ensemble, YZ représente —$(CH_2)_4$—; et de 0,01% à 3% en poids d'un agent dispersant et/ou d'un agent tensio-actif non ionique.

**Patentansprüche**

1. Verfahren zur Verbesserung der axillaren Verzweigung, der Schößlings- und Blütenbildung sowie des Ertrags von landwirtschaftlichen und gartenbaulichen Nutzpflanzen sowie zur Beschleunigung des Reifungsprozesses von Getreidenutzpflanzen, dadurch gekennzeichnet, daß man den Nutzpflanzen oder dem Boden, der Samen oder andere Fortpflanzungsorgane dieser Pflanzen enthält, eine Verbindung der folgenden Formel (I) appliziert:

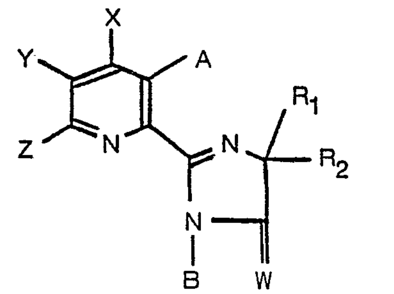

(I)

wobei
$R_1$ für $C_{1-4}$-Alkyl steht;
$R_2$ für $C_{1-4}$-Alkyl oder $C_{3-6}$-Cycloalkyl; wobei $R_1$ und $R_2$ gemeinsam eine gegebenenfalls mit Methyl substituierte $C_{3-6}$-Cycloalkylgruppe bilden können;
A für $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

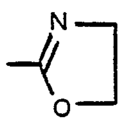

oder

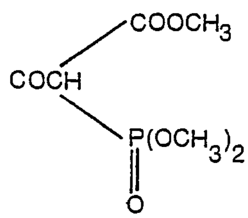

;

wobei
$R_3$ für Wasserstoff;
Niederalkylidenamino
$C_{1-12}$-Alkyl, gegebenenfalls substituiert mit einer der folgenden Gruppen: $C_{1-3}$-Alkoxy, Halogen, Hydroxyl, $C_{3-6}$-Cycloalkyl, Benzyloxy, Furyl, Phenyl, Halogenphenyl, Niederalkylphenyl, Niederalkoxyphenyl, Nitrophenyl, Carboxyl, Niederalkoxycarbonyl, Cyano oder Tri-niederalkylammonium;
$C_{3-12}$-Alkenyl, gegebenenfalls substituiert mit einer der folgenden Gruppen: $C_{1-3}$-Alkoxy, Phenyl, Halogen oder Niederalkoxycarbonyl oder mit zwei $C_{1-3}$-Alkoxygruppen oder mit zwei Halogenatomen;
$C_{3-6}$-Cycloalkyl, gegebenenfalls substituiert mit einer oder zwei $C_{1-3}$-Alkylgruppe(n);
$C_{3-10}$-Alkinyl, gegebenenfalls substituiert mit einer oder zwei $C_{1-3}$-Alkylgruppe(n); oder ein Kation steht, ausgewählt aus der Gruppe Alkalimetalle, Erdalkalimetalle, Mangan, Kupfer, Eisen, Zink, Kobalt, Blei, Silber, Nickel, Ammonium und organische Ammoniumverbindungen;
$R_6$ für Wasserstoff, Hydroxyl, $C_3$-Alkenyl, $C_3$-Alkinyl oder $C_{1-4}$-Alkyl, gegebenenfalls substituiert durch eine Hydroxylgruppe oder ein Chloratom;
B für H, $COR_4$ oder $SO_2R_5$, unter der Voraussetzung, daß dann, wenn B für $COR_4$ oder $SO_2R_5$ steht, der Rest A für $COOR_3$ steht, wobei $R_3$ eine andere Bedeutung hat als H oder ein salzbildendes Kation, oder für $CH_3$ oder CN steht; der Rest W für O steht; und die Reste Y und Z nicht für Alkylamino, Hydroxyl oder Hydroxyniederalkyl stehen;
$R_4$ für $C_{1-11}$-Alkyl, Chlormethyl oder Phenyl, das gegebenenfalls mit einem Chloratom, einer Nitro- oder einer Methoxygruppe substituiert ist;
$R_5$ für $C_{1-4}$-Alkyl oder Phenyl, das gegebenenfalls mit einer Methylgruppe substituiert ist;
W für O oder S;
$R_8$ für $C_{1-4}$-Alkyl oder Phenyl;
X für Wasserstoff, Halogen, Hydroxyl oder Methyl, mit der Maßgabe, daß dann, wenn Y und Z gemeinsam einen Ring bilden und YZ für die Struktur $—(CH_2)_n—$ steht, wobei n 3 oder 4 bedeutet, X für Wasserstoff steht;
Y und Z jeweils für einen Rest aus der folgenden Gruppe stehen: Wasserstoff, Halogen, $C_{1-6}$-Alkyl, Hydroxy-niederalkyl, $C_{1-6}$-Alkoxy, $C_{1-4}$-Alkylthio, Phenoxy, $C_{1-4}$-Halogenalkyl, Nitro, Cyano, $C_{1-4}$-Alkylamino, Di-niederalkylamino oder $C_{1-4}$-Alkylsulfonyl oder Phenyl, das gegebenenfalls mit einer $C_{1-4}$-Alkylgruppe, einer $C_{1-4}$-Alkoxygruppe oder einem Halogenatom substituiert ist; oder wobei Y und Z gemeinsam einen Ring bilden können, wobei die Gruppe YZ die folgende Struktur hat: $—(CH_2)_n—$,

wobei n für 3 oder 4 steht und wobei die Bedingung X=H gilt; oder wobei die Gruppe YZ die folgende Struktur hat:

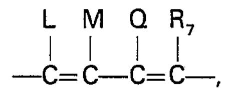

in der L, M, Q und $R_7$ jeweils ausgewählt sind unter Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$, CN, Phenyl, Phenoxy, Amino, $C_{1-4}$-Alkyamino, Di-niederalkylamino, Chlorphenyl, Methylphenyl oder Phenoxy, das gegebenenfalls mit einer der Gruppen Cl, $CF_3$, $NO_2$ oder $CH_3$ substituiert ist, mit der Maßgabe, daß nur einer der Reste L, M, Q oder $R_7$ für einen Substituenten stehen kann, welcher abweicht von Wasserstoff, Halogen, $C_{1-4}$-Alkyl oder $C_{1-4}$-Alkoxy;
sowie die N-Oxide derselben, falls W für O steht und A für CN, $CH_3$ oder $COOR_3$ steht, vorausgesetzt, daß $R_3$ nicht ungesättigtes Alkyl sein kann und Y und Z weder für Alkylamino noch für Dialkylamino noch für Alkylthio stehen können;
sowie die optischen Isomeren derselben, falls $R_1$ und $R_2$ nicht gleich sind;
sowie die Säureadditionssalze derselben, falls $R_3$ kein salzbildendes Kation bedeutet.

2. Verfahren nach Anspruch 1, dadurch gekennzeichnet, daß man zur Induzierung des Ährenschiebens, zur Beschleunigung des Reifungsprozesses und zur Steigerung des Ertrags von Getreidenutzpflanzen den Pflanzen oder dem Boden, der die Saat derselben enthält, von 0,0001 bis 0,01 kg/ha einer Verbindung gemäß Anspruch 1 appliziert.

3. Verfahren nach Anspruch 1, dadurch gekennzeichnet, daß man zur Induzierung der axillaren Verzweigung, der Schößlingsbildung sowie zur Verbesserung des Blühens, des Bestockens und des Ertrags von Leguminosen den Pflanzen oder dem Boden, der die Saat derselben enthält, von 0,0001 bis 0,01 kg/ha einer Verbindung gemäß Anspruch 1 appliziert.

4. Verfahren nach Anspruch 1, dadurch gekennzeichnet, daß man zur Verbesserung der axillaren Verzweigung, und des Blühens von krautartigen Breitblatt-Zierpflanzen den Blättern der Pflanzen oder dem Boden, der Samen oder andere Fortpflanzungsorgane derselben enthält, von 0,0001 bis 0,02 kg/ha einer Verbindung gemäß Anspruch 1 appliziert.

5. Verfahren nach Anspruch 1, dadurch gekennzeichnet, daß die verwendete Verbindung die folgende formel aufweist:

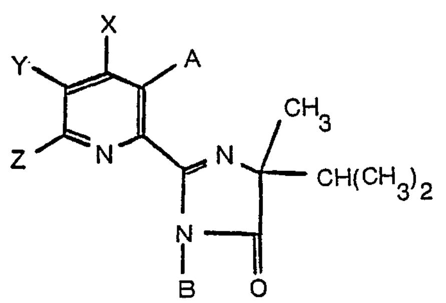

; (Ia)

wobei B für Wasserstoff, CO-alkyl-$C_{1-6}$ oder CO-phenyl steht; A für $COOR_3$ steht, wobei $R_3$ die bei Formel (I) angegebene Bedeutung hat; X für Wasserstoff steht und Y und Z jeweils Wasserstoff, $C_{1-6}$-Alkyl, $C_{1-4}$-Alkoxy, Halogen, $C_{1-4}$-Halogenalkyl oder Phenyl bedeuten und wobei, wenn Y und Z zusammengefaßt sind, YZ für $-(CH_2)_4-$ steht.

6. Verfahren nach Anspruch 1, dadurch gekennzeichnet, daß die eingesetzte Verbindung die folgende Formel aufweist:

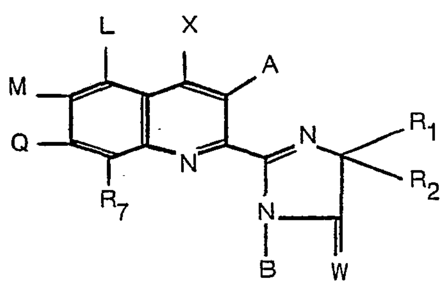

; (II)

wobei $R_1$, $R_2$, W, B, A, X, L, M, Q und $R_7$ die mit Bezug zur obigen Formel (I) angegebenen Bedeutungen haben, insbesondere jedoch diejenigen, bei denen $R_1$ für Methyl steht; $R_2$ Methyl, Äthyl, Isopropyl oder Cyclopropyl bedeutet; W für Sauerstoff steht; B Wasserstoff, CO-alkyl-$C_{1-6}$, CO-phenyl, gegebenenfalls substituiert mit einem Chloratom, einer Nitro- oder Methoxygruppe, bedeutet; A für $COOR_3$, $CH_2OH$

oder CHO steht; wobei $R_3$ die bei Formel (I) angegebene Bedeutung hat; X für Wasserstoff steht und L, M, Q und $R_7$ jeweils ausgewählt sind unter Wasserstoff, Halogen, Methoxy, Nitro, $C_{1-4}$-Alkyl, $CF_3$, CN, $N(CH_3)_2$, $NH_2$, $SCH_3$ oder $SO_2CH_3$, mit der Maßgabe, daß lediglich einer der Reste L, M, Q oder $R_7$ für Nitro, $CF_3$, CN, $N(CH_3)_2$, $NH_2$, $SCH_3$ oder $SO_2CH_3$ stehen darf.

7. Verfahren nach Anspruch 2, dadurch gekennzeichnet, daß es sich bei den Getreidenutzpflanzen um Gerste, Hafer, Reis, Roggen, Weizen und Mais handelt.

8. Verfahren nach Anspruch 3, dadurch gekennzeichnet, daß es sich bei den Leguminosen um Sojabohnen, Bohnen, Erbsen und Linsen handelt.

9. Verfahren nach Anspruch 4, dadurch gekennzeichnet, daß es sich bei den krautartigen Zierpflanzen um Colei, Dahlien, Tulpen, Narzissen, Krokusse, Chrysanthemen und Rosen handelt.

10. Mittel zur Behandlung einer 1 ha-Fläche zum Zwecke der Steigerung des Ernteertrags an darauf erzeugtem Getreide oder Leguminosen, gekennzeichnet durch von 100 bis 500 l Wasser; von 0,01 bis 0,001 kg einer Verbindung der Formel

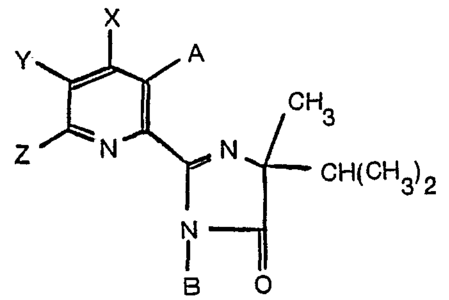 ; (Ia)

wobei B für Wasserstoff, CO-alkyl-$C_{1-6}$ oder CO-phenyl steht; A für $COOR_3$ steht, wobei $R_3$ die bei Formel (I) angegebene Bedeutung hat; X für Wasserstoff steht und Y und Z jeweils Wasserstoff, $C_{1-6}$-Alkyl, $C_{1-4}$-Alkoxy, Halogen, $C_{1-4}$-Halogenalkyl oder Phenyl bedeuten, und falls Y und Z zusammengefaßt sind, YZ für $-(CH_2)_4-$ steht; und von 0,01 bis 3 Gew.% eines Dispergiermittels und/oder eines nichtionischen Surfactants.